# EUROPÄISCHE PATENTSCHRIFT

(11) **EP 1 287 139 B1**
(45) Veröffentlichungstag und Bekanntmachung des Hinweises auf die Patenterteilung: **25.08.2010**
(21) Anmeldenummer: 01964966.4
(22) Anmeldetag: 11.06.2001
(51) Int. Cl.: C12N 15/12, C12N 15/82, C07K 14/435, A01H 5/00

(54) **SYNTHETISCHE SPINNENSEIDENPROTEINE UND DEREN EXPRESSION IN TRANSGENEN PFLANZEN**
SYNTHETIC SPIDER SILK PROTEINS AND THE EXPRESSION THEREOF IN TRANSGENIC PLANTS
PROTEINES DE SOIE D'ARAIGNEE SYNTHETIQUES ET LEUR EXPRESSION DANS DES PLANTES TRANSGENIQUES

(30) Priorität: 09.06.2000 DE 10028212; 24.10.2000 DE 10053478; 21.03.2001 DE 10113781
(43) Veröffentlichungstag der Anmeldung: 05.03.2003
(73) Patentinhaber: Leibniz-Institut für Pflanzengenetik und Kulturpflanzenforschung (IPK), 06466 Gatersleben (DE)
(72) Erfinder: SCHELLER, Jürgen, 24103 Kiel (DE); CONRAD, Udo, 06458 Hausneindorf (DE); GROSSE, Frank, 07743 Jena (DE); GUEHRS, Karl-Heinz, 07745 Jena (DE)
(74) Vertreter: Maiwald Patentanwalts GmbH
(86) Internationale Anmeldenummer: PCT/EP2001/006586
(87) Internationale Veröffentlichungsnummer: WO 2001/094393

(56) Entgegenhaltungen:
- WO-A-01/90389
- WO-A-94/29450
- WO-A-97/08315
- US-A- 5 770 697
- SCHELLER JUERGEN ET AL: "Production of spider silk proteins in tobacco and potato." NATURE BIOTECHNOLOGY, Bd. 19, Nr. 6, Juni 2001 (2001-06), Seiten 573-577, XP002189903 ISSN: 1087-0156
- JUNGHANS F. ET AL: "Preparation and mechanical properties of layers made of recombinant spider silk proteins and silk from silk worm" APPLIED PHYSICS A, vol. 82, no. 2, 1 February 2006 (2006-02-01), pages 253-260, XP019337140 MATERIALS SCIENCE & PROCESSING, SPRINGER-VERLAG, BE

## Beschreibung

Die Erfindung betrifft eine DNA-Sequenz, die für ein synthetisches Spinnenseidenprotein kodiert, rekombinante Spinnenseidenproteine, die durch die erfindungsgemäße DNA-Sequenz kodiert sind, Verfahren zur Herstellung von Pflanzen bzw. Pflanzenzellen, die rekombinantes Spinnenseidenprotein enthalten sowie transgene Pflanzenzellen und Pflanzen, die eine DNA-Sequenz enthalten, die für ein synthetisches Spinnenseidenprotein kodiert. Des weiteren betrifft die Erfindung ein Verfahren zur Gewinnung von pflanzlichem Spinnenseidenprotein aus transgenen Pflanzen sowie pflanzliche Spinnenseidenproteine, die nach einem derartigen Verfahren hergestellt worden sind.

Spinnenseide weist hervorragende mechanische Eigenschaften auf, die jene vieler bekannter natürlicher und künstlicher Materialien übertrifft. Hauptbestandteile der Spinnenseide sind Faserproteine wie beispielsweise Fibroin aus dem Seidenspinner sowie Spidroin 1 und Spidroin 2 aus *Nephila clavipes.* Die Festigkeit und Elastizität der Seide beruht auf der Gegenwart von kurzen repetitiven Aminosäure-Einheiten, die in diesen natürlichen Proteinen vorliegen. Diese mechanischen Eigenschaften prädestinieren die Spinnenseide für eine Reihe von verschiedensten technischen Anwendungen wie beispielsweise die Herstellung von stabilen Fäden bzw. Seiden. Ferner verfügen die Spinnenseidenfäden aufgrund ihrer proteinchemischen Eigenschaften über ein geringes immunogenes und allergenes Potential, weshalb sich in Kombination mit den mechanischen Eigenschaften eine Anwendung in der Medizin beispielsweise als natürliches Garn zum Vernähen von Wunden, als Anheftungsflächen für kultivierte Zellen, als Gerüste für künstliche Organe und dergleichen anbietet.

Voraussetzung für eine derartige technische bzw. medizinische Nutzung der Spinnenseide ist jedoch die Herstellung von Spinnenfäden bzw.

Spinnenseidenproteinen in großem Maßstab. Zu diesem Zweck wurde bislang versucht, die für die Produktion der Spinnenseide verantwortlichen Spidroin- bzw. Fibroingene in *E. coli* zu exprimieren. Die sich häufig wiederholenden Sequenzen in den entsprechenden Genen gehen jedoch bei der Reproduktion in Bakterien nach und nach verloren. Ein weiteres Problem ist die Größe der genetischen Information, die für das Bakterium zu umfangreich zu sein scheint, so daß die Spinnenseiden-Gene nicht immer vollständig ausgelesen werden.

Versuche der Expression in Hefezellen ergaben zwar stabilere und längeren Seidenproteine, die Fäden, die daraus gesponnen wurden, weisen jedoch nicht die selben vorteilhaften Eigenschaften der natürlichen Seide auf, so daß beispielsweise eine medizinische Anwendung einer derart synthetisch hergestellten Seide nicht möglich ist. Es besteht somit ein Bedarf an synthetischen Seidenproteinen, die in technischem Maßstab hergestellt werden können und nach dem Verspinnen zu Fäden mechanische Eigenschaften aufweisen, die mit jenen der natürlichen Seide vergleichbar sind.

Aufgabe der vorliegenden Erfindung ist es deshalb, DNA-Sequenzen bereitzustellen, die für ein synthetisches Spinnenseidenprotein kodieren, das eine möglichst große Ähnlichkeit mit den bisher bekannten natürlichen Sequenzen von Faserproteinen der Spinnenseide aufweist. Ferner ist es Aufgabe der vorliegenden Erfindung, ein Verfahren bereitzustellen, mit dem synthetische Spinnenseidenproteine in großem Maßstab hergestellt werden können.

Aufgabe der Erfindung ist es auch, DNA-Sequenzen bereitzustellen, die für ein synthetisches Spinnenseidenprotein kodieren, das zwar die vorteilhaften und wünschenswerten Eigenschaften von nativem Spinnenseidenprotein aufweist, bei dem das Eigenschaftsspektrum des nativen Proteins aber zusätzlich in die eine oder andere Richtung, je nach dem jeweiligen Anwendungszweck, modifiziert bzw. optimiert ist

Weitere Aufgaben der vorliegenden Erfindung ergeben sich aus der folgenden Beschreibung.

Oben genannte Aufgaben werden durch die Merkmale der unabhängigen Schutzansprüche gelöst.

Vorteilhafte Ausgestaltungen sind in den Unteransprüchen definiert.

Im Rahmen der vorliegenden Erfindung wird jetzt eine DNA-Sequenz offenbart, die für ein synthetisches Faserprotein, insbesondere ein synthetisches Spinnenseidenprotein kodiert, das eine mindestens 94%ige Homologie zu Spidroin- Proteinen, insbesondere zu dem Spidroin 1-Protein, besonders bevorzugt zu dem Spidroin 1-Protein aus *Nephila clavipes* aufweist.

Homologie bedeutet im Rahmen dieser Erfindung Ähnlichkeit zwischen Aminosäuresequenzen aufgrund von identischen bzw. homologen Aminosäurebausteinen. Welche Aminosäuren als homolog anzusehen sind, ist dem Fachmann bekannt, z.B. (i) Isoleucin, Leucin und Valin untereinander, (ii) Asparagin und Glutamin, (iii) Asparaginsäure und Glutaminsäure.

Die erfindungsgemäße DNA-Sequenz ist aus Modulen aufgebaut, die eine Gruppe von aneinandergereihten Oligonukleotidsequenzen umfassen, wobei die Oligonukleotidsequenzen jeweils für repetitive Einheiten aus Spidroin-Proteinen kodieren.

Der Aufbau der erfindungsgemäßen DNA-Sequenz aus verschiedenen Modulen, welche wiederum aus unterschiedlichen, für Spidroine typischen kurzen Aminosäure-Repeats konstruiert sind, wobei sich das Prinzip der Aneinanderreihung der entsprechenden Oligonukleotidsequenzen bzw. der Module an natürlichen Spidroin- Sequenzen orientiert, gewährleistet eine sehr hohe Homologie zu bisher bekannten natürlichen Spidroin-Sequenzen. Dadurch wird sichergestellt, daß die durch die erfindungsgemäße DNA-Sequenz kodierten Spinnenseidenproteine nach dem Verspinnen zu Fäden hervorragende mechanische Eigenschaften bezüglich ihrer Festigkeit und Elastizität aufweisen, die mit den mechanischen Eigenschaften von natürlichen Spinnenfäden vergleichbar sind.

Des weiteren ermöglicht der modulartige Aufbau der erfindungsgemäßen DNA-Sequenz eine einfache gentechnische Modifizierung der synthetischen Gene, so daß Multimere von synthetischen Spinnenseidenproteinen mit beliebiger Größe je nach Wunsch hergestellt werden können. Ferner können die durch die erfindungsgemäße DNA-Sequenz kodierten Spinnenseidenproteine aufgrund des modulartigen Aufbaus mit anderen Faserproteinsequenzen fusioniert werden. Ein besonderer Vorteil der erfindungsgemäßen DNA-Sequenz ist, daß sie aufgrund ihres modulartigen Aufbaus auf einfache Weise mit für Reinigungselemente oder Löslichkeits-verändernde Peptide kodierenden Sequenzen fusioniert werden kann.

Die Erfindung betrifft auch DNA-Sequenzen, die für ein synthetisches Spinnenseidenprotein kodieren und aus Modulen aufgebaut sind, die eine Gruppe von aneinandergereihten Oligonukleotidsequenzen umfassen, wobei die Oligonukleotidsequenzen jeweils für repetitive Einheiten aus Spidroin-Proteinen kodieren und die Module in freier Weise angeordnet sind, wobei die freie Anordnung ermöglicht, daß das synthetische Spinnenseidenprotein ein im Vergleich zu nativem Spinnenseidenprotein verändertes Eigenschaftsspektrum aufweist.

Die Erfindung liefert somit erstmals die Möglichkeit, neuartige Seidenproteine auf der Grundlage von modular aufgebauten Seidenproteingenen zu synthetisieren, wobei die neuartigen Seidenprateine ein im Vergleich zu nativem Seidenprotein modifiziertes Eigenschaftsspektrum aufweisen, gleichzeitig aber die wesentlichen Strukturdeterminanten von natürlich vorkommenden Seidenproteinen enthalten. Ausgehend vom Erhalt der wesentlichen strukturellen Abschnitte der natürlichen Seidenproteine, die erfindungsgemäß in neuartiger Weise miteinander kombiniert werden, werden synthetische Seidenproteine bereitgestellt, die bspw. hinsichtlich ihrer Elastizität, ihrer Reißfestigkeit, ihres Löslichkeitsverhalten, ihrer Hitze- und Säurebestandigkeit, ihres Quellungwermögens in eine bestimmte, für den jeweiligen Einsatz vorteilhafte Richtung modifiziert bzw. optimiert sind.

In jedem Fall läßt sich die Eigenscbaftskombiwation, welche die materialtechnische Attraktivität und Nützlichkeit der erfindungsgemäßen rekombinanten Spinnenseidenproteine als Werkstoff ausmacht, durch die Anordnung der Module in gewünschten Grenzen beeinflussen, ohne sich jedoch zu stark von dem attraktiven Eigenschaftsspektrum des zugrunde liegenden natürlichen Proteins zu unterscheiden.

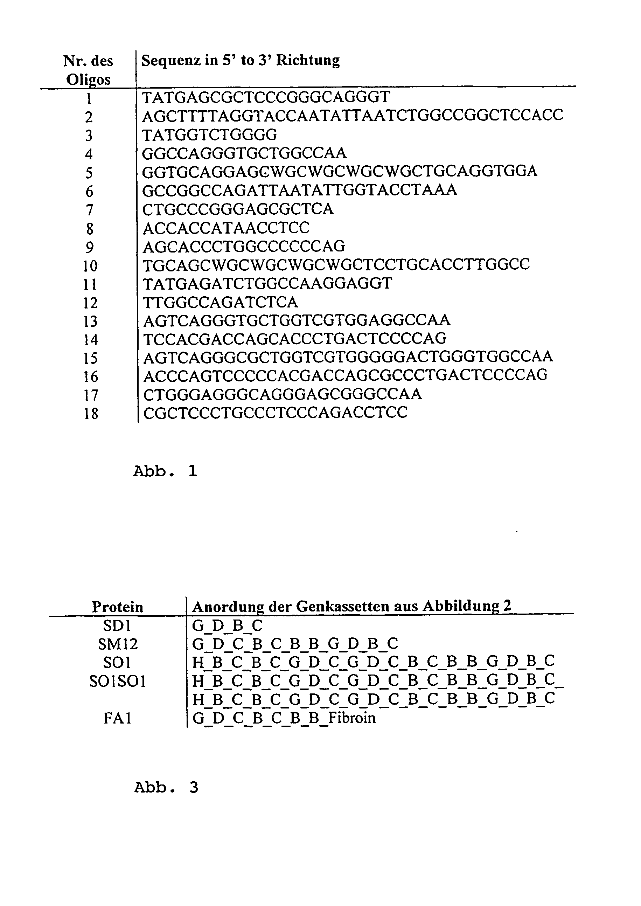

Die Genkassette mit der höchsten Homologie zur aus dem nativen Wirt isolierten cDNA, SO1 genannt, weist die nachfolgende Kombination der als Modul (mit verschiedenen Buchstaben dargestellt) bezeichneten strukturellen Abschnitte auf:
H_B_C_B_C_G_D_C_G_D_C_B_C_B_B_G_D_B_C
(siehe auch Abbildung 3). Im Gegensatz zu den Ansätzen im Stand der Technik in Bezug auf Spinnenseiden und Naturseiden ermöglicht die erfindungsgemäße Lehre zur Assemblierung der Genkassetten eine neuartige und gezielte Anordnung dieser Module in vollständig freier Weise. Dadurch können vollkommen neuartige Proteine erzeugt werden, aber auch das natürlich vorkommende Protein nachgebaut werden.

Zusätzlich zu den bereits genannten Eigenschaften, die modifiziert bzw. optimiert werden können, kann bspw. durch zusätzliche RGD-Sequenzen eine verstärkte Anheftung von Zellen erreicht werden (Massia et al. (2001) J. Biomed. Mater. Res. 56.390-399). Weitere nützliche Eigenschaften der synthetischen Spinnenseidenproteine gemäß der Erfindung ergeben sich auch der nachfolgenden Beschreibung und den Beispielen.

Bei einer besonders bevorzugten Ausführungsform der vorliegenden Erfindung weist das durch die erfindungsgemäße DNA-Sequenz kodierte Spinnenseidenprotein eine mindestens 94 %ige Homologie zu dem Spidroin 1-Protein aus *Nephila clavipes* auf. Spidroin 1 aus *Nephila clavipes* ist wesentlich am Aufbau eines mechanisch besonders stabilen und elastischen Tragfadens beteiligt.

Aufgrund des modulartigen Aufbaus der erfindungsgemäßen DNA-Sequenz ist die Konstruktion von Genen, die sehr große Spinnenseidenproteine kodieren, ohne weiteres möglich, wobei die hohe Homologie zu Spidroin-Proteinen, insbesondere zu Spidroin 1, besonders bevorzugt zu Spidroin 1 von *Nephila clavipes* immer erhalten bleibt. Die so erzielbare Größenverteilung der durch die erfindungsgemäßen DNA-Sequenzen kodierten Proteine entspricht dem Spektrum von Spinnenseidenproteinen, das nach der Auflösung von natürlicher Spinnenseide beobachten werden kann. Dieses identische Größenspektrum sowie die hohe Sequenzhomologie definieren die erfindungsgemäßen synthetischen Gene als Gene, die für Spinnenseidenproteine kodieren. Im Gegensatz zu natürlicher Spinnenseide, die aus einem Gemisch von Spinnenseidenproteinen besteht, werden durch die vorliegende Erfindung Spinnenseidenprotein-Gene bereitgestellt, die mit hoher Homologie eine Genklasse repräsentieren und eine einfache gentechnische Manipulation erlauben.

Der Aufbau der erfindungsgemäßen DNA-Sequenz wird im folgenden beispielhaft dargestellt. Zunächst werden die in Abbildung 1 angegebenen Oligonukleotide bereitgestellt, die für Aminosäuresequenzen kodieren, die Spidroin-typischen kurzen Aminosäure-Repeats entsprechen. Diese Oligonukleotide werden durch gentechnische Verfahren miteinander kombiniert, wobei sich die Kombination an der natürlichen Spidroin-Sequenz richtet (siehe Abbildung 2). Die so entstandenen Module A, B, C, D, E und F werden erneut miteinander kombiniert (siehe Abbildung 3). Auf diese Weise werden erfindungsgemäße DNA-Sequenzen bereitgestellt, die auf Aminosäureebene eine mindestens 94%ige Homologie zu Spidroin-Proteinen zeigen.

Bei einer weiteren Ausführungsform umfaßt die erfindungsgemäße DNA-Sequenz zusätzlich zu den vorstehend beschriebenen Modulen Nukleinsäuresequenzen, die für repetitive Einheiten aus Fibroin-Proteinen, vorzugsweise aus dem Fibroin-Protein des Seidenspinners kodieren.

Die erfindungsgemäße DNA-Sequenz weist die Sequenz SEQ ID No. 27 oder eine Sequenz, die zu der in SEQ ID NO. 27 angegebenen Sequenz eine Sequenzidentität von mindestens 98% aufweist, auf.

Erfindungsgemäß ist es ferner überraschenderweise erstmals gelungen, synthetische Spinnenseidenproteine in transgenen Pflanzen zu erzeugen. Auf diese Weise können synthetische Spinnenseidenproteine in großem Maßstab hergestellt werden. Um eine stabile Expression der erfindungsgemäßen DNA-Sequenz in Pflanzen zu gewährleisten, wird erfindungsgemäß ein rekombinantes Nukleinsäuremolekül bereitgestellt, das die vorstehend beschriebene erfindungsgemäße DNA-Sequenz sowie einen ubiquitär wirkenden Promotor, vorzugsweise den CaMV35S-Pcomotor umfaßt Die Bereitstellung des erfindungsgemäßen rekombinanten Nukleinsäuremoleküls ermöglicht die Expression und Akkumulation von synthetischen Spidroin sequenzen in transgenen Pflanzen.

Um sicherzustellen, daß die erfindungsgemäße DNA-Sequenz in geeigneten Kompartimenten von transgenen Pflanzen exprimiert und akkumuliert wird, umfaßt das erfindungsgemäße Nuklemsäuremolekül zusätzlich zu der erfindungsgemäßen DNA-Sequenz und einem ubiquitär wirkenden Promotor vorzugsweise mindestens eine Nukleinsäuresequenz, die für ein pflanzliches Signalpeptid kodiert.

Bei einer bevorzugten Ausführungsform wird als Zielkompartiment für die Expression bzw. Akkumulation des synthetischen Spinnenseidenproteins das endoplasmatische Retikulum (ER) ausgewählt. Dieses Kompartiment ist für die stabile Akkumulation von Fremdproteinen in Pflanzen besonders geeignet. Um den Transport in das ER zu gewährleisten, umfaßt das erfindungsgemäße Nukleinsäuremolekül bevorzugt entsprechende Signalpeptide, besonders bevorzugt die LeB4Sp-Sequenz.

Die Retention im ER, falls gewünscht, wird erfindungsgemäß dadurch gewährleistet, daß das erfindungsgemäße Nukleinsäuremolekül zusätzlich eine Nukleinsäuresequenz umfaßt, die für ein ER-Retentionspeptid kodiert. Vorzugsweise wird die Retention in ER durch die C-terminal angefügte Aminosäuresequenz KDEL erreicht.

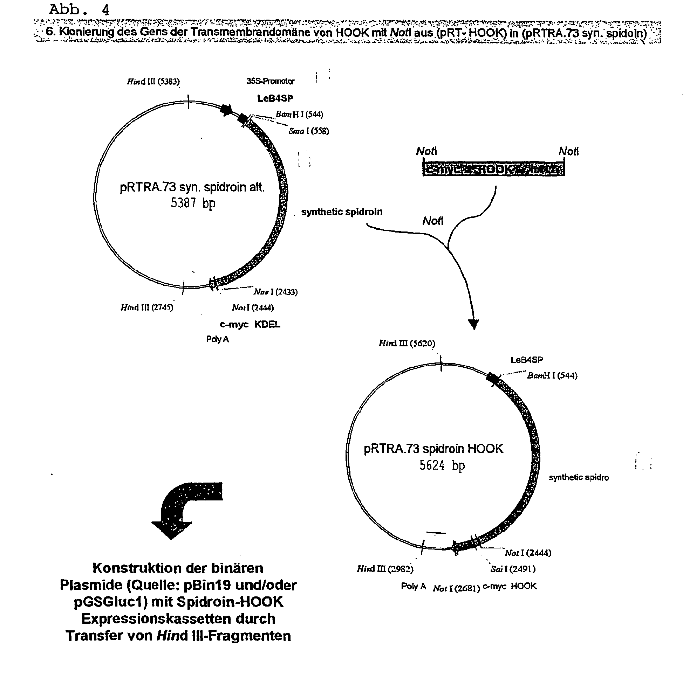

Ferner kann es vorteilhaft sein, die erfindungsgemäße DNA-Sequenz an der Plasmalemma, d.h. der Zellmembran, zu plazieren. Deshalb umfaßt das erfindungsgemäße rekombinante Nukleinsäuremolekül bei einer alternativen Ausführungsform die erfindungsgemäße DNA-Sequenz, fusioniert an den N-Terminus einer Transmembrandomäne. Vorzugsweise ist diese Transmembrandomäne die Transmembrandomäne des PDGF-Rezeptors, die sogenannte HOOK-Sequenz (siehe Abbildung 4).

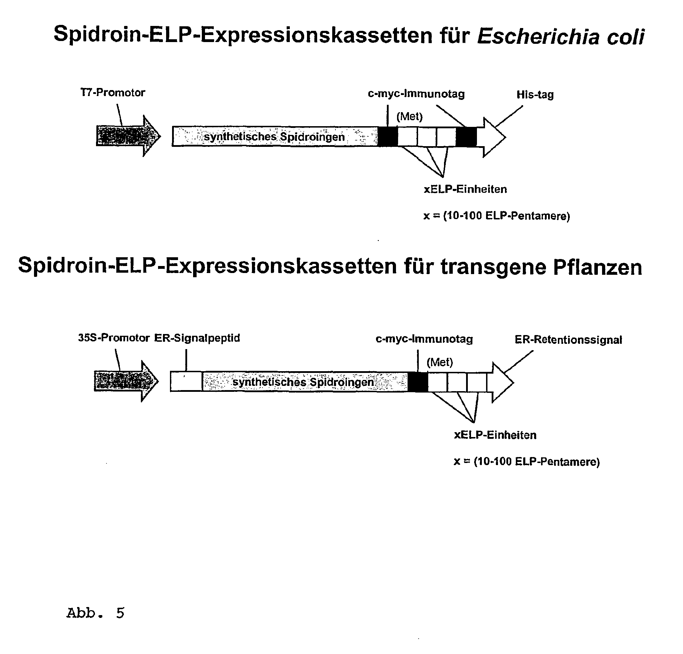

Das erfindungsgemäBe Nukleinsäuremolekül ist mit ELP's (elastin-like polypeptides) fusioniert. ELP's sind oligomere Repeats des Pentapeptids Val-Pro-Gly-Xaa-Gly (wobei Xaa jede Aminosäure außer Prolin und vorzugsweise Gly ist) und unterliegen einem reversiblen inversen Temperaturübergang. Sie sind in Wasser unterhalb der inversen Übergangstemperatur (Tₜ) sehr gut löslich, unterliegen jedoch einem scharfen Phasenübergang im Bereich von 2°C bis 3°C, wenn die Temperatur über Tₜ erhöht wird, was zur Ausfällung und Aggregation des Polypeptids führt. D. E. Meyer und A. Chilkoti, Nat. Biotech. 1999, 17, 1112-1115, haben beschrieben, daß ELP-Fusionen mit rekombinanten Proteinen das Löslichkeitsverhalten dieser rekombinanten Proteine bei verschiedenen Temperaturen und Konzentrationen gezielt verändern. Bei der vorliegenden Erfindung wird dies zur Etablierung von im nachfolgenden detailliert beschriebenen Reinigungsstrategien für das durch die erfindungsgemäße DNA-Sequenz kodierte Spinnenseidenprotein genutzt. Die durch die Nukleinsäuresequenz in dem erfindungsgemäßen Nukleinsäuremolekül kodierten ELP's umfassen von 10 bis 100 der vorstehend beschriebenen Pentamer-Einheiten (siehe Abbildung 5).

Die Herstellung der vorstehend beschriebenen chimären Genkonstrukte bzw. rekombinanten Nukleinsäuremoleküle erfolgt mittels konventioneller Klonierungstechniken (siehe beispielsweise Sambrook et al. (1989, Molecular Cloning: A Laboratory Manual, 2. Auflage, Cold Spring Harbour Laboratory Press, Cold Spring Harbour, New York). Mittels dieser gängigen molekularbiologischen Techniken ist es möglich, gewünschte Konstrukte für die Transformation von Pflanzen vorzubereiten bzw. herzustellen. Die für die gentechnische Manipulation in prokaryontischen Zellen üblicherweise eingesetzten Klonierungs-, Mutagenisierungs-, Sequenzanalyse- und Restriktionsanalyse-Methoden sowie weitere biochermsch-molokulerbiologische Methoden sind dem Fachmann wohlbekannt. So können nicht nur geeignete chimäre Genkonstrukte mit der jeweils gewünschten Fusion von Promotor, erfindungsgemäßer DNA-Sequenz, für ein pflanzliches Signalpeptid kodierender Sequenz, für ein ER-Retentionspeptid kodierender Sequenz, für eine Transmembrandomäne kodierender Sequenz und für Reinigungselemente bzw. Löslichkeits-verändernde Peptide kodierenden Sequenzen hergestellt werden. Vielmehr kann der Fachmann mittels Routinetechniken, falls erwünscht, verschiedenartige Mutationen oder Deletionen in die jeweiligen Gene einführen.

Die Erfindung betrifft weiterhin Vektoren und Mikroorganismen, die erfindungsgemäße Nukleinsäuremoleküle enthalten und deren Verwendung die Herstellung von Pflanzenzellen bzw. Pflanzen ermöglicht, die Spinnenseidenproteine produzieren. Dabei handelt es sich bei den Vektoren insbesondere um Plasmide, Cosmide, Viren, Bakteriophagen und andere in der Gentechnik gängige Vektoren. Bei den Mikroorganismen handelt es sich in erster Linie um Bakterien, Viren, Pilze, Hefen und Algen.

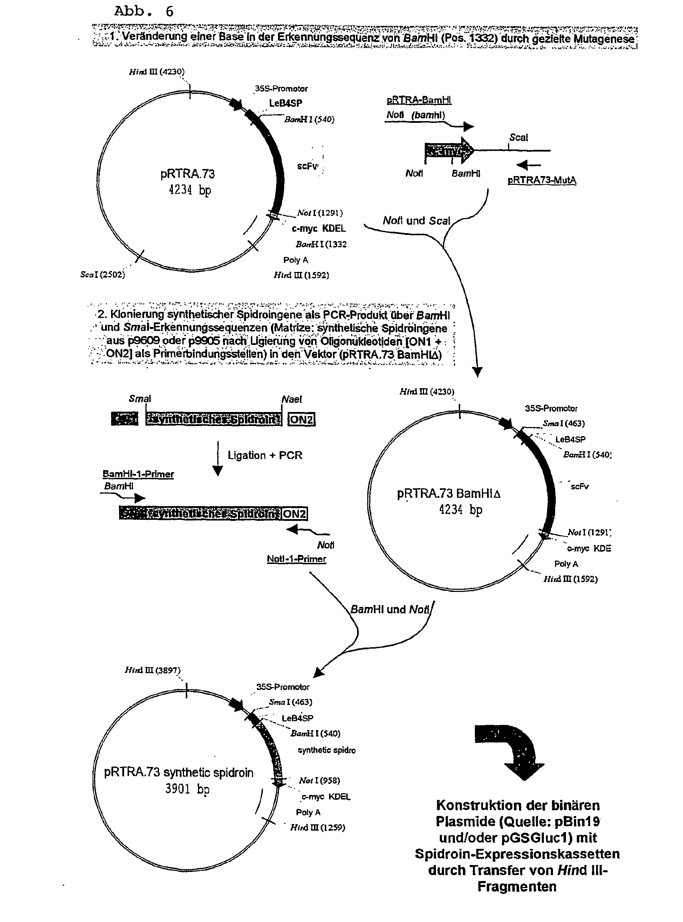

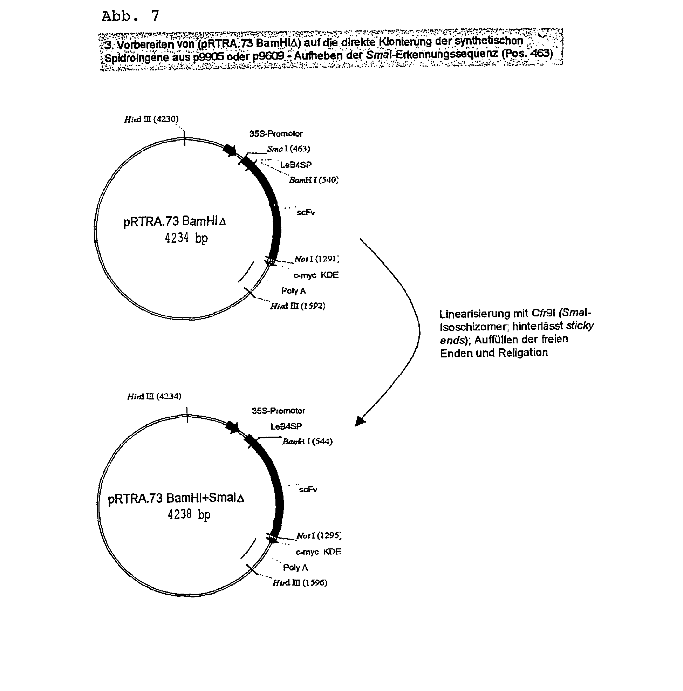

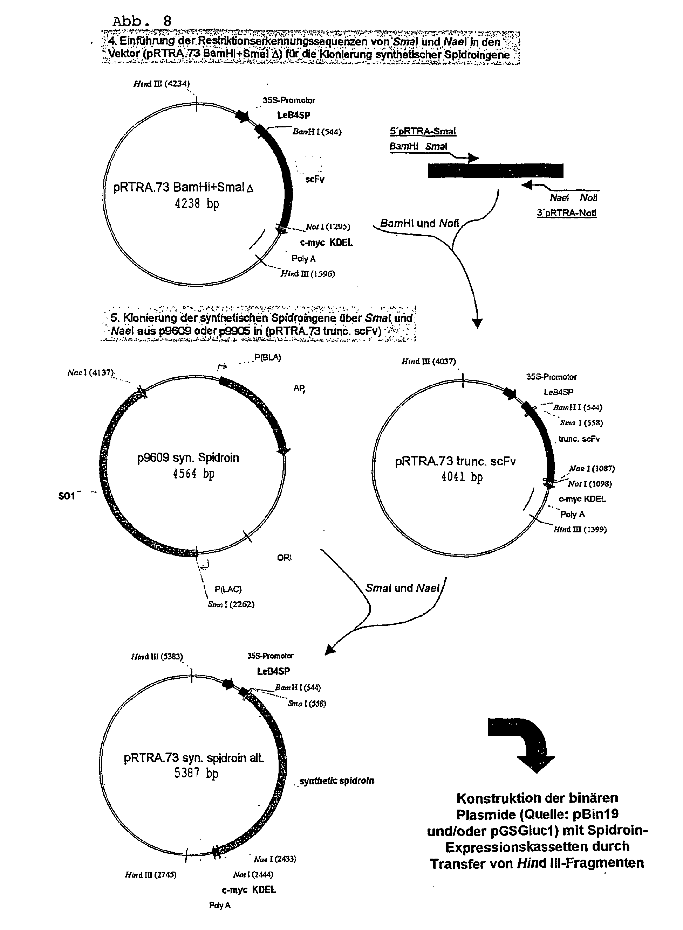

Da die erfindungsgemäßen DNA-Sequenzen aufgrund ihres repetitiven Charakters kaum unikale Restriktionsorte aufweisen, wurden die erfindungsgemäßen Vektoren bzw. die das synthetische Spinnenseidenprotein kodierenden Gene durch verschiedene Strategien entsprechend angepaßt (siehe Abbildungen 6 bis 8). Bei der Amplifizierung der erfindungsgemäßen DNA-Sequenzen durch PCR werden aufgrund des extrem repetitiven Charakters der erfindungsgemäßen DNA-Sequenzen vorzugsweise zunächst Oligonukleotide anligiert, welche dann als Matrizen für die nachfolgenden PCR-Reaktionen dienen (siehe Abbildung 7).

Des weiteren wird bei der vorliegenden Erfindung ein rekombinantes Spinnenseidenprotein bereitgestellt, das durch die erfindungsgemäße DNA-Sequenz kodiert wird. Dieses erfindungsgemäße synthetische Spinnenseidenprotein, vorzugsweise mit einem Molekulargewicht im Bereich von 10 bis 160 kDa, weist eine mindestens 94%ige Homologie zu Spidroin-Proteinen auf. Durch diese hohe Homologie mit den natürlichen Faserproteinen der Spinne und des Seidenspinners wird gewährleistet, daß die herausragenden mechanischen Eigenschaften der natürlichen Spinnenfäden erreicht werden, wenn die erfindungsgemäßen Proteine zu Fäden gesponnen werden.

Ferner weisen die erfindungsgemäßen Proteine überraschenderweise neuartige physikochemische Eigenschaften auf. So bleibt die Löslichkeit dieser erfindungsgemäßen synthetischen Faserproteine in wäßrigen Lösungen auch nach längerem Kochen außerordentlich gut erhalten. Gemeinsam mit der ebenfalls auftretenden Löslichkeit in organischen Lösungen und dem Fällungsverhalten bei hohen Salzkonzentrationen können diese neuen Eigenschaften der erfindungsgemäßen synthetischen Spinnenseidenproteine somit für die Entwicklung technisch durchführbarer Extraktions- und Reinigungsverfahren genützt werden. Diese Eigenschaften werden noch verstärkt, wenn die erfindungsgemäßen synthetischen Spinnenseidenproteine gezielt in bestimmten Kompartimenten, insbesondere im ER von transgenen Pflanzen akkumuliert werden.

Die Aminosäuresequenz des erfindungsgemäßen rekombinanten synthetischen Spinnenseidenproteins weist die Sequenz SEQ ID No. 38 auf. Die erfindungsgemäßen Spinnenseidenproteine können alternativ auch nach chemischen, dem Fachmann bekannten Methoden synthetisiert werden, eine rekombinante Herstellung ist jedoch bevorzugt.

Ferner betrifft die Erfindung ein Verfahren zur Herstellung von Spinnenseidenprotein-produzieronden Pflanzen bzw. Pflanzenzellen, umfassend die folgenden Schritte:
a) Herstellung eines wie vorstehend beschriebenen erfindungsgemäßen rekombinanten Nukleinsäuremoleküls;
b) Übertragung des Nukleinsäuremoleküls aus a) auf pflanzliche Zellen; und
c) gegebenenfalls die Regeneration fertiler Pflanzen aus den transformierten Pflanzenzellen.

Des weiteren betrifft die Erfindung Pflanzenzellen, die die erfindungsgemäßen Nukleinsäuremoleküle bzw. den erfindungsgemäßen Vektor enthalten. Die Erfindung betrifft ferner Ernteprodukte und Vermehrungsmaterial transgener Pflanzen sowie die transgenen Pflanzen selbst, die ein erfindungsgemäßes Nukleinsäuremolekül enthalten.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von *E*. *coli*-Zellen verwendet. Transformierte *E. coli*-Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente mit anderen DNA-Sequenzen verknüpft werden.

Für die Einführung von DNA in eine pflanzliche Wirtszelle stehen eine Vielzahl von Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmedium, die Fusion von Protoplasten, die Injektion, die Elektroporation, den direkten Gentransfer isolierter DNA in Protoplasten, die Einbringung von DNA mittels der biolistischen Methode sowie weitere Möglichkeiten die bereits seit mehreren Jahren gut etabliert sind und zum üblichen Repertoire des Fachmanns in der pflanzlichen Molekularbiologie bzw. Pflanzenbiotechnologie gehören.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muss mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediär Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E*. *coli* als auch in Agrobakterien replizieren. Sie enthalten ein Selektionsmsrkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden. Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt Der individuell gewählte Marker sollte daher die Selektion transformierter Zellen gegenüber Zellen, denen die eingeführte DNA fehlt, gestatten. Hierzu sind auch alternative Marker geeignet, wie nutretive Marker, Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgenen Pflanzen erwünscht sind, stehen dem Fachmann auch Strategien zur Verfügung, die eine nachträgliche Entfernung des Markergens erlauben, z.B. Cotransformation, Sequenz-spezifische Rekombinasen.

Die Regeneration der transgenen Pflanzen aus transgenen Pflanzenzellen erfolgt nach üblichen Regenerationsmethoden unter Verwendung bekannter Nährmedien. Die so erhaltenen Pflanzen können dann mittels üblicher Verfahren, einschließlich molekularbiologischer Methoden, wie PCR, Blot-Analysen, auf Anwesenheit der eingeführten Nukleinsäure, die ein synthetisches Spinnenseidenprotein kodiert, untersucht werden.

Bei der transgenen Pflanze bzw. der transgenen Pflanzenzelle kann es sich um jede beliebige monokotyle oder dikotyle Pflanze bzw. Pflanzenzelle handeln. Vorzugsweise handelt es sich um Nutzpflanzen bzw. Zellen von Nutzpflanzen. Besonders bevorzugt handelt es sich um transgene Pflanzen ausgewählt aus der Gruppe, bestehend aus der Tabakpflanze (*Nicotiana tabacum*) und der Kartoffelpflanze (*Solanum tuberosran*).

Die Expression des erfindungsgemäßen synthetischen Spinneseidenproteins in den erfindungsgemäßen Pflanzen bzw. in den erfindungsgemäßen Pflanzenzellen kann mit Hilfe herkömmlicher molekularbiologiseher und biochemischer Methoden nachgewiesen und verfolgt werden. Dem Fachmann sind diese Techniken bekannt und er ist problemlos in der Lage, eine geeignete Nachweismethode zu wählen, beispielsweise eine Northem-Blot-Analyse oder eine Southem-Blot-Analyse.

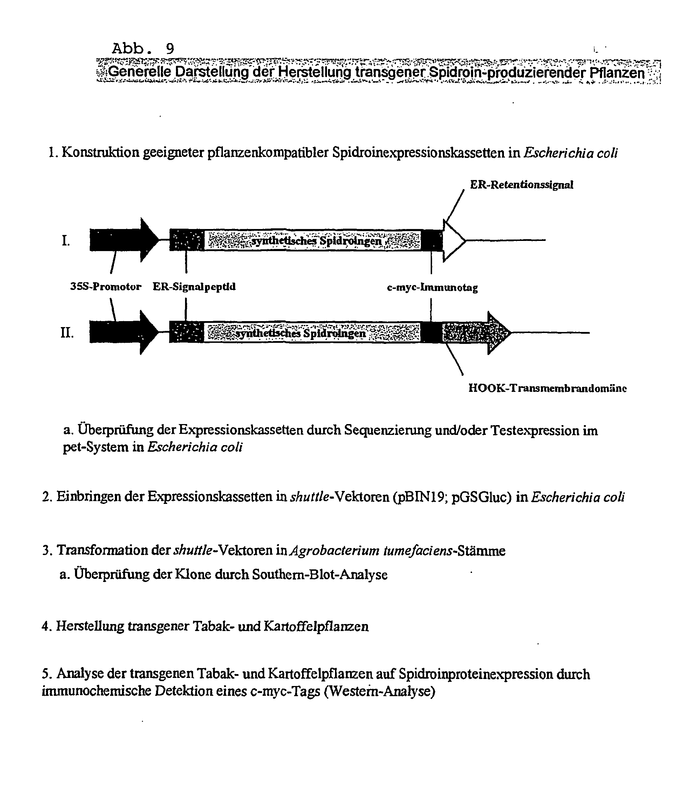

Ein Beispiel für die Herstellung von transgenen Spinnenseidenproteinproduzierenden Pflanzen ist in Abbildung 9 angegeben. Die durch PCR amplifizierten Sequenzen können möglicherweise frameshift-Mutationen enthalten. Deshalb müssen die erfindungsgemäßen Sequenzen vor der Erzeugung transgener Pflanzen überprüft werden. Dies kann durch Sequenzanalyse jeweils von den flankierenden Vektorsequenzen aus erfolgen. Längere Konstrukte über 1 kB können auf diese Weise nicht geprüft werden, da aufgrund der repetitiven Eigenschaften der erfindungsgemäßen DNA-Sequenzen interne Sequenzierungsprimer keine auswertbaren sicheren Sequenzen liefern. Aus diesem Grund wurden amplifizierte Spidroinsequenzen vorzugsweise in den bakteriellen Expressionsvektor pet23a (Novagen, Madison, USA) kloniert. Durch immunchemischen Nachweis der Expression können dann frameshift-Mutationen ausgeschlossen werden.

Die erfindungsgemäßen Nukleinsäuremoleküle bzw. Expressionskassetten werden erfindungsgemäß üblicherweise als HindIII-Fragmente in Shuttle-Vektoren wie pBIN, pCB301 und/oder pGSGLUC1 kloniert. Diese Shuttle-Vektoren werden vorzugsweise in *Agrobacterium tumefaciens* transformiert. Die Transformation von *Agrobacterium tumefaciens* wird üblicherweise durch Southern-Blot-Analyse und/oder PCR-Screening überprüft.

Die Erfindung betrifft ebenfalls Vermehrungsmaterial und Ernteprodukte der erfindungsgemäßen Pflanzen, beispielsweise Früchte, Samen, Knollen, Wurzelstöcke, Sämlinge, Stecklinge usw.

Ferner betrifft die Erfindung ein Verfahren zur Gewinnung von pflanzlichem Spinnenseidenprotein, umfassend die folgenden Schritte:
a) die Übertragung eines erfindungsgemäßen rekombinanten Nukleinsäuremoleküls oder Vektors, der eine DNA-Sequenz erhält, die für ein synthetisches Spinnenseidenprotein kodiert, auf Pflanzenzellen;
b) gegebenenfalls die Regeneration von Pflanzen aus den transformierten Pflanzenzellen;
c) die Verarbeitung der Pflanzenzellen aus a) bzw. der Pflanzen aus b) zur Gewinnung von pflanzlichem Spinnenseidenprotein.

Bei einem weiteren wesentlichen Aspekt der vorliegenden Erfindung werden Verfahren zur Gewinnung von rekombinant hergestellten Spinnenseidenproteinen bereitgestellt, die die Übertragung eines erfindungsgemäßen rekombinanten Nukleinsäuremoleküls oder Vektors, der eine DNA-Sequenz enthält, die für ein synthetisches Spinnenseidenprotein kodiert, auf beliebige Zellen, d.h. neben Pflanzenzellen beispielsweise auch bakterielle oder tierische Zellen, umfassen. Wesentliches Merkmal bei diesen erfindungsgemäßen Verfahren ist dabei der Schritt der Reinigung der rekombinant hergestellten Spinnenseidenproteine, bei dem u.a. deren besondere Eigenschaften hinsichtlich der Löslichkeit bei Erwärmung und/oder Säurezugabe ausgenutzt werden.

So erfolgt bei einer Ausführungsform des erfindungsgemäßen Verfahrens die Reinigung des rekombinant hergestellten Spinnenseidenproteins durch Hitzebehandlung des Zellextrakts, z.B. eines Pflanzensamen-Extrakts, und anschließende Abtrennung der denaturierten zelleigenen, z.B. der pflanzeneigenen Proteine beispielsweise durch Zeatrifugation. Dabei wird die Eigenschaft der rekombinant hergestellten Spinnenseidenproteine ausgenutzt, daß sie beim Erhitzen von wäßrigen Lösungen bis zum Siedepunkt löslich bleiben. Dagegen bleiben beispielsweise synthetische Faserproteine der Spinne und des Seidenspinners nach Expression in *Pichia pastoris* beim Erhitzen nur bis zu einer Temperatur von 63°C und dann nur für 10 Minuten in Lösung.

Bei einer weiteren Ausführungsform des erfindungsgemäßen Verfahrens zur Gewinnung von rekombinant hergestellten Spinnenseidenproteinen erfolgt die Reinigung durch Einstellung eines sauren pH mittels Zugabe von Säure, vorzugsweise Salzsäure zu dem Zellextrakt, beispielsweise zu dem Pflanzenextrakt. Der saure pH, insbesondere ein pH im Bereich von 1,0 bis 4,0, besonders bevorzugt im Bereich von 2,5 bis 3,5, am meisten bevorzugt ein pH von 3,0, wird dabei vorzugsweise für eine Dauer von mehreren Minuten, besonders bevorzugt etwa 30 Minuten, bei einer Temperatur unterhalb Raumtemperatur, vorzugsweise etwa 4°C, beibehalten. Wiederum wird eine nicht zu erwartende Eigenschaft der durch das erfindungsgemäße Verfahren gewonnenen Proteine ausgenutzt, nämlich daß sie beim Ansäuern, insbesondere bis zu einem pH von 3,0 bei 4°C in Lösung bleiben. Die zelleigenen, beispielsweise pflanzeneigenen Proteine fallen dagegen aus und werden insbesondere durch Zentrifugaion abgetrennt.

Die vorstehend beschriebenen Löslichkeitseigenschaften der nach dem erfindunpgemäßen Verfahren rekombinant hergestellten Spinnenseidenproteine sind sehr überraschend und waren in dieser Form nicht vorhersehbar und ermöglichen eine effiziente, schnelle und kostengünstige Reinigung bei deren Extraktion aus Zellen, insbesondere Pflanzenzellen.

Im erfindungsgemäßen Verfahren wird ein Nukleinsäuremolekül auf die Zellen übertragen, das zusätzlich eine Nukleinsäuresequenz umfaßt, die für ELP's kodiert. In diesem Fall erfolgt die Reinigung des rekombinant hergestellten Spinnenseidenproteins auf die folgende Weise: In einem ersten Schritt wird das Spinnenseiden-ELP-Fusionsprotein durch Hitzebehandlung des Rohextrakts angereichert. Überraschenderweise behalten die Fusionsproteine dabei die außerordentliche Löslichkeit der Spinnenseidenpmteine bei hohen Temperaturen bei. Ein Großteil der zelleigenen Proteine fällt bei dieser Temperaturerhöhung aus. Im nächsten Schritt werden die Spinnenseiden-ELP-Fusionsproteine durch weitere Temperaturerhöhung, vorzugsweise auf eine Temperatur von mindestens 60°C, ausgefüllt Vorzugsweise erfolgt die Ausfällung bei einer geeigneten Salz-Konzentration, beispielsweise einer NaCl-Konzentration von mindestens 0,5 M, vorzugsweise im Bereich von 1 M bis 2 M. Schließlich wird das ELP-Fragment, vorzugsweise durch Verdauung mit CNBr, abgespalten.

Durch das vorstehend beschriebene erfindungsgemäße Verfahren zur Gewinnung von rekombinant hergestelltem Spinnenseidenprotein können die Proteine in Pflanzen zu hohen Konzentrationen, vorzugsweise bis zu einer Expressionshöhe von etwa 4% des gesamten löslichen Proteins angereichert werden. Damit werden erstmals Verfahren bereitgestellt, die zur technisch realisierbaren Anreicherung von rekombinantern Spinnenseidenprotein verwendet werden können.

Die erfindungsgemäßn Spinnenseidenproteine können bei einem weiteren Aspekt der vorliegenden Erfindung zur Herstellung von synthetischen Faden sowie von Folien und Membranen verwendet werden. Insbesondere sind derartige Produkte für medizinische Anwendungen, insbesondere zum Vernähen von Wunden und/oder als Gerüste bzw. als Abdeckung für künstliche Organe, geeignet. Ferner können die aus den erfindungsgemäßen Spinnenseidenproteine hergestellten Folien und Membrane u.a. als Anheftungsflächen für kultivierte Zellen sowie zur Filterung verwendet werden.

Die vorliegende Erfindung wird in den nachfolgenden Beispielen, die der Veranschaulichung der Erfindung dienen und in keiner Weise als einschränkend zu verstehen sind, erläutert.

### Beispiele

Beispiel 1: Expression und stabile Akkumulation von synthetischen Faserproteinen der Spinne und des Seidenspinners im endoplasmatischen Retikulum von Blättern bzw. Knollen transgener Tabak- und Kartoffelpflanzen.

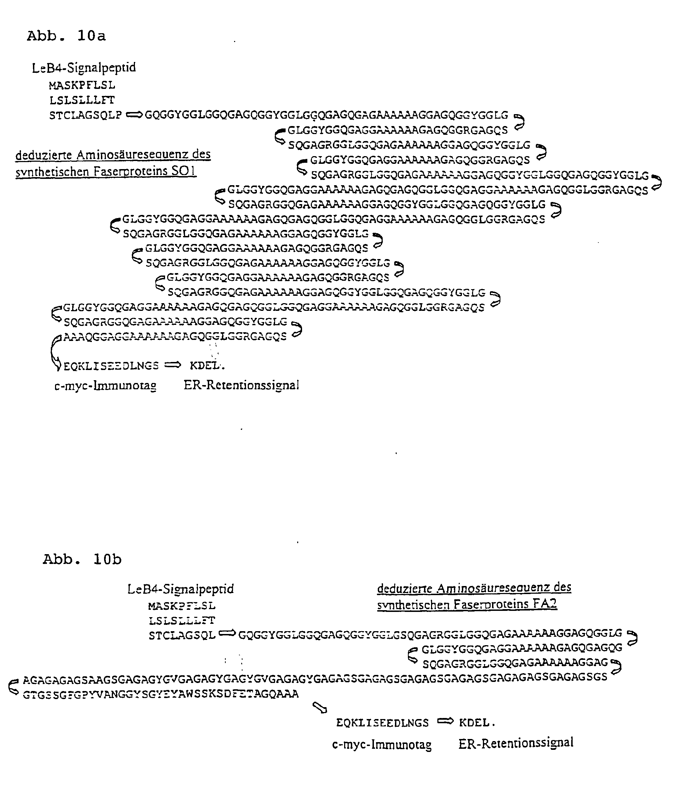

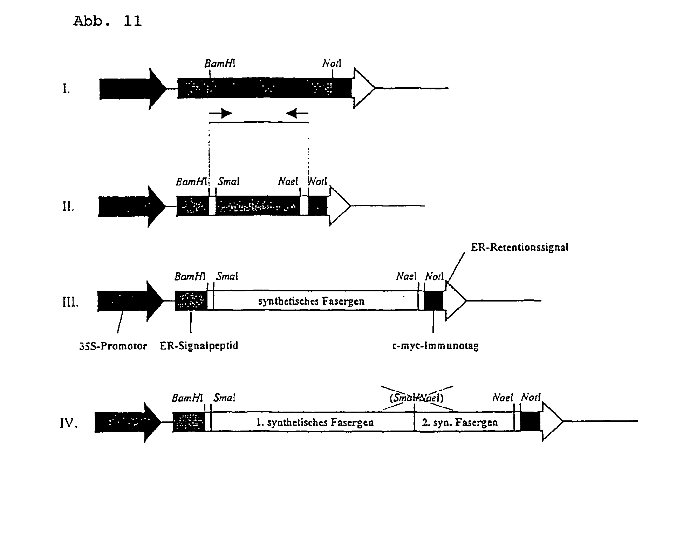

In den Abbildungen 10a und b sind Aminosäuresequenzen von synthetischen Spinnenseidenproteinen mit einer hohen Homologie zu dem Spidroin 1-Protein aus *Nephila clavipes* dargestellt, wobei der C-tetminale und nicht repetitive konstante Bereich nicht abgebildet sind. Diese synthetischen Spinnenseidenproteine bestehen aus Modulen, die wiederum aneinandergereihte Oligonukleotidsequenzen umfassen. Durch Kombination mehrerer Module wurden die verschiedenen synthetischen Gene assembliert, wobei auch Mischformen mit Fibroin 1-nachempfundenen Sequenzen erzeugt wurden.

In der nachfolgenden Tabelle 1 sind verschiedene Pflanzenexpressionskassetten aufgelistet, die für verschiedene erfindungsgemäße synthetische Faserproteine mit den Sequenzen SEQ ID No. 30 bis 40 kodieren.

**Tabelle 1**

| Pflanzenexpressions-Kassette | Anzahl der Aminosäuren (mit Leadersequenz) | Berechnetes Molekulargewicht (mit Leadersequenz) | Homologie |
|---|---|---|---|
| | | | |
| SB1 (SEQ ID No. 19) | Nr. 1 - 149 AS | 11 kDa | Spidroin 1 |
| SD1 (SEQ ID No. 21) | Nr. 2 - 182 AS | 13 kDa | Spidroin 1 |
| SA1 (SEQ ID No. 26) | Nr. 3 - 215 AS | 16 kDa | Spidroin 1 |
| SE1 (SEQ ID No. 20) | Nr. 4 - 275 AS | 20 kDa | Spidroin 1 |
| SF1 (SEQ ID No. 29) | Nr. 5 - 317 AS | 24 kDa | Spidroin 1 |
| SM12 (SEQ ID No. 28) | Nr. 6 - 410 AS | 31 kDa | Spidroin 1 |
| SO1 (SEQ ID No. 27) | Nr. 7 - 676 AS | 52 kDa | Spidroin 1 |
| SO1SM12 (SEQ ID No. 23) | Nr. 8 - 1035 AS | 82 kDa | Spidroin 1 |
| SO1SO1 (SEQ ID No. 22) | Nr. 9 - 1301 AS | 102 kDa | Spidroin 1 |
| SO1SO1SO1 (SEQ ID No. 24) | Nr. 10 - 1926 AS | 151 kDa | Spidroin 1 |
| FA2 (SEQ ID No. 25) | Nr. 11 - 264 AS | 20 kDa | Spidroin 1 und Fibroin |

Durch eine N-terminale Signalpeptidsequenz und eine C-terminale ER-Retentionssequenz (KDEL) wurde der zielgerichtete Transport und die Akkumulation der erfindungsgemäßen Sequenzen im endoplasmatischen Retikulum von Zellen transgener Pflanzen erreicht. Eine Nachweissequenz in Form eines c-myc-Tags am C-terminalen Ende der transgenen synthetischen Faserproteine erlaubt den Nachweis der transgenen Produkte in Pflanzenextrakten.

Die Kassetten SO1 und FA2 sind beispielhaft in den Abbildungen 10a und 10b im Detail dargestellt. Nach demselben Aufbauprinzip wurden die Pflamzenexpressionskassetten SB1, SD1, SA1, SE1, SF1, SM12, SO1SM12, SO1SO1 und SO1SO1SO1 erstellt. Durch Variation der Grundmodulwiederholungen entstehen synthetische Faserproteine verschiedener Aminosäureanzahl und entsprechend unterschiedlichen Molekulargewichts (siehe Tabelle 1).

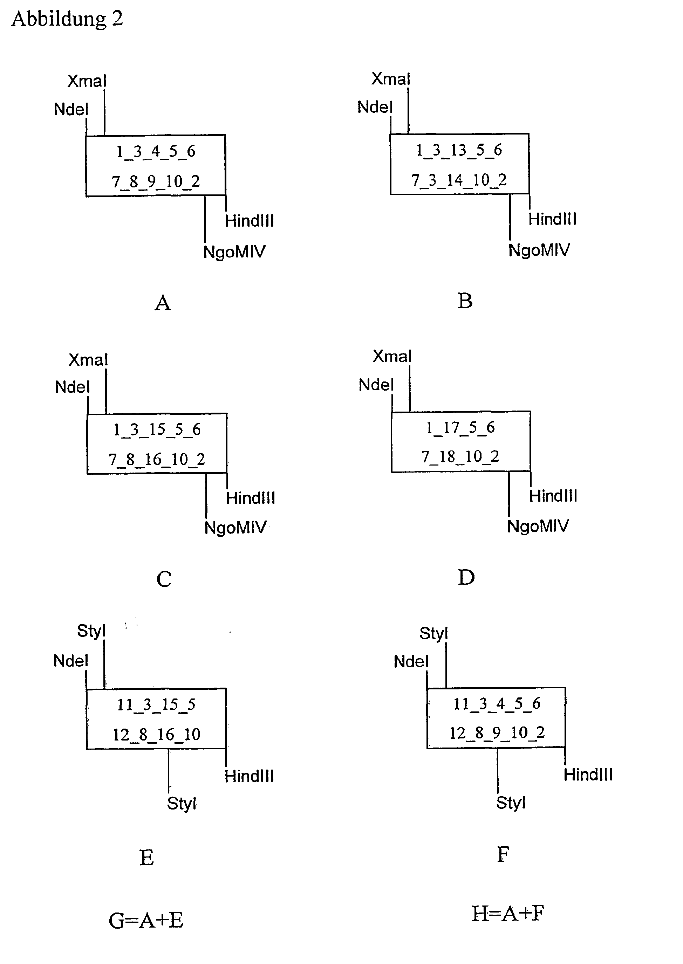

Die Abbildung 2 beschreibt schematisch den Weg zur Einstellung der oben genannten Konstrukte. Zur direkten Klonierung der erfindungsgemäßen synthetischen Faserproteingene wurden die Schnittstellen SmaI und NaeI eingeführt. Dazu wurde ein PCR-Produkt, welches die entsprechenden Schnittstellen enthielt, mit der Primerkombination 5'-pRTRA-SmaI und 3'-pRTRA-NotI in das Plasmid pRTRA ScFv SmaIΔ/BamHIΔ über BamHI und NotI kloniert. Synthetische Faserproteingene wurden aus den Faserproteingenderivaten der Plasmide 9905 oder 9609 in den Vektor pRTRA.7/3-Platzhalter kloniert. Durch die Wahl der Restriktionsendonukleaseerkennungssequenzen am 5'- und 3'-Ende der synthetischen Faserproteingene (SmaI und NaeI) sind diese frei miteinander kombinierbar, und größere Faserproteingene können erfindungsgemäß in einem Klonierungsschritt assembliert werden.

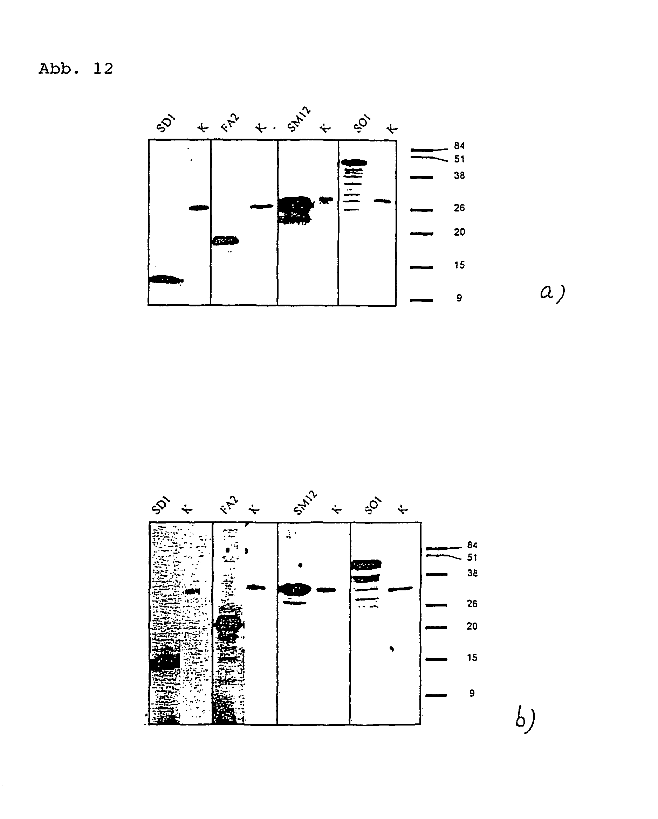

Auf diese Weise wurden transgene synthetische Spinnenseidenproteine zu hohen Konzentrationen im endoplasmatischen Retikulum transgener Tabak- und Kartoffelpflanzen akkumuliert (siehe Abbildungen 12a und 12b). In der folgenden Tabelle 2 ist die maximale Akkumulationshöhe von erfindungsgemäßen synthetischen Spinnenseidenproteinen im ER von Blättern transgener Tabak- und Kartoffelpflanzen dargestellt. Die Abschätzung der Anreicherung der transgenen synthetischen Faserproteine erfolgte über einen Vergleich mit transgenen rekombinanten Antikörpern, die auf identische Weise mit dem gleichen Tag versehen wurden. Damit wird erstmals eine Akkumulation von Spinnenseidenproteinen in Pflanzen am Beispiel von Kartoffeln und Tabak beschrieben.

**Tabelle 2**

| | **Faserprotein** | | | |
|---|---|---|---|---|
| | SD1 | SM12 | SO1 | FA2 |
| Tabak Akkumulationsmenge in Prozent Gesamtprotein | ∼ 0,5 % | ∼ 0,5 % | ∼ 0,5 % | ∼ 0,5 % |
| Kartoffel Akkumulationsruenge in Prozent Gesamtprotein | ∼ 0,5 % | ∼ 0,5 % | ∼ 0,5 % | ∼ 0,5 % |

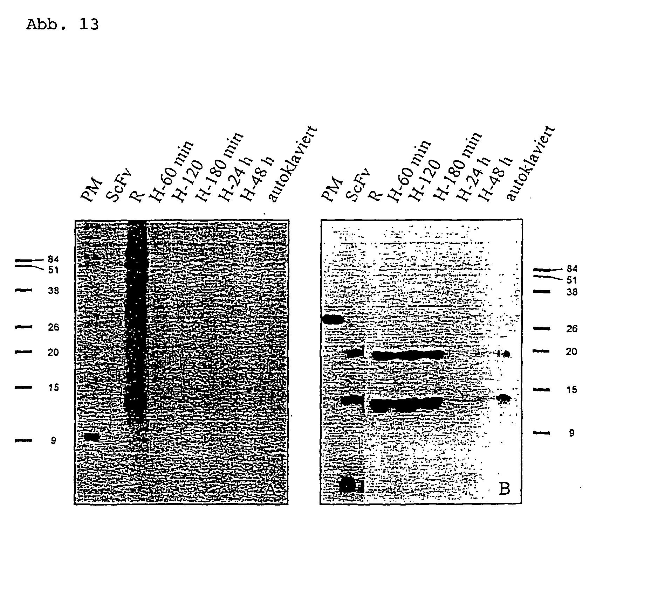

Eine definierte Menge des faserproteinhaltigen Gesamtproteinextrakts (40 µg) und eine definierte Menge eines Referenzproteins mit c-myc-Immunotag (50 ng ScFv) wurden mittels SDS-Gelelektrophorese aufgetrennt, und synthetische Faserproteine und Referenzproteine wurden im Western Blot durch einen Anti-c-myc-Antikörper nachgewiesen (siehe Abbildungen 12 und 13). Die prozentualen Angaben resultieren aus dem Vergleich zwischen der Bandenintensität der Referenzproteine und der Bandenintensität der synthetischen erfindungsgemäßen Spinnenseidenproteine und stellen geschätzte Werte dar. Größenunterschiede der synthetischen Faserproteine und des Referenzproteins wurden berücksichtigt. Mögliche Unterschiede in der Markierungseffizienz können nahezu ausgeschlossen werden.

In Abbildung 13 ist die Hitzestabilität von verschiedenen erfindungsgemäßen synthetischen Spinnenseidenproteinen in pflanzlichen Extrakten dargestellt. Überraschenderweise bleiben die erfindungsgemäßen Spinnenseidenproteine auch bei einer längeren Hitzebehandlung von 3 h in Lösung (Vergleich der Referenzprobe R zu den Proben H-60 min, H-120 min und H-180 min). Die übrigen pflanzlichen Proteine werden zu mehr als 90 % denaturiert und können durch Zentrifugation einfach abgetrennt werden (Abbildung 13a: Vergleich der Probe R zu H-60 min). Diese ungewöhnlichen Eigenschaften der erfindungsgemäßen synthetischen Spinnenseidenproteine, die unter anderem durch ihre Aminosäuresequenz und ihre Faltung im pflanzlichen ER bedingt sind, ermöglichen die Entwicklung von großtechnisch realisierbaren und kostengünstigen Reinigungsstrategien.

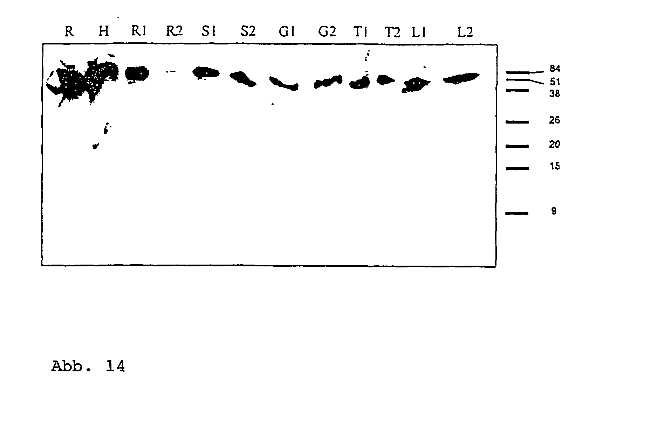

In Abbildung 14 wird die Löslichkeit von synthetischen Faserproteinen aus transgenen Pflanzen dargestellt. Im Gegensatz zu den im Stand der Technik beschriebenen bakteriell exprimierten synthetischen Faserproteinen weisen die erfinduagsgemäßen Spinnenseidenproteine eine überraschend gute Löslichkeit in wäßrigen Puffern auf (R1, R2 = Tris-Puffer; T1, T2 = Phosphatpuffer). Auch diese Eigenschaften beruhen unter anderem auf der Aminosäuresequenz und insbesondere auf der Faltung im endoplasmatischen Retikulum pflanzlicher Zellen.

Beispiel 2: Expression und stabile Akkumulation von synthetischen Spinnenseidenproteinen im Plasmalemma von Blättern transgener Tabak- und Kartoffelptlanzen.

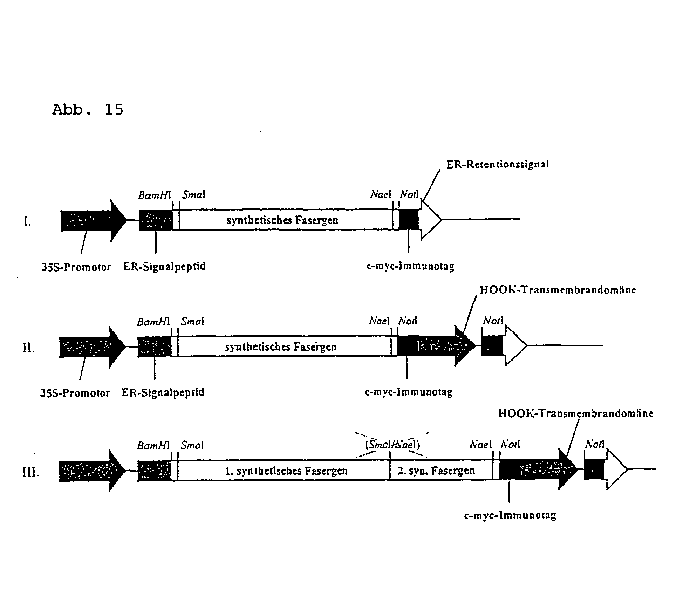

In diesem Beispiel wird die membranassoziierte Akkumulation von erfindungsgemäßen Spinnenseidenproteinen in transgenen Tabak- und Kartoffelpflanzen beschrieben. Dabei wurden ausgehend von den in Beispiel 1 beschriebenen Konstrukten Fusionsgene hergestellt, die für ein Spinnenseidenprotein und für eine Membrandomäne kodieren. Das allgemeine Schema dieser Konstruktionen ist in Abbildung 15 dargestellt. Dabei wurde aus dem Plasmid pRT-HOOK ein NotI-Fragment isoliert, welches sowohl für die HOOK-Domäne als auch für einen c-myc-Immunotag kodiert, welches dann in Spinnenseidenproteingen-tragende Derivate des Vektors pRTA.7/3 kloniert wurde. Durch die Wahl der Restriktionsendonukleaseerlaennungssequenzen am 5'- und 3'-Ende der synthetischen Spinnenseidenproteingene (SmaI und NaeI) sind diese wiederum miteinander kombinierbar, wodurch größere Faserproteingene in einem Klonierungsschritt assimiliert werden können.

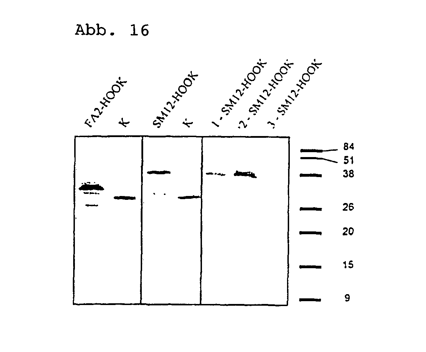

Abbildung 16 zeigt die Expression der vorstehend beschriebenen Gene in transgenen Tabak- und Kartoffelpflanzen. Wie aus dem Vergleich der Proben 1, 2 und 3 in dieser Abbildung ersichtlich ist, sind diese transgenen Spinnenseidenproteine im Gegensatz zu den im Beispiel 1 beschriebenen erfindungsgemäßen Proteinen in der wäßrigen Phase nicht löslich. Auch diese Eigenschaft kann für die Entwicklung von Reinigungsstrategien ausgenutzt werden.

Beispiel 3: Gezielte Veränderung der Löslichkeit von Spinnenseidenproteinen durch Fusion mit elastin-like peptides.

In einem ersten Schritt wurde gezeigt, daß Fusionen mit elastin-like peptides auch bei bakteriell exprimierten Spinnenseidenproteinen zu einer gezielten Veränderung des Löslichkeitsverhaltens in Abhängigkeit von Temperatur und Konzentration führen.

Eine entsprechende Expressionskassette ist in Abbildung 5 dargestellt. Beispiele für ELP mit 10, 20, 30, 40, 60, 70 und 100 Pentamereinheiten sind in den Sequenzen SEQ ID No. 41 bis 47 angegeben. Beispiele für DNA-Sequenzen und Aminosäuresequen in Form des Konstrukts SM12-70xELP als Pflanzenexpressionskassette bzw. als Expressionskassette für *E. coli* sind in den Sequenzen SEQ ID No. 48-51 bzw. in den Abbildungen 19 bis 22 angegeben.

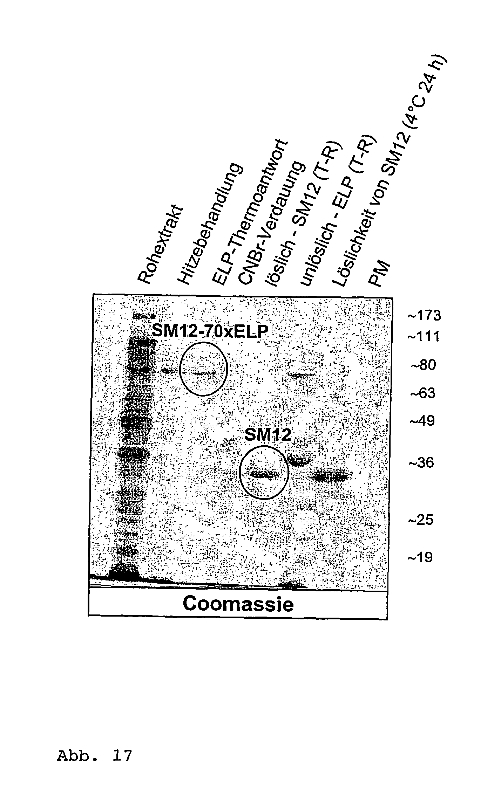

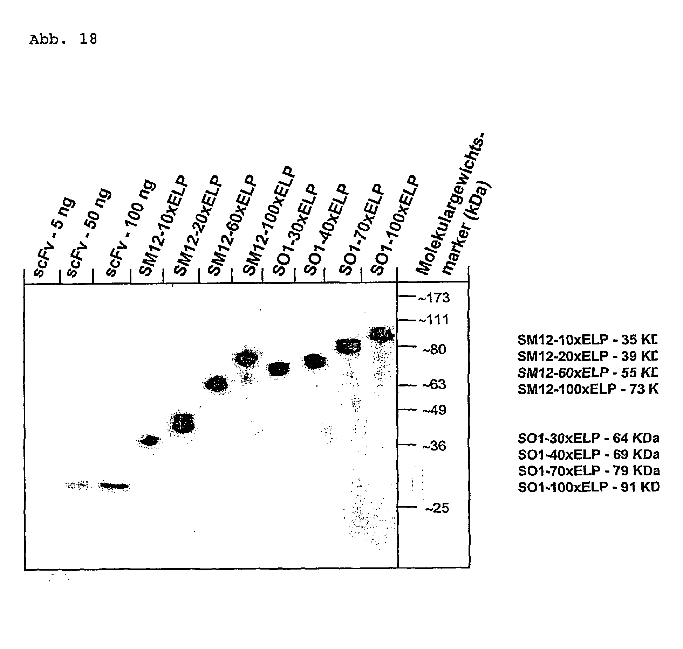

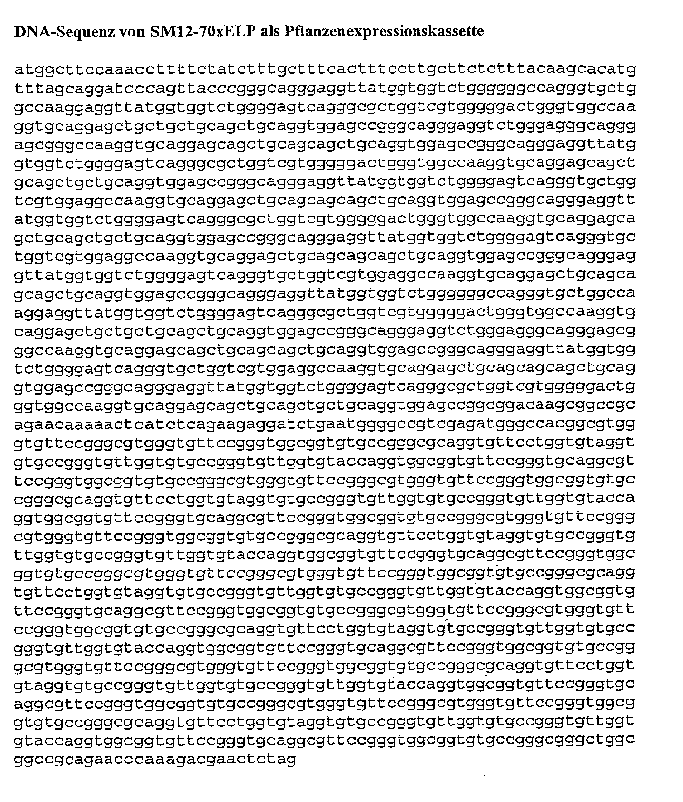

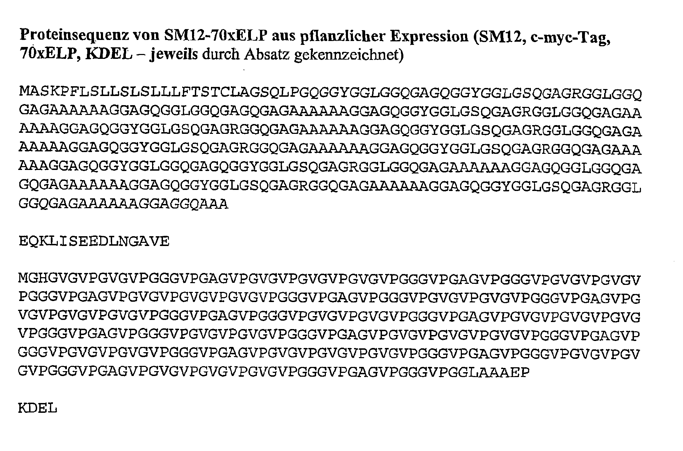

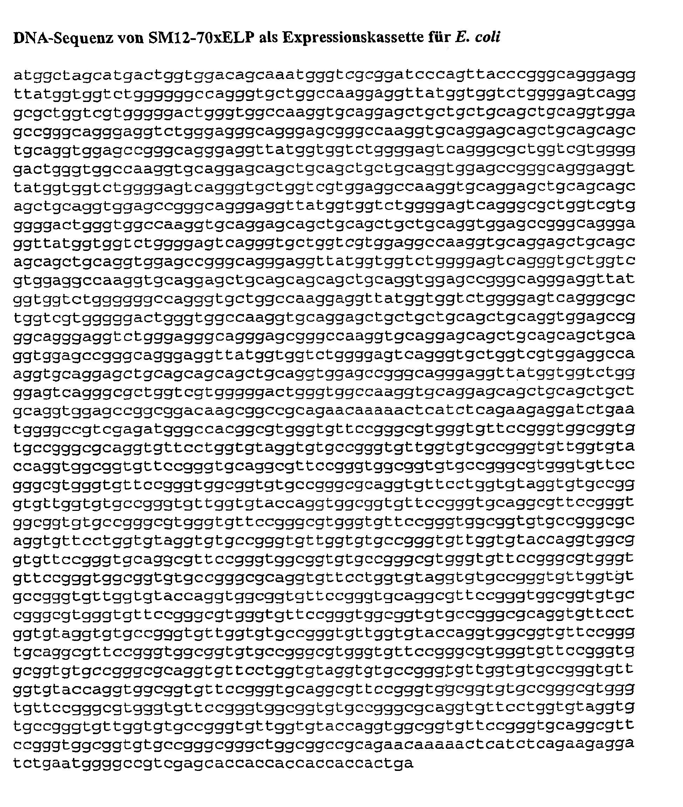

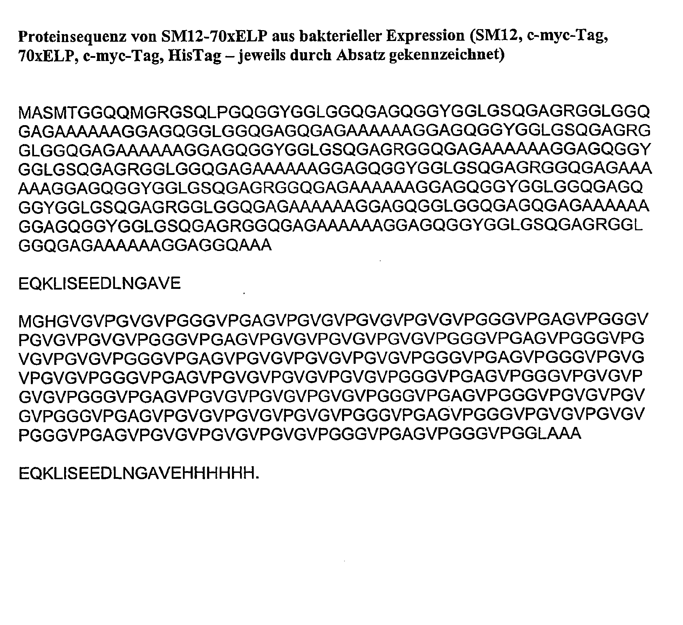

In Abbildung 17 wird die gelelekdrophoretische Analyse eines solchen Reinigungsverfahrens dargestellt. Durch Hitzebehandlung des Rohextrakts wurde das Spinnenseiden-ELP-Fusionsprotein angereichert. Überraschenderweise behielten die Fusionsproteine die außerordentliche Löslichkeit der Spinnenseidenproteine bei hohen Temperaturen bei. Ein Großteil der *E. coli-*Proteine wurde bei diesen Temperaturen ausgefällt.

Nach starker Konzentration des angereicherten Spinnenseidenproteinextrakts wurde das Extrakt einer Temperatur von 60°C ausgesetzt, woraufhin das ELP-Spinnenseidenprotein ausfiel und pelletiert wurde. Das Pellet wurde bei Raumtemperatur in Wasser gelöst, und unlösliche Bestandteile wurden durch Pelletieren entfernt.

Anschließend wurde die Spinnenseidenproteinfraktion lyophilisiert und durch Cyanbromidspaltung verdaut. Die Cyanbromidspaltung wurde durch den MethioninRest zwischen dem Spinnenseidenprotein und dem ELP-Peptid ermöglicht.

Anschließend wurde erneut lyophilisiert und in wäßrigem Puffer gelöst Dann erfolgte eine starke Konzentration, wobei das abgespaltene ELP-Fragment (ELP(T-R); siehe Abbildung 2) ausfiel und durch Pelletieren entfernt wurde. Das Spinnenseidenprotein blieb dabei in Lösung (SM12(T-R); siehe Abbildung 17). Die Löslichkeit blieb für einen längeren Zeitraum erhalten, bei SM12 bei 4°C für 24 H. Die Identität des auf diese Weise gereinigten Spinnenseidenproteins wurde durch Peptidsequenzierung des N-terminalen Endes gezeigt.

In einem zweiten Schritt wurden Spinnenseidenproteine als ELP-Fusionen im endoplasmatischen Retikulum von transgenen Tabakpflanzen akkumuliert. Der prinzipielle Aufbau dieser Expressionskassetten ist ebenfalls in Abbildung 5 dargestellt. Diese Fusionsproteine mit Molekulargewichten von 35.000 Dalton bis 100.000 Dalton wurden sämtlich in Pflanzen zu hohen Konzentrationen mit einer Expressionshöhe von etwa 4 % des gesamt löslichen Proteins angereichert

Allgemeine molekularbiologische Verfahren
- Klonierungen: Restriktionsspaltungen wurden in 100 µl Endvolumen vorgenommen. Standardmäßig wurde 10 µg Plasmid-DNA, 10 u je Restriktionsendonuklease, 10 µl eines geeigneten Puffers (10x) eingesetzt. DNA-Fragmente wurden durch Gelelektrophorese voneinander getrennt und gegebenenfalls per DNA-Gelexbraktion gereinigt. Für Ligationen wurde das zu klonierende DNA-Fragment (Insert) in dreifachem molaren Überschuß zum Vektorfragment eingesetzt. Sticky-end Ligationen wurden in einer Stunde und blunt-end Ligationen in 12 h bei 4°C mit 1 u Ligase durchgerührt. DNA wurde sowohl in Zellen von *E. coli* als auch von *A. tumefaciens* durch Elektroporation eingebracht. Transformanten wurden auf geeigneten Nährböden mit Antibiotikazusatz (Ampicillin oder Kanamycin) selektiert.
- PCR: PCR Reaktionen wurden in 50 µl Endvolumen vorgenommen. Standardmäßig wurde 100 ng Template-DNA, 100 pmol eines jeden Primer, 1 µl dNTP's (10 mM) und 5 µl eines geeigneten Puffers, und 1 u Tfl- oder Taq-DNA-Polymerase eingesetzt. Folgende Bedingungen wurden für eine PCR-Reaktion gewählt: 2 min 95°C, dann 30 Zyklen á 45 sec 95°C, 45 sec 50°C oder 55°C, 1 min 72°C, gefolgt von 5 min 72°C.
- Expression und Akkumulation in Tabak- und Kartoffelpflanzen: Transgene Pflanzen wurden im Brutraum bei gleichmäßiger Beleuchtung und ca. 20°C auf geeigneten Nährmedien mit Antibiotikazusatz (Kanamycin, Rifampicin und Carbenicillin) selektiert. Nach Bewurzelung wurden sie im Gewächshaus in erdehaltigen Töpfen weiter wachsen gelassen.

Im übrigen können die im Rahmen dieser Erfindung eingesetzten molekularbiologischen und biochemischen Techniken in gängigen Laborhandbüchern nachgeschlagen werden, wie z.B. in Sambrook et al. (1989) Molecular Cloning: A Laboratory Manual, 2. Auflage, Cold Spring Harbour Laboratory Press, Cold Spring Harbour, New York.

### Abbildungen

### Abbildung 1:

Oligonukleotid-Sequenzen, die für Spidroin-typische kurze Aminosäurerepeats kodieren.

### Abbildung 2:

Aneinanderreihung von Oligonukleotid-Sequenzen zum Aufbau von Modulen der erfindungsgemäßen DNA-Sequenzen.

### Abbildung 3:

Aufbau der erfindungsgemäßen DNA-Sequenzen aus Modulen.

### Abbildung 4:

Klonierung des Gens der Transmembrandomäne von HOOK mit *Not*I aus (pRT-HOOK) in (pRTA.73 syn.spidroin).

### Abbildung 5:

Schematische Darstellung der Spidroin-ELP-Expressionskassetten. xELP-Einheiten: 10, 20, 30, 40, 60, 70 oder 100 Pentamere (Val-Pro-Gly-Val-Gly). Das Methionin zwischen dem Spinnenseidenprotein und dem ELP-Peptid ermöglicht die Cyanbromidspaltung.

### Abbildung 6:

Veränderung einer Base in der Erkennungssequenz von *Bam*HI (Position 1332) durch gezielte Mutagenese.

### Abbildung 7:

Vorbereiten von (pRTRA.73, *Bam*HIΔ) auf die direkte Klonierung der synthetischen Spidroingene aus p9905 oder p9609 - Aufheben der *Sma*I-Erkennungssequenz (Position 463).

### Abbildung 8:

Einführung der Restriktionserkennungssequenzen von SmaI und NaeI in den Vektor (pRTRA.73, *Bam*HIΔ+SmaIΔ) für die Klonierung synthetischer Spidroingene.

### Abbildung 9:

Allgemeine Darstellung der Herstellung transgener Spinnseidenproteinproduzierender Pflanzen.

### Abbildung 10:

(a) Darstellung des modulhaften Aufbaus der erfindungsgemäßen Spinnenseidenproteine am Beispiel der SO1-Sequenz. Aminosäuren 1-28: LeB4-Signalpeptid; Aminosäuren 29 - 659: synthetische Spinnenseidenproteinsequenz; Aminosäuren 660 - 672: c-myc-Tag; Aminosäuren 673 - 676 ER-Retentionssignal.

Anordnung der Sequenzmodule nach der in Simmons et al., 1996. Molecular orientation and two-component nature of the cristalline fraction of spider dragline silk. Science 271: 84 - 87 angegebenen Originalsequenz.
(b) Darstellung des modulhaften Aufbaus des synthetischen Faserhybridproteins FA2. Aminosäuren 1 - 28: LeB4-Signalpeptid; Aminosäuren 28 -130: synthetische Faserproteinsequenz der Spinne; Aminosäuren 131 - 247: synthetische Faserproteinsequenz des Seidenspinners; Aminosäuren 248 - 260: c-myc-Tag; Aminosäuren 261 - 264: ER-Retentionssignal.

### Abbildung 11:

Schematische Darstellung der Erstellung von Genkassetten für die Akkumulation von synthetischen Faserproteinen der Spinne und des Seidenspinners im ER von transgenen Pflanzen.

### Abbildung 12:

(a) Expression der synthetischen Faserproteine der Spinne (SD1, SM12, SO1) bzw. des Hybrids aus Spinne und Seidenspinner (FA2) in Blättern von transgenen Tabakpflanzen. Analysiert wurden jeweils 40 µg Gesamtprotein in SDS-Probenpuffer. SD1: 13kDa; FA2: 20 kDa; SM12: 31 kDa; SO1: 52 kDa; K: Positivkontrolle 50 ng ScFv.
(b) Expression der synthetischen Faserproteine der Spinne (SD1, SM12, SO1) bzw. des Hybrids aus Spinne und Seidenspinner (FA2) in transgenen Kartoffelpflanzen.

Analysiert wurden ebenfalls jeweils 40 µg Gesamtprotein in SDS-Probenpuffer. SD1: 13 kDa; FA2: 20 kDa; SM12: 31 kDa; SO1: 52 kDa; K: Positivkontrolle 50 ng ScFv.

### Abbildung 13:

Darstellung der Hitzebeständigkeit der synthetischen Faserproteine der Spinne und des Seidenspinners anhand der Konstrukte SD1 und FA2. A: Coomassie-gefärbtes Gel. B: Immunochemischer Nachweis der synthetischen Faserproteine SD1 und FA2 mittels Anti-c-myc-Antikörper. PM: Proteinmarker; ScFv: 50 ng ScFv; R: wäßriges Pflanzenextrakt von Blättern von transgenen Pflanzen für SD1 und FA2; H: Hitzeschritt 60 min, 120 min, 180 min, 24 H und 48 H bei 90°C.

Bei der Hitzebehandlung ausgefallene Pflanzenextraktbestandteile wurden durch Zentrifugieren abgetrennt.

### Abbildung 14:

Untersuchung der Lösungseigenschaften und Stabilität des synthetischen Spinnenseidenproteins SO1 nach Ammoniumsulfatfällung.

10 g Blattmaterial wurden in Stickstoff schockgefroren, zermörsert, in 20 ml Rohextraktpuffer aufgenommen, für 30 min bei 38°C geschüttelt, und unlösliche Bestandteile wurden durch Zentrifugieren entfernt (30 min, 10.000 rpm). Anschließend wurde der Überstand (R) für 10 min auf 90°C erhitzt und das Präzipitat durch Zentrifugieren entfernt (30 min, 10.000 rpm). Der Überstand (H) wurde mit 20 % Ammoniumsulfat versetzt, 4 h bei Raumtemperatur gerollt und das Präzipitat durch Zentrifugieren für 60 min bei 4000 rpm und 4°C entfernt. Der Überstand wurde auf 30 % Endkonzentration Ammoniumsulfat eingestellt und über Nacht bei Raumtemperatur gerollt. Die Lösung wurde in 5 Aliquote getrennt und das Präzipitat durch Zentrifugieren entfernt (60 min, 4000 rpm, 4°C). Die Überstände wurden verworfen und die verbliebenen Pellets in folgenden Lösungen aufgenommen: R1: Rohextraktpuffer (50 mM Tris/HCl pH 8,0; 100 mM NaCl, 10 mM MgSO₄); S: SDS-Probenpnffer; G: 0,1 M Phosphatpuffer, 0,01 M Tris/HCl, 6 M Guanidiniumhydrochlorid/HCl pH 6,5; T: 1 x PBS, 1 % TritonX-100; L: LiBr.

Die Ansätze wurden für 1 h bei 37°C geschüttelt, und durch Zentrifugieren wurden unlösliche Bestandteile entfernt (30 min, 10.000 rpm). Anschließend wurde ein Aliquot jedes Ansatzes entnommen und für die SDS-Gelelektrophorese vorbereitet (R1, S1, G1, T1, L1). Die Ansätze wurden nun 36 h bei Raumtemperatur stehen gelassen. Durch Zentrifugieren wurden unlösliche Bestandteile entfernt (30 min, 10.000 rpm). Wiederum wurde ein Aliquot jedes Ansatzes entnommen und für die SDS-Gelelektrophorese vorbereitet (R2, S2, G2, T2, L2). Es wurden jeweils vergleichbare Volumina analysiert.

### Abbildung 15:

Schematische Darstellung der Konstruktion von Genkassetten für die Akkumulation von Plasmalemma-tändigen synthetischen Faserproteinen der Spinne und des Seidenspinners in transgenen Pflanzen.

### Abbildung 16:

Expression der Faserfusionsproteine SM12-HOOK, SO1-HOOK und FA2-HOOK in Blättern von transgenen Kartoffelpflanzen.

### Abbildung 17:

Gelelektrophoretische Analyse der Anreicherung von bakteriell exprimierten Spinnenseidenproteinen nach Fusion mit ELP's. Spinnenseidenprotein: 30.000 Dalton.

### Abbildung 18:

Western Blot-Analyse der Expression von Spinnenseiden-ELP-Fusionsproteinen in transgenen Tabakpflanzen. Jeweils 2,5 µg Gesamtpflanzenprotein wurden getrennt und die Spinnenseidenproteine auf dem Western Blot durch ECL nachgewiesen. Durch Vergleich mit dem Standard wird die Spinnenseidenproteinkonzentrstion auf mindestens 4 % des gesamtlöslichen Proteins geschätzt.

### Abbildung 19:

DNA-Sequenz von SM12-70xELP als Pflanzenexpressionskassette.

### Abbildung 20:

Proteinsequenz von SM12-70xELP aus pflanzlicher Expression (SM12, c-myc-Tag, 70xELP, KDEL - jeweils durch Absatz gekennzeichnet),

### Abbildung 21:

DNA-Sequenz von SM12-70xELP als Expressionskassette für *E. coli*.

### Abbildung 22:

Proteinsequenz von SM12-70xELP aus bakterieller Expression (SM12, c-myc-Tag, 70xELP, c-myc-Tag, HisTag - jeweils durch Absatz gekennzeichnet).

## Patentansprüche

1. DNA-Sequenz, die die in SEQ ID No. 27 angegebene Sequenz oder eine Sequenz, die zu der in SEQ ID No. 27 angegebenen Sequenz eine Sequenzidentität von mindestens 98% aufweist, umfasst und für ein synthetisches Spinnenseidenprotein kodiert, wobei die DNA-Sequenz in Fusion mit Nukleinsäuresequenzen vorliegt, die für 10 bis 100 Repeats des Pentapeptids Val-Pro-Gly-Xaa-Gly (ELP) kodieren, wobei Xaajede Aminosäure außer Prolin ist, und wobei das Spinnenseidenprotein beim Erhitzen von wässrigen Lösungen bis zum Siedepunkt löslich bleibt.

2. Rekombinantes Nukleinsäuremolekül, umfassend
a) eine DNA-Sequenz nach Anspruch 1 und
b) einen ubiquitär wirkenden Promotor.

3. Nukleinsäuremolekül nach Anspruch 2, zusätzlich umfassend mindestens eine Nukleinsäuresequenz, die für ein pflanzliches Signalpeptid kodiert.

4. Nukleinsäuremolekül nach Anspruch 3,
**dadurch gekennzeichnet, dass** das pflanzliche Signalpeptid den Transport in das endoplasmatische Retikulum (ER) gewährleistet.

5. Nukleinsäuremolekül nach Anspruch 3 oder 4,
**dadurch gekennzeichnet, dass** die für das pflanzliche Signalpeptid kodierende Nukleinsäuresequenz eine LeB4Sp-Sequenz ist.

6. Nukleinsäuremolekül nach einem der Ansprüche 2 bis 5, zusätzlich umfassend eine Nukleinsäuresequenz, die für ein ER-Retentionspeptid kodiert.

7. Nukleinsäuremolekül nach Anspruch 6,
**dadurch gekennzeichnet, dass** das ER-Retentionspeptid die Sequenz KDEL umfasst.

8. Nukleinsäuremolekül nach einem der Ansprüche 2 bis 5, zusätzlich umfassend eine Nukleinsäuresequenz, die für eine Transmembrandomäne kodiert.

9. Nukleinsäuremolekül nach Anspruch 8,
**dadurch gekennzeichnet, dass** die Nukleinsäuresequenz für die Transmembrandomäne des PDGF-Rezeptors kodiert.

10. Nukleinsäuremolekül nach einem der Ansprüche 2 bis 9, umfassend den CaMV 35S-Promotor.

11. Vektor, umfassend ein rekombinantes Nukleinsäuremolekül nach einem der Ansprüche 2 bis 10.

12. Mikroorganismus, enthaltend ein rekombinantes Nukleinsäuremolekül oder einen Vektor nach einem der Ansprüche 2 bis 11.

13. Rekombinantes Spinnenseidenprotein, kodiert durch eine DNA-Sequenz nach Anspruch 1.

14. Rekombinantes Spinnenseidenprotein, umfassend die in SEQ ID No. 38 angegebene Aminosäuresequenz, wobei die Aminosäuresequenz in Fusion mit 10 bis 100 Repeats des Pentapeptids Val-Pro-Gly-Xaa-Gly (ELP) vorliegt, wobei Xaa jede Aminosäure außer Prolin ist.

15. Verfahren zur Herstellung von Spinnenseidenprotein-produzierenden Pflanzen bzw. Pflanzenzellen, umfassend die folgenden Schritte:
a) Herstellung eines rekombinanten Nukleinsäuremoleküls nach einem der Ansprüche 2 bis 10;
b) Übertragung des Nukleinsäuremoleküls aus a) auf pflanzliche Zellen; und
c) ggf. die Regeneration fertiler Pflanzen aus den transformierten Pflanzenzellen.

16. Transgene Pflanzenzellen, enthaltend ein rekombinantes Nukleinsäuremolekül oder einen Vektor nach einem der Ansprüche 2 bis 11, oder hergestellt nach einem Verfahren nach Anspruch 15.

17. Transgene Pflanzen, enthaltend eine Pflanzenzelle nach Anspruch 16 oder hergestellt nach Anspruch 15, sowie transgene Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

18. Transgene Pflanzen nach Anspruch 17, ausgewählt aus der Gruppe, bestehend aus Tabakpflanze und Kartoffelpflanze.

19. Verfahren zur Gewinnung von pflanzlichem Spinnenseidenprotein, umfassend die folgenden Schritte:
a) die Übertragung eines rekombinanten Nukleinsäuremoleküls oder Vektors nach einem der Ansprüche 2 bis 11 auf Pflanzenzellen;
b) gegebenenfalls die Regeneration von Pflanzen aus den transformierten Pflanzenzellen; und
c) die Verarbeitung der Pflanzenzellen aus a) bzw. der Pflanzen aus b) zur Gewinnung von pflanzlichem Spinnenseidenprotein.

20. Verfahren zur Gewinnung von rekombinant hergestelltem Spinnenseidenprotein, umfassend die folgenden Schritte:
a) die Übertragung eines rekombinanten Nukleinsäuremoleküls oder Vektors, nach einem der Ansprüche 2 bis 11, auf Zellen;
b) die Reinigung des Spinnenseidenproteins durch Hitzebehandlung des Zellextrakts und anschließende Abtrennung der denaturierten zelleigenen Proteine.

21. Verfahren zur Gewinnung von rekombinant hergestelltem Spinnenseidenprotein, umfassend die folgenden Schritte:
a) die Übertragung eines rekombinanten Nukleinsäuremoleküls oder Vektors nach einem der Ansprüche 2 bis 11, auf Zellen;
b) die Reinigung des Spinnenseidenproteins durch Einstellung eines sauren pH, vorzugsweise eines pH im Bereich von 2,5 bis 3,5 mittels Zugabe von Säure, vorzugsweise Salzsäure zu dem Zellextrakt und anschließende Abtrennung der denaturierten zelleigenen Proteine.

22. Verfahren zur Gewinnung von rekombinant hergestelltem Spinnenseidenprotein, umfassend die folgenden Schritte:
a) die Übertragung eines rekombinanten Nukleinsäuremoleküls nach einem der Ansprüche 2 bis 10 auf Zellen;
b) die Reinigung des Spinnenseidenproteins auf folgende Weise:
- Anreicherung des Spinnenseiden-ELP-Fusionsproteins durch Hitzebehandlung des Zellextrakts;
- Ausfällung des Spinnenseiden-ELP-Fusionsproteins durch weitere Temperaturerhöhung, vorzugsweise auf eine Temperatur von mindestens 60°C, und vorzugsweise bei einer Salzkonzentration von 1 M bis 2 M; und
- Abspaltung des ELP-Fragments, vorzugsweise durch Verdauung mit CNBr.

23. Verfahren nach einem der Ansprüche 20 bis 22,
**dadurch gekennzeichnet, dass** die Zellen ausgewählt werden aus Pflanzenzellen, tierischen Zellen und bakteriellen Zellen.

24. Pflanzliches Spinnenseidenprotein, hergestellt nach einem Verfahren nach einem der Ansprüche 19 bis 23.

25. Verwendung der Spinnenseidenproteine nach einem der Ansprüche 13, 14 oder 24 zur Herstellung von synthetischen Fäden, Folien und/oder Membranen.

26. Verwendung nach Anspruch 25, wobei die Fäden, Folien und/oder Membrane für medizinische Anwendungen, insbesondere zum Vernähen von Wunden und/oder als Gerüste bzw. als Abdeckung für künstliche Organe, eingesetzt werden.

27. Verwendung nach Anspruch 26, wobei die Folien und/oder Membrane als Anheftungsflächen für kultivierte Zellen und/oder zur Filterung verwendet werden.

28. DNA-Sequenz nach Anspruch 1 oder Spinnenseidenprotein nach einem der Ansprüche 13, 14 oder 24, wobei das Eigenschaftsspektrum bezüglich mindestens einer Eigenschaft, ausgewählt aus Reißfestigkeit, Elastizität, Quellungsvermögen, Löslichkeitsverhalten, Säurestabilität, Hitzebeständigkeit gegenüber nativem Spinnenseidenprotein verändert ist.

## Claims

1. DNA sequence comprising the sequence identified in SEQ ID NO: 27 or a sequence having a sequence identity of at least 98% to the sequence identified in SEQ ID NO: 27 and which codes for a synthetic spider silk protein, wherein the DNA sequence is fused with nucleic acid sequences coding for 10 to 100 repeats of the pentapeptide Val-Pro-Gly-Xaa-Gly (ELP), wherein Xaa can be any amino acid except proline, and wherein the spider silk protein remains soluble upon heating of aqueous solutions up to the boiling point.

2. Recombinant nucleic acid molecule, comprising
a) a DNA sequence according to claim 1 and
b) an ubiquitously acting promoter.

3. The nucleic acid molecule according to claim 2, additionally comprising at least one nucleic acid sequence that codes for a plant signal peptide.

4. The nucleic acid molecule according to claim 3, **characterized in that** the plant signal peptide mediates the transport into the endoplasmatic reticulum (ER).

5. The nucleic acid molecule according to claim 3 or 4, **characterized in that** the nucleic acid sequence that codes for the plant signal peptide is a LeB4Sp sequence.

6. The nucleic acid molecule according to any of the claims 2 to 5, additionally comprising a nucleic acid sequence that codes for an ER retention peptide.

7. The nucleic acid molecule according to claim 6, **characterized in that** the ER retention peptide comprises the sequence KDEL.

8. The nucleic acid molecule according to any of the claims 2 to 5, additionally comprising a nucleic acid sequence that codes for a transmembrane domain.

9. The nucleic acid molecule according to claim 8, **characterized in that** the nucleic acid sequence codes for the transmembrane domain of the PDGF receptor.

10. The nucleic acid molecule according to any of the claims 2 to 9, comprising the CaMV 35S promoter.

11. A vector comprising a recombinant nucleic acid molecule according to any of the claims 2 to 10.

12. A microorganism containing a recombinant nucleic acid molecule or a vector according to any of the claims 2 to 11.

13. A recombinant spider silk protein, coded by a DNA sequence according to claim 1.

14. A recombinant spider silk protein, comprising the amino acid sequence identified in SEQ ID NO. 38, wherein the amino acid sequence is fused with 10 to 100 repeats of the pentapeptide Val-Pro-Gly-Xaa-Gly (ELP), wherein Xaa can be any amino acid except proline.

15. A method of manufacturing spider silk protein-producing plants or plant cells, comprising the following steps:
a) Manufacture of a recombinant nucleic acid molecule according to any of the claims 2 to 10;
b) transfer of the nucleic acid molecule from a) to plant cells; and
c) optionally, regeneration of fertile plants from the transformed plant cells.

16. Transgenic plant cells containing a recombinant nucleic acid molecule or a vector according to any of the claims 2 to 11, or produced in a method according to claim 15.

17. Transgenic plants containing a plant cell according to claim 16 or produced according to claim 15, as well as transgenic parts of these plants, transgenic harvest products and transgenic propagating material of these plants, such as protoplasts, plant cells, calli, seeds, tubers, cuttings, and the transgenic progeny of these plants.

18. Transgenic plants according to claim 17, selected from the group consisting of tobacco plants and potato plants.

19. A method of obtaining plant spider silk protein, comprising the following steps:
a) Transfer of a recombinant nucleic acid molecule or vector according to any of the claims 2 to 11 to plant cells;
b) optionally, regeneration of plants from the transformed plant cells; and
c) processing of the plant cells from a) or plants from b) to obtain plant spider silk protein.

20. A method of obtaining recombinantly manufactured spider silk protein, comprising the following steps:
a) Transfer of a recombinant nucleic acid molecule or vector according to any of the claims 2 to 11 to cells;
b) purification of the spider silk protein by heat-treating the cell extract and then separating the denatured proteins naturally occurring in the cells.

21. A method of obtaining recombinantly manufactured spider silk protein, comprising the following steps:
a) Transfer of a recombinant nucleic acid molecule or vector according to any of the claims 2 to 11 to cells;
b) purification of the spider silk protein by adjusting an acidic pH, preferably a pH ranging from 2.5 to 3.5, by adding acid, preferably hydrochloric acid, to the cell extract and then separating the denatured proteins naturally occurring in the cell.

22. A method of obtaining recombinantly manufactured spider silk protein, comprising the following steps:
a) Transfer of a recombinant nucleic acid molecule according to any of the claims 2 to 10 to cells;
b) purification of the spider silk protein as follows:
- enriching the spider silk-ELP fusion protein by heat-treating the cell extract,
- precipitating the spider silk-ELP fusion protein by further increasing the temperature, preferably to a temperature of at least 60 °C, and preferably at a salt concentration from 1 M to 2 M; and
- cleaving off the ELP fragment, preferably via digestion with CNBr.

23. A method according to any of the claims 20 to 22, **characterized in that** the cells are selected from among plant cells, animal cells and bacterial cells.

24. A plant spider silk protein, produced in a method according to any of the claims 19 to 23.

25. Use of the spider silk proteins according to any of the claims 13, 14 or 24 to manufacture synthetic threads, films and/or membranes.

26. Use according to claim 25, wherein the threads, films and/or membranes are used for medical purposes, in particular for closing wounds and/or as frames or covers for artificial organs.

27. Use according to claim 26, wherein the films and/or membranes are used as adhesion surfaces for cultivated cells and/or for filtering purposes.

28. The DNA sequence according to claim 1 or the spider silk protein according to any of the claims 13, 14 or 24, wherein the range of properties is altered compared to native spider silk protein with respect to at least one property, selected from among tensile strength, elasticity, swelling capacity, solubility behaviour, acid stability and heat resistance.

## Revendications

1. Séquence d'ADN comprenant la séquence donnée dans SEQ ID No : 27 ou une séquence qui a une identité de séquence d'au moins 98 % avec la séquence donnée dans SEQ ID No : 27, et codant pour une protéine de soie d'araignée, la séquence d'ADN étant présente en fusion avec des séquences d'acide nucléique codant pour 10 à 100 répétitions du penta-peptide Val-Pro-Gly-Xaa-Gly (ELP), dans lequel Xaa est un acide aminé quelconque à l'exception de proline, et dans lequel la protéine de soie d'araignée reste soluble jusqu'au point d'ébullition pendant le chauffage de solutions aqueuses.

2. Molécule d'acide nucléique recombinante comprenant
a) une séquence d'ADN selon la revendication 1 et
b) un promoteur agissant ubiquitairement.

3. Molécule d'acide nucléique selon la revendication 2, comprenant en outre au moins une séquence d'acide nucléique codant pour un peptide signal végétal.

4. Molécule d'acide nucléique selon la revendication 3, **caractérisée par le fait que** le peptide signal végétal assure le transport dans le réticulum endoplasmatique (ER).

5. Molécule d'acide nucléique selon la revendication 3 ou 4, **caractérisée par le fait que** l'acide nucléique codant pour le peptide signal végétal est une séquence LeB4Sp.

6. Molécule d'acide nucléique selon l'une des revendications 2 à 5, comprenant en outre au moins une séquence d'acide nucléique codant pour un peptide de rétention de l'ER.

7. Molécule d'acide nucléique selon la revendication 6, **caractérisée par le fait que** le peptide de rétention de l'ER comprend la séquence KDEL.

8. Molécule d'acide nucléique selon l'une des revendications 2 à 5, comprenant en outre une séquence d'acide nucléique codant pour un domaine transmembranaire.

9. Molécule d'acide nucléique selon la revendication 8, **caractérisée par le fait que** la séquence d'acide nucléique code pour le domaine transmembranaire du récepteur PDGF.

10. Molécule d'acide nucléique selon l'une des revendications 2 à 9, comprenant le promoteur CaMV 35S.

11. Vecteur comprenant une molécule d'acide nucléique recombinante selon l'une des revendications 2 à 10.

12. Microorganisme contenant une molécule d'acide nucléique recombinate ou un vecteur selon l'une des revendications 2 à 11.

13. Protéine de soie d'araignée recombinante codée par une séquence d'ADN selon la revendication 1.

14. Protéine de soie d'araignée recombinante comprenant la séquence d'acide aminé donnée dans SEQ ID No : 38, la séquence d'acide aminé étant présente en fusion avec 10 à 100 répétitions du penta-peptide Val-Pro-Gly-Xaa-Gly (ELP), dans lequel Xaa est un acide aminé quelconque à l'exception de proline.

15. Procédé pour la fabrication des plantes et des cellules végétales produisant de protéine de soie d'araignée, respectivement, comprenant les étapes suivantes :
a) la fabrication d'une molécule d'acide nucléique selon l'une des revendications 2 à 10;
b) la transformation de la molécule d'acide nucléique de a) sur des cellules végétales ; et
c) le cas échéant, la régénération des plantes fertiles à partir des cellules végétales transformées.

16. Cellules végétales transgéniques contenant une molécule d'acide nucléique recombinante ou un vecteur selon l'une des revendications 2 à 11 ou fabriquées selon un procédé selon la revendication 15.

17. Plantes transgéniques contenant une cellule végétale selon la revendication 16 ou fabriquées selon la revendication 15 ainsi que des parts transgéniques de ces plantes, des produits de récolte transgéniques et du matériau de propagation transgénique de ces plantes, comme des protoplastes, des cellules végétales, des calli, des semences, des tubercules, des boutures, ainsi que les descendants transgéniques de ces plantes.

18. Plantes transgéniques selon la revendication 17, sélectionnées parmi le groupe consistant en le tabac et la pomme de terre.

19. Procédé pour l'obtention de protéine de soie d'araignée végétale, comprenant les étapes suivantes :
a) la transmission d'une molécule d'acide nucléique recombinante ou d'un vecteur selon l'une des revendications 2 à 11 sur des cellules végétales ;
b) le cas échéant, la régénération de plantes à partir des cellules végétales transformées ; et
c) l'usinage des cellules végétales de a) et des plantes de b) respectivement pour l'obtention de protéine de soie d'araignée végétale.

20. Procédé pour l'obtention de protéine de soie d'araignée produit de manière recombinante comprenant les étapes suivantes :
a) la transmission d'une molécule d'acide nucléique recombinante ou d'un vecteur selon l'une des revendications 2 à 11 sur des cellules ;
b) la purification de la protéine de soie d'araignée par le traitement thermique de l'extrait cellulaire et la séparation postérieure des protéines dénaturées propres à la cellule.

21. Procédé pour l'obtention de protéine de soie d'araignée produit de manière recombinante comprenant les étapes suivantes :
a) la transmission d'une molécule d'acide nucléique recombinante ou d'un vecteur selon l'une des revendications 2 à 11 sur des cellules ;
b) la purification de la protéine de soie d'araignée par l'ajustement d'un pH acidic, préférentiellement d'un pH dans la gamme de 2,5 à 3,5 par l'addition de l'acide, préférentiellement de l'acide chlorhydrique à l'extrait cellulaire et la séparation postérieure des protéines dénaturées propres à la cellule.

22. Procédé pour l'obtention de protéine de soie d'araignée produit de manière recombinante comprenant les étapes suivantes :
a) la transmission d'une molécule d'acide nucléique recombinante selon l'une des revendications 2 à 10 sur des cellules ;
b) la purification de la protéine de soie d'araignée de la manière suivante :
- l'enrichissement de la protéine de soie d'araignée-protéine ELP fusionnée par le traitement thermique de l'extrait cellulaire ;
- la précipitation de la protéine de soie d'araignée-protéine ELP fusionnée par une augmentation ultérieure de la température, préférentiellement à une temperature d'au moins 60°C et préférentiellement à une concentration de sel de 1 M à 2 M ; et
- l'élimination du fragment ELP, préférentiellement par digestion avec du CNBr.

23. Procédé selon l'une des revendications 20 à 22,
**caractérisé par le fait que** les cellules sont sélectionnées parmi les cellules végétales, les cellules animales et les cellules bactériennes.

24. Protéine de soie d'araignée végétale fabriquée par un procédé selon l'une des revendications 19 à 23.

25. Utilisation de la protéine de soie d'araignée selon l'une des revendications 13, 14 ou 24 pour la fabrication des fils synthétiques, des feuilles et/ou des membranes.

26. Utilisation selon la revendication 25, dans laquelle les fils, les feuilles et/ou les membranes sont employés pour des applications médicales, en particulier pour suturer des blessures et/ou comme échafaudage et comme couverture, respectivement, pour des organes artificiels.

27. Utilisation selon la revendication 26, dans laquelle les feuilles et/ou les membranes sont employées comme une face de fixation pour des cellules cultivées et/ou pour la filtration.

28. Séquence d'ADN selon la revendication 1 ou protéine de soie d'araignée selon l'une des revendications 13, 14 ou 24, dans lesquels la gamme des propriétés en relation à au moins une propriété, sélectionnée parmi la force de déchirure, l'élasticité, la capacité de gonflage, le comportement de la solubilité, la stabilité à l'acide, la stabilité thermique, est changée en relation à la protéine de soie d'araignée native.


### SEQUENZPROTOKOLL

<110> IPK - Institut für Pflanzengenetik und Kulturpflan
<120> Synthetische Spinnenseidenproteine und deren Expression in transgenen Pflanzen
<130> I 7222
<140>
   <141>
<150> DE 100 28 212.1
   <151> 2000-06-09
<150> DE 100 53 478.3
   <151> 2000-10-24
<160> 51
<170> PatentIn Ver. 2.1
<210> 1
   <211> 22
   <212> DNA
   <213> Künstliche Sequenz
<220>
   <223> Beschreibung der künstlichen Sequenz:repetitive Einheit aus Spidroin-Proteinen
<400> 1
   tatgagcgct cccgggcagg gt 22
<210> 2
   <211> 38
   <212> DNA
   <213> Künstliche Sequenz
<220>
   <223> Beschreibung der künstlichen Sequenz:repetitive Einheit aus Spidroin-Proteinen
<400> 2
   agcttttagg taccaatatt aatctggccg gctccacc 38
<210> 3
   <211> 12
   <212> DNA
   <213> Künstliche Sequenz
<220>
   <223> Beschreibung der künstlichen Sequenz:repetitive Einheit aus Spidroin-Proteinen
<400> 3
   tatggtctgg gg 12
<210> 4
   <211> 18
   <212> DNA
   <213> Künstliche Sequenz
<220>
   <223> Beschreibung der künstlichen Sequenz:repetitive Einheit aus Spidroin-Proteinen
<400> 4
   ggccagggtg ctggccaa 18
<210> 5
   <211> 33
   <212> DNA
   <213> Künstliche Sequenz
<220>
   <223> Beschreibung der künstlichen Sequenz:repetitive Einheit aus Spidroin-Proteinen
<400> 5
   ggtgcaggag cwgcwgcwgc wgctgcaggt gga 33
<210> 6
   <211> 28
   <212> DNA
   <213> Künstliche Sequenz
<220>
   <223> Beschreibung der künstlichen Sequenz:repetitive Einheit aus Spidroin-Proteinen
<400> 6
   gccggccaga ttaatattgg tacctaaa 28
<210> 7
   <211> 17
   <212> DNA
   <213> Künstliche Sequenz
<220>
   <223> Beschreibung der künstlichen Sequenz:repetitive Einheit aus Spidroin-Proteinen
<400> 7
   ctgcccggga gcgctca 17
<210> 8
   <211> 15
   <212> DNA
   <213> Künstliche Sequenz
<220>
   <223> Beschreibung der künstlichen Sequenz:repetitive Einheit aus Spidroin-Proteinen
<400> 8
   accaccataa cctcc 15
<210> 9
   <211> 18
   <212> DNA
   <213> Künstliche Sequenz
<220>
   <223> Beschreibung der künstlichen Sequenz:repetitive Einheit aus Spidroin-Proteinen
<400> 9
   agcaccctgg ccccccag 18
<210> 10
   <211> 33
   <212> DNA
   <213> Künstliche Sequenz
<220>
   <223> Beschreibung der künstlichen Sequenz:repetitive Einheit aus Spidroin-Proteinen
<400> 10
   tgcagcwgcw gcwgcwgctc ctgcaccttg gcc 33
<210> 11
   <211> 22
   <212> DNA
   <213> Künstliche Sequenz
<220>
   <223> Beschreibung der künstlichen Sequenz:repetitive Einheit aus Spidroin-Proteinen
<400> 11
   tatgagatct ggccaaggag gt 22
<210> 12
   <211> 14
   <212> DNA
   <213> Künstliche Sequenz
<220>
   <223> Beschreibung der künstlichen Sequenz:repetitive Einheit aus Spidroin-Proteinen
<400> 12
   ttggccagat ctca 14
<210> 13
   <211> 27
   <212> DNA
   <213> Künstliche Sequenz
<220>
   <223> Beschreibung der künstlichen Sequenz:repetitive Einheit aus Spidroin-Proteinen
<400> 13
   agtcagggtg ctggtcgtgg aggccaa 27
<210> 14
   <211> 27
   <212> DNA
   <213> Künstliche Sequenz
<220>
   <223> Beschreibung der künstlichen Sequenz:repetitive Einheit aus Spidroin-Proteinen
<400> 14
   tccacgacca gcaccctgac tccccag 27
<210> 15
   <211> 36
   <212> DNA
   <213> Künstliche Sequenz
<220>
   <223> Beschreibung der künstlichen Sequenz:repetitive Einheit aus Spidroin-Proteinen
<400> 15
   agtcagggcg ctggtcgtgg gggactgggt ggccaa 36
<210> 16
   <211> 36
   <212> DNA
   <213> Künstliche Sequenz
<220>
   <223> Beschreibung der künstlichen Sequenz:repetitive Einheit aus Spidroin-Proteinen
<400> 16
   acccagtccc ccacgaccag cgccctgact ccccag 36
<210> 17
   <211> 24
   <212> DNA
   <213> Künstliche Sequenz
<220>
   <223> Beschreibung der künstlichen Sequenz:repetitive Einheit aus Spidroin-Proteinen
<400> 17
   ctgggagggc agggagcggg ccaa 24
<210> 18
   <211> 24
   <212> DNA
   <213> Künstliche Sequenz
<220>
   <223> Beschreibung der künstlichen Sequenz:repetitive Einheit aus Spidroin-Proteinen
<400> 18
   cgctccctgc cctcccagac ctcc 24
<210> 19
   <211> 327
   <212> DNA
   <213> Künstliche Sequenz
<220>
   <223> Beschreibung der künstlichen Sequenz:Konstrukt SB1
<400> 19
   ggatcccagt tagggcaggg aggttatggt ggtctggggg gccagggtgc tggccaagga 60
   ggttatggtg gtctggggag tcagggcgct ggtcgtgggg gactgggtgg ccaaggtgca 120
   ggagctgctg ctgcagctgc aggtggagcc gggcagggag gtctgggagg gcagggagcg 180
   ggccaaggtg caggagcagc tgcagcagct gcaggtggag ccgggcaggg aggttatggt 240
   ggtctgggga gtcagggtgc tggtcgtgga ggccaaggtg caggagctgc agcagcagct 300
   gcaggtggag ccggacaagc ggccgca                                     327
<210> 20
   <210> 20
   <211> 705
   <212> DNA
   <213> Künstliche Sequenz
<220>
   <223> Beschreibung der künstlichen Sequenz:Konstrukt SE1
<400> 20
   ggatcccagt tagggcaggg aggttatggt ggtctggggg gccagggtgc tggccaagga 60
   ggttatggtg gtctggggag tcagggcgct ggtcgtgggg gactgggtgg ccaaggtgca 120
   ggagctgctg ctgcagctgc aggtggagcc gggcagggag gtctgggagg gcagggagcg 180
   ggccaaggtg caggagcagc tgcagcagct gcaggtggag ccgggcaggg aggttatggt 240
   ggtctgggga gtcagggcgc tggtcgtggg ggactgggtg gccaaggtgc aggagcagct 300
   gcagctgctg caggtggagc cgggcaggga ggttatggtg gtctggggag tcagggtgct 360
   ggtcgtggag gccaaggtgc aggagctgca gcagcagctg caggtggagc cgggcaggga 420
   ggttatggtg gtctggggag tcagggcgct ggtcgtgggg gactgggtgg ccaaggtgca 480
   ggagcagctg cagctgctgc aggtggagcc gggcagggag gttatggtgg tctggggagt 540
   cagggtgctg gtcgtggagg ccaaggtgca ggagctgcag cagcagctgc aggtggagcc 600
   gggcagggag gttatggtgg tctggggagt cagggtgctg gtcgtggagg ccaaggtgca 660
   ggagctgcag cagcagctgc aggtggagcc ggacaagcgg ccgca 705
<210> 21
   <211> 426
   <212> DNA
   <213> Künstliche Sequenz
<220>
   <223> Beschreibung der künstlichen Sequenz:Konstrukt SD1
<400> 21
   ggatcccagt tagggcaggg aggttatggt ggtctggggg gccagggtgc tggccaagga 60
   ggttatggtg gtctggggag tcagggcgct ggtcgtgggg gactgggtgg ccaaggtgca 120
   ggagctgctg ctgcagctgc aggtggagcc gggcagggag gtctgggagg gcagggagcg 180
   ggccaaggtg caggagcagc tgcagcagct gcaggtggag ccgggcaggg aggttatggt 240
   ggtctgggga gtcagggtgc tggtcgtgga ggccaaggtg caggagctgc agcagcagct 300
   gcaggtggag ccgggcaggg aggttatggt ggtctgggga gtcagggcgc tggtcgtggg 360
   ggactgggtg gccaaggtgc aggagcagct gcagctgctg caggtggagc cggacaagcg 420
   gccgca 426
<210> 22
   <211> 3783
   <212> DNA
   <213> Künstliche Sequenz
<220>
   <223> Beschreibung der künstlichen Sequenz:Konstrukt SO1SO1
<400> 22
   ggatcccagt tacccgggca gggaggttat ggtggtctgg ggggccaggg tgctggccaa 60
   ggaggttatg gtggtctggg gggccagggt gctggccaag gtgcaggagc tgctgctgca 120
   gctgcaggtg gagccgggca gggaggttat ggtggtctgg ggagtcaggg tgctggtcgt 180
   ggaggccaag gtgcaggagc tgcagcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg gggactgggt tggccaaggt 480
   gcaggagcag ctgcagctgc tgcaggtgga gccgggcagg gaggttatgg tggtctgggg 540
   ggccagggtg ctggccaagg aggttatggt ggtctgggga gtcagggcgc tggtcgtggg 600
   ggactgggtg gccaaggtgc aggagctgct gctgcagctg caggtggagc cgggcaggga 660
   ggtctgggag ggcagggagc gggccaaggt gcaggagcag ctgcagcagc tgcaggtgga 720
   gccgggcagg gaggttatgg tggtctgggg agtcagggtg ctggtcgtgg aggccaaggt 780
   gcaggagctg cagcagcagc tgcaggtgga gccgggcagg gaggttatgg tggtctgggg 840
   ggccagggtg ctggccaagg aggttatggt ggtctgggga gtcagggcgc tggtcgtggg 900
   ggactgggtg gccaaggtgc aggagctgct gctgcagctg caggtggagc cgggcaggga 960
   ggtctgggag ggcagggagc gggccaaggt gcaggagcag ctgcagcagc tgcaggtgga 1020
   gccgggcagg gaggttatgg tggtctgggg agtcagggcg ctggtcgtgg gggactgggt 1080
   ggccaaggtg caggagcagc tgcagctgct gcaggtggag ccgggcaggg aggttatggt 1140
   ggtctgggga gtcagggtgc tggtcgtgga ggccaaggtg caggagctgc agcagcagct 1200
   gcaggtggag ccgggcaggg aggttatggt ggtctgggga gtcagggcgc tggtcgtggg 1260
   ggactgggtg gccaaggtgc aggagcagct gcagctgctg caggtggagc cgggcaggga 1320
   ggttatggtg gtctggggag tcagggtgct ggtcgtggag gccaaggtgc aggagctgca 1380
   gcagcagctg caggtggagc cgggcaggga ggttatggtg gtctggggag tcagggtgct 1440
   ggtcgtggag gccaaggtgc aggagctgca gcagcagctg caggtggagc cgggcaggga 1500
   ggttatggtg gtctgggggg ccagggtgct ggccaaggag gttatggtgg tctggggagt 1560
   cagggcgctg gtcgtggggg actgggtggc caaggtgcag gagctgctgc tgcagctgca 1620
   ggtggagccg ggcagggagg tctgggaggg cagggagcgg gccaaggtgc aggagcagct 1680
   gcagcagctg caggtggagc cgggcaggga ggttatggtg gtctggggag tcagggtgct 1740
   ggtcgtggag gccaaggtgc aggagctgca gcagcagctg caggtggagc cgggcaggga 1800
   ggttatggtg gtctggggag tcagggcgct ggtcgtgggg gactgggtgg ccaaggtgca 1860
   ggagcagctg cagctgctgc aggtggagcc gggcagggag gttatggtgg tctggggggc 1920
   cagggtgctg gccaaggagg ttatggtggt ctggggggcc agggtgctgg ccaaggtgca 1980
   ggagctgctg ctgcagctgc aggtggagcc gggcagggag gttatggtgg tctggggagt 2040
   cagggtgctg gtcgtggagg ccaaggtgca ggagctgcag cagcagctgc aggtggagcc 2100
   gggcagggag gttatggtgg tctggggagt cagggcgctg gtcgtggggg actgggtggc 2160
   caaggtgcag gagcagctgc agctgctgca ggtggagccg ggcagggagg ttatggtggt 2220
   ctggggagtc agggtgctgg tcgtggaggc caaggtgcag gagctgcagc agcagctgca 2280
   ggtggagccg ggcagggagg ttatggtggt ctggggagtc agggcgctgg tcgtggggga 2340
   ctgggtggcc aaggtgcagg agcagctgca gctgctgcag gtggagccgg gcagggaggt 2400
   tatggtggtc tggggggcca gggtgctggc caaggaggtt atggtggtct ggggagtcag 2460
   ggcgctggtc gtgggggact gggtggccaa ggtgcaggag ctgctgctgc agctgcaggt 2520
   ggagccgggc agggaggtct gggagggcag ggagcgggcc aaggtgcagg agcagctgca 2580
   gcagctgcag gtggagccgg gcagggaggt tatggtggtc tggggagtca gggtgctggt 2640
   cgtggaggcc aaggtgcagg agctgcagca gcagctgcag gtggagccgg gcagggaggt 2700
   tatggtggtc tggggggcca gggtgctggc caaggaggtt atggtggtct ggggagtcag 2760
   ggcgctggtc gtgggggact gggtggccaa ggtgcaggag ctgctgctgc agctgcaggt 2820
   ggagccgggc agggaggtct gggagggcag ggagcgggcc aaggtgcagg agcagctgca 2880
   gcagctgcag gtggagccgg gcagggaggt tatggtggtc tggggagtca gggcgctggt 2940
   cgtgggggac tgggtggcca aggtgcagga gcagctgcag ctgctgcagg tggagccggg 3000
   cagggaggtt atggtggtct ggggagtcag ggtgctggtc gtggaggcca aggtgcagga 3060
   gctgcagcag cagctgcagg tggagccggg cagggaggtt atggtggtct ggggagtcag 3120
   ggcgctggtc gtgggggact gggtggccaa ggtgcaggag cagctgcagc tgctgcaggt 3180
   ggagccgggc agggaggtta tggtggtctg gggagtcagg gtgctggtcg tggaggccaa 3240
   ggtgcaggag ctgcagcagc agctgcaggt ggagccgggc agggaggtta tggtggtctg 3300
   gggagtcagg gtgctggtcg tggaggccaa ggtgcaggag ctgcagcagc agctgcaggt 3360
   ggagccgggc agggaggtta tggtggtctg gggggccagg gtgctggcca aggaggttat 3420
   ggtggtctgg ggagtcaggg cgctggtcgt gggggactgg gtggccaagg tgcaggagct 3480
   gctgctgcag ctgcaggtgg agccgggcag ggaggtctgg gagggcaggg agcgggccaa 3540
   ggtgcaggag cagctgcagc agctgcaggt ggagccgggc agggaggtta tggtggtctg 3600
   gggagtcagg gtgctggtcg tggaggccaa ggtgcaggag ctgcagcagc agctgcaggt 3660
   ggagccgggc agggaggtta tggtggtctg gggagtcagg gcgctggtcg tgggggactg 3720
   ggtggccaag gtgcaggagc agctgcagct gctgcaggtg gagccggcgg acaagcggcc 3780
   gca 3783
<210> 23
   <211> 2985
   <212> DNA
   <213> Künstliche Sequenz
<220>
   <223> Beschreibung der künstlichen Sequenz:Konstrukt SO1SM12
<400> 23
   ggatcccagt tacccgggca gggaggttat ggtggtctgg ggggccaggg tgctggccaa 60
   ggaggttatg gtggtctggg gggccagggt gctggccaag gtgcaggagc tgctgctgca 120
   gctgcaggtg gagccgggca gggaggttat ggtggtctgg ggagtcaggg tgctggtcgt 180
   ggaggccaag gtgcaggagc tgcagcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg gggactgggg tggccaaggt 480
   gcaggagcag ctgcagctgc tgcaggtgga gccgggcagg gaggttatgg tggtctgggg 540
   ggccagggtg ctggccaagg aggttatggt ggtctgggga gtcagggcgc tggtcgtggg 600
   ggactgggtg gccaaggtgc aggagctgct gctgcagctg caggtggagc cgggcaggga 660
   ggtctgggag ggcagggagc gggccaaggt gcaggagcag ctgcagcagc tgcaggtgga 720
   gccgggcagg gaggttatgg tggtctgggg agtcagggtg ctggtcgtgg aggccaaggt 780
   gcaggagctg cagcagcagc tgcaggtgga gccgggcagg gaggttatgg tggtctgggg 840
   ggccagggtg ctggccaagg aggttatggt ggtctgggga gtcagggcgc tggtcgtggg 900
   ggactgggtg gccaaggtgc aggagctgct gctgcagctg caggtggagc cgggcaggga 960
   ggtctgggag ggcagggagc gggccaaggt gcaggagcag ctgcagcagc tgcaggtgga 1020
   gccgggcagg gaggttatgg tggtctgggg agtcagggcg ctggtcgtgg gggactgggt 1080
   ggccaaggtg caggagcagc tgcagctgct gcaggtggag ccgggcaggg aggttatggt 1140
   ggtctgggga gtcagggtgc tggtcgtgga ggccaaggtg caggagctgc agcagcagct 1200
   gcaggtggag ccgggcaggg aggttatggt ggtctgggga gtcagggcgc tggtcgtggg 1260
   ggactgggtg gccaaggtgc aggagcagct gcagctgctg caggtggagc cgggcaggga 1320
   ggttatggtg gtctggggag tcagggtgct ggtcgtggag gccaaggtgc aggagctgca 1380
   gcagcagctg caggtggagc cgggcaggga ggttatggtg gtctggggag tcagggtgct 1440
   ggtcgtggag gccaaggtgc aggagctgca gcagcagctg caggtggagc cgggcaggga 1500
   ggttatggtg gtctgggggg ccagggtgct ggccaaggag gttatggtgg tctggggagt 1560
   cagggcgctg gtcgtggggg actgggtggc caaggtgcag gagctgctgc tgcagctgca 1620
   ggtggagccg ggcagggagg tctgggaggg cagggagcgg gccaaggtgc aggagcagct 1680
   gcagcagctg caggtggagc cgggcaggga ggttatggtg gtctggggag tcagggtgct 1740
   ggtcgtggag gccaaggtgc aggagctgca gcagcagctg caggtggagc cgggcaggga 1800
   ggttatggtg gtctggggag tcagggcgct ggtcgtgggg gactgggtgg ccaaggtgca 1860
   ggagcagctg cagctgctgc aggtggagcc gggcagggag gttatggtgg tctggggggc 1920
   cagggtgctg gccaaggagg ttatggtggt ctggggagtc agggcgctgg tcgtggggga 1980
   ctgggtggcc aaggtgcagg agctgctgct gcagctgcag gtggagccgg gcagggaggt 2040
   ctgggagggc agggagcggg ccaaggtgca ggagcagctg cagcagctgc aggtggagcc 2100
   gggcagggag gttatggtgg tctggggagt cagggcgctg gtcgtggggg actgggtggc 2160
   caaggtgcag gagcagctgc agctgctgca ggtggagccg ggcagggagg ttatggtggt 2220
   ctggggagtc agggtgctgg tcgtggaggc caaggtgcag gagctgcagc agcagctgca 2280
   ggtggagccg ggcagggagg ttatggtggt ctggggagtc agggcgctgg tcgtggggga 2340
   ctgggtggcc aaggtgcagg agcagctgca gctgctgcag gtggagccgg gcagggaggt 2400
   tatggtggtc tggggagtca gggtgctggt cgtggaggcc aaggtgcagg agctgcagca 2460
   gcagctgcag gtggagccgg gcagggaggt tatggtggtc tggggagtca gggtgctggt 2520
   cgtggaggcc aaggtgcagg agctgcagca gcagctgcag gtggagccgg gcagggaggt 2580
   tatggtggtc tggggggcca gggtgctggc caaggaggtt atggtggtct ggggagtcag 2640
   ggcgctggtc gtgggggact gggtggccaa ggtgcaggag ctgctgctgc agctgcaggt 2700
   ggagccgggc agggaggtct gggagggcag ggagcgggcc aaggtgcagg agcagctgca 2760
   gcagctgcag gtggagccgg gcagggaggt tatggtggtc tggggagtca gggtgctggt 2820
   cgtggaggcc aaggtgcagg agctgcagca gcagctgcag gtggagccgg gcagggaggt 2880
   tatggtggtc tggggagtca gggcgctggt cgtgggggac tgggtggcca aggtgcagga 2940
   gcagctgcag ctgctgcagg tggagccggc ggacaagcgg ccgca 2985
<210> 24
   <211> 5658
   <212> DNA
   <213> Künstliche Sequenz
<220>
   <223> Beschreibung der künstlichen Sequenz:Konstrukt SO1SO1SO1
<400> 24
   ggatcccagt tacccgggca gggaggttat ggtggtctgg ggggccaggg tgctggccaa 60
   ggaggttatg gtggtctggg gggccagggt gctggccaag gtgcaggagc tgctgctgca 120
   gctgcaggtg gagccgggca gggaggttat ggtggtctgg ggagtcaggg tgctggtcgt 180
   ggaggccaag gtgcaggagc tgcagcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg ggggactggg tggccaaggt 480
   gcaggagcag ctgcagctgc tgcaggtgga gccgggcagg gaggttatgg tggtctgggg 540
   ggccagggtg ctggccaagg aggttatggt ggtctgggga gtcagggcgc tggtcgtggg 600
   ggactgggtg gccaaggtgc aggagctgct gctgcagctg caggtggagc cgggcaggga 660
   ggtctgggag ggcagggagc gggccaaggt gcaggagcag ctgcagcagc tgcaggtgga 720
   gccgggcagg gaggttatgg tggtctgggg agtcagggtg ctggtcgtgg aggccaaggt 780
   gcaggagctg cagcagcagc tgcaggtgga gccgggcagg gaggttatgg tggtctgggg 840
   ggccagggtg ctggccaagg aggttatggt ggtctgggga gtcagggcgc tggtcgtggg 900
   ggactgggtg gccaaggtgc aggagctgct gctgcagctg caggtggagc cgggcaggga 960
   ggtctgggag ggcagggagc gggccaaggt gcaggagcag ctgcagcagc tgcaggtgga 1020
   gccgggcagg gaggttatgg tggtctgggg agtcagggcg ctggtcgtgg gggactgggt 1080
   ggccaaggtg caggagcagc tgcagctgct gcaggtggag ccgggcaggg aggttatggt 1140
   ggtctgggga gtcagggtgc tggtcgtgga ggccaaggtg caggagctgc agcagcagct 1200
   gcaggtggag ccgggcaggg aggttatggt ggtctgggga gtcagggcgc tggtcgtggg 1260
   ggactgggtg gccaaggtgc aggagcagct gcagctgctg caggtggagc cgggcaggga 1320
   ggttatggtg gtctggggag tcagggtgct ggtcgtggag gccaaggtgc aggagctgca 1380
   gcagcagctg caggtggagc cgggcaggga ggttatggtg gtctggggag tcagggtgct 1440
   ggtcgtggag gccaaggtgc aggagctgca gcagcagctg caggtggagc cgggcaggga 1500
   ggttatggtg gtctgggggg ccagggtgct ggccaaggag gttatggtgg tctggggagt 1560
   cagggcgctg gtcgtggggg actgggtggc caaggtgcag gagctgctgc tgcagctgca 1620
   ggtggagccg ggcagggagg tctgggaggg cagggagcgg gccaaggtgc aggagcagct 1680
   gcagcagctg caggtggagc cgggcaggga ggttatggtg gtctggggag tcagggtgct 1740
   ggtcgtggag gccaaggtgc aggagctgca gcagcagctg caggtggagc cgggcaggga 1800
   ggttatggtg gtctggggag tcagggcgct ggtcgtgggg gactgggtgg ccaaggtgca 1860
   ggagcagctg cagctgctgc aggtggagcc gggcagggag gttatggtgg tctggggggc 1920
   cagggtgctg gccaaggagg ttatggtggt ctggggggcc agggtgctgg ccaaggtgca 1980
   ggagctgctg ctgcagctgc aggtggagcc gggcagggag gttatggtgg tctggggagt 2040
   cagggtgctg gtcgtggagg ccaaggtgca ggagctgcag cagcagctgc aggtggagcc 2100
   gggcagggag gttatggtgg tctggggagt cagggcgctg gtcgtggggg actgggtggc 2160
   caaggtgcag gagcagctgc agctgctgca ggtggagccg ggcagggagg ttatggtggt 2220
   ctggggagtc agggtgctgg tcgtggaggc caaggtgcag gagctgcagc agcagctgca 2280
   ggtggagccg ggcagggagg ttatggtggt ctggggagtc agggcgctgg tcgtggggga 2340
   ctgggtggcc aaggtgcagg agcagctgca gctgctgcag gtggagccgg gcagggaggt 2400
   tatggtggtc tggggggcca gggtgctggc caaggaggtt atggtggtct ggggagtcag 2460
   ggcgctggtc gtgggggact gggtggccaa ggtgcaggag ctgctgctgc agctgcaggt 2520
   ggagccgggc agggaggtct gggagggcag ggagcgggcc aaggtgcagg agcagctgca 2580
   gcagctgcag gtggagccgg gcagggaggt tatggtggtc tggggagtca gggtgctggt 2640
   cgtggaggcc aaggtgcagg agctgcagca gcagctgcag gtggagccgg gcagggaggt 2700
   tatggtggtc tggggggcca gggtgctggc caaggaggtt atggtggtct ggggagtcag 2760
   ggcgctggtc gtgggggact gggtggccaa ggtgcaggag ctgctgctgc agctgcaggt 2820
   ggagccgggc agggaggtct gggagggcag ggagcgggcc aaggtgcagg agcagctgca 2880
   gcagctgcag gtggagccgg gcagggaggt tatggtggtc tggggagtca gggcgctggt 2940
   cgtgggggac tgggtggcca aggtgcagga gcagctgcag ctgctgcagg tggagccggg 3000
   cagggaggtt atggtggtct ggggagtcag ggtgctggtc gtggaggcca aggtgcagga 3060
   gctgcagcag cagctgcagg tggagccggg cagggaggtt atggtggtct ggggagtcag 3120
   ggcgctggtc gtgggggact gggtggccaa ggtgcaggag cagctgcagc tgctgcaggt 3180
   ggagccgggc agggaggtta tggtggtctg gggagtcagg gtgctggtcg tggaggccaa 3240
   ggtgcaggag ctgcagcagc agctgcaggt ggagccgggc agggaggtta tggtggtctg 3300
   gggagtcagg gtgctggtcg tggaggccaa ggtgcaggag ctgcagcagc agctgcaggt 3360
   ggagccgggc agggaggtta tggtggtctg gggggccagg gtgctggcca aggaggttat 3420
   ggtggtctgg ggagtcaggg cgctggtcgt gggggactgg gtggccaagg tgcaggagct 3480
   gctgctgcag ctgcaggtgg agccgggcag ggaggtctgg gagggcaggg agcgggccaa 3540
   ggtgcaggag cagctgcagc agctgcaggt ggagccgggc agggaggtta tggtggtctg 3600
   gggagtcagg gtgctggtcg tggaggccaa ggtgcaggag ctgcagcagc agctgcaggt 3660
   ggagccgggc agggaggtta tggtggtctg gggagtcagg gcgctggtcg tgggggactg 3720
   ggtggccaag gtgcaggagc agctgcagct gctgcaggtg gagccgggca gggaggttat 3780
   ggtggtctgg ggggccaggg tgctggccaa ggaggttatg gtggtctggg gggccagggt 3840
   gctggccaag gtgcaggagc tgctgctgca gctgcaggtg gagccgggca gggaggttat 3900
   ggtggtctgg ggagtcaggg tgctggtcgt ggaggccaag gtgcaggagc tgcagcagca 3960
   gctgcaggtg gagccgggca gggaggttat ggtggtctgg ggagtcaggg cgctggtcgt 4020
   gggggactgg gtggccaagg tgcaggagca gctgcagctg ctgcaggtgg agccgggcag 4080
   ggaggttatg gtggtctggg gagtcagggt gctggtcgtg gaggccaagg tgcaggagct 4140
   gcagcagcag ctgcaggtgg agccgggcag ggaggttatg gtggtctggg gagtcagggc 4200
   gctggtcgtg ggggactggg tggccaaggt gcaggagcag ctgcagctgc tgcaggtgga 4260
   gccgggcagg gaggttatgg tggtctgggg ggccagggtg ctggccaagg aggttatggt 4320
   ggtctgggga gtcagggcgc tggtcgtggg ggactgggtg gccaaggtgc aggagctgct 4380
   gctgcagctg caggtggagc cgggcaggga ggtctgggag ggcagggagc gggccaaggt 4440
   gcaggagcag ctgcagcagc tgcaggtgga gccgggcagg gaggttatgg tggtctgggg 4500
   agtcagggtg ctggtcgtgg aggccaaggt gcaggagctg cagcagcagc tgcaggtgga 4560
   gccgggcagg gaggttatgg tggtctgggg ggccagggtg ctggccaagg aggttatggt 4620
   ggtctgggga gtcagggcgc tggtcgtggg ggactgggtg gccaaggtgc aggagctgct 4680
   gctgcagctg caggtggagc cgggcaggga ggtctgggag ggcagggagc gggccaaggt 4740
   gcaggagcag ctgcagcagc tgcaggtgga gccgggcagg gaggttatgg tggtctgggg 4800
   agtcagggcg ctggtcgtgg gggactgggt ggccaaggtg caggagcagc tgcagctgct 4860
   gcaggtggag ccgggcaggg aggttatggt ggtctgggga gtcagggtgc tggtcgtgga 4920
   ggccaaggtg caggagctgc agcagcagct gcaggtggag ccgggcaggg aggttatggt 4980
   ggtctgggga gtcagggcgc tggtcgtggg ggactgggtg gccaaggtgc aggagcagct 5040
   gcagctgctg caggtggagc cgggcaggga ggttatggtg gtctggggag tcagggtgct 5100
   ggtcgtggag gccaaggtgc aggagctgca gcagcagctg caggtggagc cgggcaggga 5160
   ggttatggtg gtctggggag tcagggtgct ggtcgtggag gccaaggtgc aggagctgca 5220
   gcagcagctg caggtggagc cgggcaggga ggttatggtg gtctgggggg ccagggtgct 5280
   ggccaaggag gttatggtgg tctggggagt cagggcgctg gtcgtggggg actgggtggc 5340
   caaggtgcag gagctgctgc tgcagctgca ggtggagccg ggcagggagg tctgggaggg 5400
   cagggagcgg gccaaggtgc aggagcagct gcagcagctg caggtggagc cgggcaggga 5460
   ggttatggtg gtctggggag tcagggtgct ggtcgtggag gccaaggtgc aggagctgca 5520
   gcagcagctg caggtggagc cgggcaggga ggttatggtg gtctggggag tcagggcgct 5580
   ggtcgtgggg gactgggtgg ccaaggtgca ggagcagctg cagctgctgc aggtggagcc 5640
   ggcggacaag cggccgca                                               5658
<210> 25
   <211> 672
   <212> DNA
   <213> Künstliche Sequenz
<220>
   <223> Beschreibung der künstlichen Sequenz:Konstrukt FA2
<400> 25
   ggatcccagt tagggcaggg aggttatggt ggtctggggg gccagggtgc tggccaagga 60
   ggttatggtg gtctggggag tcagggcgct ggtcgtgggg gactgggtgg ccaaggtgca 120
   ggagctgctg ctgcagctgc aggtggagcc gggcagggag gtctgggagg gcagggagcg 180
   ggccaaggtg caggagcagc tgcagcagct gcaggtggag ccgggcaggg aggttatggt 240
   ggtctgggga gtcagggcgc tggtcgtggg ggactgggtg gccaaggtgc aggagcagct 300
   gcagctgctg caggtggagc cgggtccgga agtggtgcag gtgccggaag cggagcagga 360
   gccggtgccg gatctggtgc cggtgccgga agcggtgctg gtgccggaag cggtgctggt 420
   gccggatcag gagcgggtgc cggttatggt gcgggagccg gtgttgggta cggagccggt 480
   tatggagcgg gagccggtgt tgggtacgga gccggtgcag gttccggggc cgcaagcggc 540
   gcaggagccg gtgccggagc tgggacaggg agttcaggat ttgggcccta cgttgcaaat 600
   ggtggttatt caggctatga atacgcgtgg agtagtaagt ctgattttga gactgccgga 660
   caagcggccg ca                                                     672
<210> 26
   <211> 525
   <212> DNA
   <213> Künstliche Sequenz
<220>
   <223> Beschreibung der künstlichen Sequenz:Konstrukt SA1
<400> 26
   ggatcccagt tagggcaggg aggttatggt ggtctggggg gccagggtgc tggccaagga 60
   ggttatggtg gtctgggggg ccagggtgct ggccaaggtg caggagctgc tgctgcagct 120
   gcaggtggag ccgggcaggg aggttatggt ggtctgggga gtcagggtgc tggtcgtgga 180
   ggccaaggtg caggagctgc agcagcagct gcaggtggag ccgggcaggg aggttatggt 240
   ggtctgggga gtcagggcgc tggtcgtggg ggactgggtg gccaaggtgc aggagcagct 300
   gcagctgctg caggtggagc cgggcaggga ggttatggtg gtctggggag tcagggtgct 360
   ggtcgtggag gccaaggtgc aggagctgca gcagcagctg caggtggagc cgggcaggga 420
   ggttatggtg gtctggggag tcagggcgct ggtcgtgggg gactgggtgg ccaaggtgca 480
   ggagcagctg cagctgctgc aggtggagcc ggacaagcgg ccgca                 525
<210> 27
   <211> 1908
   <212> DNA
   <213> Künstliche Sequenz
<220>
   <223> Beschreibung der künstlichen Sequenz:Konstrukt SO1
<400> 27
   ggatcccagt tacccgggca gggaggttat ggtggtctgg ggggccaggg tgctggccaa 60
   ggaggttatg gtggtctggg gggccagggt gctggccaag gtgcaggagc tgctgctgca 120
   gctgcaggtg gagccgggca gggaggttat ggtggtctgg ggagtcaggg tgctggtcgt 180
   ggaggccaag gtgcaggagc tgcagcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg gggactgggt tggccaaggt 480
   gcaggagcag ctgcagctgc tgcaggtgga gccgggcagg gaggttatgg tggtctgggg 540
   ggccagggtg ctggccaagg aggttatggt ggtctgggga gtcagggcgc tggtcgtggg 600
   ggactgggtg gccaaggtgc aggagctgct gctgcagctg caggtggagc cgggcaggga 660
   ggtctgggag ggcagggagc gggccaaggt gcaggagcag ctgcagcagc tgcaggtgga 720
   gccgggcagg gaggttatgg tggtctgggg agtcagggtg ctggtcgtgg aggccaaggt 780
   gcaggagctg cagcagcagc tgcaggtgga gccgggcagg gaggttatgg tggtctgggg 840
   ggccagggtg ctggccaagg aggttatggt ggtctgggga gtcagggcgc tggtcgtggg 900
   ggactgggtg gccaaggtgc aggagctgct gctgcagctg caggtggagc cgggcaggga 960
   ggtctgggag ggcagggagc gggccaaggt gcaggagcag ctgcagcagc tgcaggtgga 1020
   gccgggcagg gaggttatgg tggtctgggg agtcagggcg ctggtcgtgg gggactgggt 1080
   ggccaaggtg caggagcagc tgcagctgct gcaggtggag ccgggcaggg aggttatggt 1140
   ggtctgggga gtcagggtgc tggtcgtgga ggccaaggtg caggagctgc agcagcagct 1200
   gcaggtggag ccgggcaggg aggttatggt ggtctgggga gtcagggcgc tggtcgtggg 1260
   ggactgggtg gccaaggtgc aggagcagct gcagctgctg caggtggagc cgggcaggga 1320
   ggttatggtg gtctggggag tcagggtgct ggtcgtggag gccaaggtgc aggagctgca 1380
   gcagcagctg caggtggagc cgggcaggga ggttatggtg gtctggggag tcagggtgct 1440
   ggtcgtggag gccaaggtgc aggagctgca gcagcagctg caggtggagc cgggcaggga 1500
   ggttatggtg gtctgggggg ccagggtgct ggccaaggag gttatggtgg tctggggagt 1560
   cagggcgctg gtcgtggggg actgggtggc caaggtgcag gagctgctgc tgcagctgca 1620
   ggtggagccg ggcagggagg tctgggaggg cagggagcgg gccaaggtgc aggagcagct 1680
   gcagcagctg caggtggagc cgggcaggga ggttatggtg gtctggggag tcagggtgct 1740
   ggtcgtggag gccaaggtgc aggagctgca gcagcagctg caggtggagc cgggcaggga 1800
   ggttatggtg gtctggggag tcagggcgct ggtcgtgggg gactgggtgg ccaaggtgca 1860
   ggagcagctg cagctgctgc aggtggagcc ggcggacaag cggccgca             1908
<210> 28
   <211> 1110
   <212> DNA
   <213> Künstliche Sequenz
<220>
   <223> Beschreibung der künstlichen Sequenz:Konstrukt SM12
<400> 28
   ggatcccagt tacccgggca gggaggttat ggtggtctgg ggggccaggg tgctggccaa 60
   ggaggttatg gtggtctggg gagtcagggc gctggtcgtg ggggactggg tggccaaggt 120
   gcaggagctg ctgctgcagc tgcaggtgga gccgggcagg gaggtctggg agggcaggga 180
   gcgggccaag gtgcaggagc agctgcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg ggggactggg tggccaaggt 480
   gcaggagcag ctgcagctgc tgcaggtgga gccgggcagg gaggttatgg tggtctgggg 540
   agtcagggtg ctggtcgtgg aggccaaggt gcaggagctg cagcagcagc tgcaggtgga 600
   gccgggcagg gaggttatgg tggtctgggg agtcagggtg ctggtcgtgg aggccaaggt 660
   gcaggagctg cagcagcagc tgcaggtgga gccgggcagg gaggttatgg tggtctgggg 720
   ggccagggtg ctggccaagg aggttatggt ggtctgggga gtcagggcgc tggtcgtggg 780
   ggactgggtg gccaaggtgc aggagctgct gctgcagctg caggtggagc cgggcaggga 840
   ggtctgggag ggcagggagc gggccaaggt gcaggagcag ctgcagcagc tgcaggtgga 900
   gccgggcagg gaggttatgg tggtctgggg agtcagggtg ctggtcgtgg aggccaaggt 960
   gcaggagctg cagcagcagc tgcaggtgga gccgggcagg gaggttatgg tggtctgggg 1020
   agtcagggcg ctggtcgtgg gggactgggt ggccaaggtg caggagcagc tgcagctgct 1080
   gcaggtggag ccggcggaca agcggccgca                                  1110
<210> 29
   <211> 831
   <212> DNA
   <213> Künstliche Sequenz
<220>
   <223> Beschreibung der künstlichen Sequenz:Konstrukt SF1
<400> 29
   ggatcccagt tacccgggca gggaggttat ggtggtctgg ggggccaggg tgctggccaa 60
   ggaggttatg gtggtctggg gggccagggt gctggccaag gtgcaggagc tgctgctgca 120
   gctgcaggtg gagccgggca gggaggttat ggtggtctgg ggagtcaggg tgctggtcgt 180
   ggaggccaag gtgcaggagc tgcagcagca gctgcaggtg gagccgggca gggaggttat 240
   ggtggtctgg ggagtcaggg cgctggtcgt gggggactgg gtggccaagg tgcaggagca 300
   gctgcagctg ctgcaggtgg agccgggcag ggaggttatg gtggtctggg gagtcagggt 360
   gctggtcgtg gaggccaagg tgcaggagct gcagcagcag ctgcaggtgg agccgggcag 420
   ggaggttatg gtggtctggg gagtcagggc gctggtcgtg ggggactggg tggccaaggt 480
   gcaggagcag ctgcagctgc tgcaggtgga gccgggcagg gaggttatgg tggtctgggg 540
   ggccagggtg ctggccaagg aggttatggt ggtctgggga gtcagggcgc tggtcgtggg 600
   ggactgggtg gccaaggtgc aggagctgct gctgcagctg caggtggagc cgggcaggga 660
   ggtctgggag ggcagggagc gggccaaggt gcaggagcag ctgcagcagc tgcaggtgga 720
   gccgggcagg gaggttatgg tggtctgggg agtcagggtg ctggtcgtgg aggccaaggt 780
   gcaggagctg cagcagcagc tgcaggtgga gccggcggac aagcggccgc a          831
<210> 30
   <211> 104
   <212> PRT
   <213> Künstliche Sequenz
<220>
   <223> Beschreibung der künstlichen Sequenz:SB1-Protein
<400> 30
   Gly Gln Gly Gly Tyr Gly Gly Leu Gly Gly Gln Gly Ala Gly Gln Gly
    1               5                  10                  15
   Gly Tyr Gly Gly Leu Gly Ser Gln Gly Ala Gly Arg Gly Gly Leu Gly
               20                  25                  30
   Gly Gln Gly Ala Gly Ala Ala Ala Ala Ala Ala Gly Gly Ala Gly Gln
           35                  40                  45
   Gly Gly Leu Gly Gly Gln Gly Ala Gly Gln Gly Ala Gly Ala Ala Ala
       50                  55                  60
   Ala Ala Ala Gly Gly Ala Gly Gln Gly Gly Tyr Gly Gly Leu Gly Ser
   65                  70                  75                  80
   Gln Gly Ala Gly Arg Gly Gly Gln Gly Ala Gly Ala Ala Ala Ala Ala
                   85                  90                  95
   Ala Gly Gly Ala Gly Gln Ala Ala
               100
<210> 31
   <211> 230
   <212> PRT
   <213> Künstliche Sequenz
<220>
<210> 32
   <211> 137
   <212> PRT
   <213> Künstliche Sequenz
<220>
   <223> Beschreibung der künstlichen Sequenz:SD1-Protein
<400> 32
   Gly Gln Gly Gly Tyr Gly Gly Leu Gly Gly Gln Gly Ala Gly Gln Gly
   1 5 10 15
   Gly Tyr Gly Gly Leu Gly Ser Gln Gly Ala Gly Arg Gly Gly Leu Gly
   20 25 30
   Gly Gln Gly Ala Gly Ala Ala Ala Ala Ala Ala Gly Gly Ala Gly Gln
   35 40 45
   Gly Gly Leu Gly Gly Gln Gly Ala Gly Gln Gly Ala Gly Ala Ala Ala
   50 55 60
   Ala Ala Ala Gly Gly Ala Gly Gln Gly Gly Tyr Gly Gly Leu Gly Ser
   65 70 75 80
   Gln Gly Ala Gly Arg Gly Gly Gln Gly Ala Gly Ala Ala Ala Ala Ala
   85 90 95
   Ala Gly Gly Ala Gly Gln Gly Gly Tyr Gly Gly Leu Gly Ser Gln Gly
   100 105 110
   Ala Gly Arg Gly Gly Leu Gly Gly Gln Gly Ala Gly Ala Ala Ala Ala
   115 120 125
   Ala Ala Gly Gly Ala Gly Gln Ala Ala
   130 135
<210> 33
   <211> 1255
   <212> PRT
   <213> Künstliche Sequenz
<220>
   <223> Beschreibung der künstlichen Sequenz:SO1SO1-Protein
<400> 33
   Gly Gln Gly Gly Tyr Gly Gly Leu Gly Gly Gln Gly Ala Gly Gln Gly
    1               5                  10                  15
   Gly Tyr Gly Gly Leu Gly Gly Gln Gly Ala Gly Gln Gly Ala Gly Ala
               20                  25                  30
   Ala Ala Ala Ala Ala Gly Gly Ala Gly Gln Gly Gly Tyr Gly Gly Leu
           35                  40                  45
   Gly Ser Gln Gly Ala Gly Arg Gly Gly Gln Gly Ala Gly Ala Ala Ala
       50                  55                  60
   Ala Ala Ala Gly Gly Ala Gly Gln Gly Gly Tyr Gly Gly Leu Gly Ser
   65                  70                  75                  80
   Gln Gly Ala Gly Arg Gly Gly Leu Gly Gly Gln Gly Ala Gly Ala Ala
                   85                  90                  95
   Ala Ala Ala Ala Gly Gly Ala Gly Gln Gly Gly Tyr Gly Gly Leu Gly
               100                 105                 110
   Ser Gln Gly Ala Gly Arg Gly Gly Gln Gly Ala Gly Ala Ala Ala Ala
           115                 120                 125
   Ala Ala Gly Gly Ala Gly Gln Gly Gly Tyr Gly Gly Leu Gly Ser Gln
       130                 135                 140
   Gly Ala Gly Arg Gly Gly Leu Gly Gly Gln Gly Ala Gly Ala Ala Ala
   145                 150                 155                 160
   Ala Ala Ala Gly Gly Ala Gly Gln Gly Gly Tyr Gly Gly Leu Gly Gly
                   165                 170                 175
   Gln Gly Ala Gly Gln Gly Gly Tyr Gly Gly Leu Gly Ser Gln Gly Ala
               180                 185                 190
   Gly Arg Gly Gly Leu Gly Gly Gln Gly Ala Gly Ala Ala Ala Ala Ala
           195                 200                 205
   Ala Gly Gly Ala Gly Gln Gly Gly Leu Gly Gly Gln Gly Ala Gly Gln
       210                 215                 220
   Gly Ala Gly Ala Ala Ala Ala Ala Ala Gly Gly Ala Gly Gln Gly Gly
   225                 230                 235                 240
   Tyr Gly Gly Leu Gly Ser Gln Gly Ala Gly Arg Gly Gly Gln Gly Ala
                   245                 250                 255
   Gly Ala Ala Ala Ala Ala Ala Gly Gly Ala Gly Gln Gly Gly Tyr Gly
               260                 265                 270
   Gly Leu Gly Gly Gln Gly Ala Gly Gln Gly Gly Tyr Gly Gly Leu Gly
           275                 280                 285
   Ser Gln Gly Ala Gly Arg Gly Gly Leu Gly Gly Gln Gly Ala Gly Ala
       290                 295                 300
   Ala Ala Ala Ala Ala Gly Gly Ala Gly Gln Gly Gly Leu Gly Gly Gln
   305                 310                 315                 320
   Gly Ala Gly Gln Gly Ala Gly Ala Ala Ala Ala Ala Ala Gly Gly Ala
                   325                 330                 335
   Gly Gln Gly Gly Tyr Gly Gly Leu Gly Ser Gln Gly Ala Gly Arg Gly
               340                 345                 350
   Gly Leu Gly Gly Gln Gly Ala Gly Ala Ala Ala Ala Ala Ala Gly Gly
           355                 360                 365
   Ala Gly Gln Gly Gly Tyr Gly Gly Leu Gly Ser Gln Gly Ala Gly Arg
       370                 375                 380
   Gly Gly Gln Gly Ala Gly Ala Ala Ala Ala Ala Ala Gly Gly Ala Gly
   385                 390                 395                 400
   Gln Gly Gly Tyr Gly Gly Leu Gly Ser Gln Gly Ala Gly Arg Gly Gly
                   405                 410                 415
   Leu Gly Gly Gln Gly Ala Gly Ala Ala Ala Ala Ala Ala Gly Gly Ala
   420 425 430
   Gly Gln Gly Gly Tyr Gly Gly Leu Gly Ser Gln Gly Ala Gly Arg Gly
   435 440 445
   Gly Gln Gly Ala Gly Ala Ala Ala Ala Ala Ala Gly Gly Ala Gly Gln
   450 455 460
   Gly Gly Tyr Gly Gly Leu Gly Ser Gln Gly Ala Gly Arg Gly Gly Gln
   465 470 475 480
   Gly Ala Gly Ala Ala Ala Ala Ala Ala Gly Gly Ala Gly Gln Gly Gly
   485 490 495
   Tyr Gly Gly Leu Gly Gly Gln Gly Ala Gly Gln Gly Gly Tyr Gly Gly
   500 505 510
   Leu Gly Ser Gln Gly Ala Gly Arg Gly Gly Leu Gly Gly Gln Gly Ala
   515 520 525
   Gly Ala Ala Ala Ala Ala Ala Gly Gly Ala Gly Gln Gly Gly Leu Gly
   530 535 540
   Gly Gln Gly Ala Gly Gln Gly Ala Gly Ala Ala Ala Ala Ala Ala Gly
   545 550 555 560
   Gly Ala Gly Gln Gly Gly Tyr Gly Gly Leu Gly Ser Gln Gly Ala Gly
   565 570 575
   Arg Gly Gly Gln Gly Ala Gly Ala Ala Ala Ala Ala Ala Gly Gly Ala
   580 585 590
   Gly Gln Gly Gly Tyr Gly Gly Leu Gly Ser Gln Gly Ala Gly Arg Gly
   595 600 605
   Gly Leu Gly Gly Gln Gly Ala Gly Ala Ala Ala Ala Ala Ala Gly Gly
   610 615 620
   Ala Gly Gln Gly Gly Tyr Gly Gly Leu Gly Gly Gln Gly Ala Gly Gln
   625 630 635 640
   Gly Gly Tyr Gly Gly Leu Gly Gly Gln Gly Ala Gly Gln Gly Ala Gly
   645 650 655
   Ala Ala Ala Ala Ala Ala Gly Gly Ala Gly Gln Gly Gly Tyr Gly Gly
   660 665 670
   Leu Gly Ser Gln Gly Ala Gly Arg Gly Gly Gln Gly Ala Gly Ala Ala
   675 680 685
   Ala Ala Ala Ala Gly Gly Ala Gly Gln Gly Gly Tyr Gly Gly Leu Gly
   690 695 700
   Ser Gln Gly Ala Gly Arg Gly Gly Leu Gly Gly Gln Gly Ala Gly Ala
   705 710 715 720
   Ala Ala Ala Ala Ala Gly Gly Ala Gly Gln Gly Gly Tyr Gly Gly Leu
   725 730 735
   Gly Ser Gln Gly Ala Gly Arg Gly Gly Gln Gly Ala Gly Ala Ala Ala
   740 745 750
   Ala Ala Ala Gly Gly Ala Gly Gln Gly Gly Tyr Gly Gly Leu Gly Ser
   755 760 765
   Gln Gly Ala Gly Arg Gly Gly Leu Gly Gly Gln Gly Ala Gly Ala Ala
   770 775 780
   Ala Ala Ala Ala Gly Gly Ala Gly Gln Gly Gly Tyr Gly Gly Leu Gly
   785 790 795 800
   Gly Gln Gly Ala Gly Gln Gly Gly Tyr Gly Gly Leu Gly Ser Gln Gly
   805 810 815
   Ala Gly Arg Gly Gly Leu Gly Gly Gln Gly Ala Gly Ala Ala Ala Ala
   820 825 830
   Ala Ala Gly Gly Ala Gly Gln Gly Gly Leu Gly Gly Gln Gly Ala Gly
   835 840 845
   Gln Gly Ala Gly Ala Ala Ala Ala Ala Ala Gly Gly Ala Gly Gln Gly
   850 855 860
   Gly Tyr Gly Gly Leu Gly Ser Gln Gly Ala Gly Arg Gly Gly Gln Gly
   865 870 875 880
   Ala Gly Ala Ala Ala Ala Ala Ala Gly Gly Ala Gly Gln Gly Gly Tyr
   885 890 895
   Gly Gly Leu Gly Gly Gln Gly Ala Gly Gln Gly Gly Tyr Gly Gly Leu
   900 905 910
   Gly Ser Gln Gly Ala Gly Arg Gly Gly Leu Gly Gly Gln Gly Ala Gly
   915 920 925
   Ala Ala Ala Ala Ala Ala Gly Gly Ala Gly Gln Gly Gly Leu Gly Gly
   930 935 940
   Gln Gly Ala Gly Gln Gly Ala Gly Ala Ala Ala Ala Ala Ala Gly Gly
   945 950 955 960
   Ala Gly Gln Gly Gly Tyr Gly Gly Leu Gly Ser Gln Gly Ala Gly Arg
   965 970 975
   Gly Gly Leu Gly Gly Gln Gly Ala Gly Ala Ala Ala Ala Ala Ala Gly
   980 985 990
   Gly Ala Gly Gln Gly Gly Tyr Gly Gly Leu Gly Ser Gln Gly Ala Gly
   995 1000 1005
   Arg Gly Gly Gln Gly Ala Gly Ala Ala Ala Ala Ala Ala Gly Gly Ala
   1010 1015 1020
   Gly Gln Gly Gly Tyr Gly Gly Leu Gly Ser Gln Gly Ala Gly Arg Gly
   1025 1030 1035 1040
   Gly Leu Gly Gly Gln Gly Ala Gly Ala Ala Ala Ala Ala Ala Gly Gly
   1045 1050 1055
   Ala Gly Gln Gly Gly Tyr Gly Gly Leu Gly Ser Gln Gly Ala Gly Arg
   1060 1065 1070
   Gly Gly Gln Gly Ala Gly Ala Ala Ala Ala Ala Ala Gly Gly Ala Gly
   1075 1080 1085
   Gln Gly Gly Tyr Gly Gly Leu Gly Ser Gln Gly Ala Gly Arg Gly Gly
   1090 1095 1100
   Gln Gly Ala Gly Ala Ala Ala Ala Ala Ala Gly Gly Ala Gly Gln Gly
   1105 1110 1115 1120
   Gly Tyr Gly Gly Leu Gly Gly Gln Gly Ala Gly Gln Gly Gly Tyr Gly
   1125 1130 1135
   Gly Leu Gly Ser Gln Gly Ala Gly Arg Gly Gly Leu Gly Gly Gln Gly
   1140 1145 1150
   Ala Gly Ala Ala Ala Ala Ala Ala Gly Gly Ala Gly Gln Gly Gly Leu
   1155 1160 1165
   Gly Gly Gln Gly Ala Gly Gln Gly Ala Gly Ala Ala Ala Ala Ala Ala
   1170 1175 1180
   Gly Gly Ala Gly Gln Gly Gly Tyr Gly Gly Leu Gly Ser Gln Gly Ala
   1185 1190 1195 1200
   Gly Arg Gly Gly Gln Gly Ala Gly Ala Ala Ala Ala Ala Ala Gly Gly
   1205 1210 1215
   Ala Gly Gln Gly Gly Tyr Gly Gly Leu Gly Ser Gln Gly Ala Gly Arg
   1220 1225 1230
   Gly Gly Leu Gly Gly Gln Gly Ala Gly Ala Ala Ala Ala Ala Ala Gly
   1235 1240 1245
   Gly Ala Gly Gly Gln Ala Ala
   1250 1255
<210> 34
   <211> 989
   <212> PRT
   <213> Künstliche Sequenz
<220>
   <223> Beschreibung der künstlichen Sequenz:SO1SM12-Protein
<400> 34
   Gly Gln Gly Gly Tyr Gly Gly Leu Gly Gly Gln Gly Ala Gly Gln Gly
   1 5 10 15
   Gly Tyr Gly Gly Leu Gly Gly Gln Gly Ala Gly Gln Gly Ala Gly Ala
   20 25 30
   Ala Ala Ala Ala Ala Gly Gly Ala Gly Gln Gly Gly Tyr Gly Gly Leu
   35 40 45
   Gly Ser Gln Gly Ala Gly Arg Gly Gly Gln Gly Ala Gly Ala Ala Ala
   50 55 60
   Ala Ala Ala Gly Gly Ala Gly Gln Gly Gly Tyr Gly Gly Leu Gly Ser
   65 70 75 80
   Gln Gly Ala Gly Arg Gly Gly Leu Gly Gly Gln Gly Ala Gly Ala Ala
   85 90 95
   Ala Ala Ala Ala Gly Gly Ala Gly Gln Gly Gly Tyr Gly Gly Leu Gly
   100 105 110
   Ser Gln Gly Ala Gly Arg Gly Gly Gln Gly Ala Gly Ala Ala Ala Ala
   115 120 125
   Ala Ala Gly Gly Ala Gly Gln Gly Gly Tyr Gly Gly Leu Gly Ser Gln
   130 135 140
   Gly Ala Gly Arg Gly Gly Leu Gly Gly Gln Gly Ala Gly Ala Ala Ala
   145 150 155 160
   Ala Ala Ala Gly Gly Ala Gly Gln Gly Gly Tyr Gly Gly Leu Gly Gly
   165 170 175
   Gln Gly Ala Gly Gln Gly Gly Tyr Gly Gly Leu Gly Ser Gln Gly Ala
   180 185 190
   Gly Arg Gly Gly Leu Gly Gly Gln Gly Ala Gly Ala Ala Ala Ala Ala
   195 200 205
   Ala Gly Gly Ala Gly Gln Gly Gly Leu Gly Gly Gln Gly Ala Gly Gln
   210 215 220
   Gly Ala Gly Ala Ala Ala Ala Ala Ala Gly Gly Ala Gly Gln Gly Gly
   225 230 235 240
   Tyr Gly Gly Leu Gly Ser Gln Gly Ala Gly Arg Gly Gly Gln Gly Ala
   245 250 255
   Gly Ala Ala Ala Ala Ala Ala Gly Gly Ala Gly Gln Gly Gly Tyr Gly
   260 265 270
   Gly Leu Gly Gly Gln Gly Ala Gly Gln Gly Gly Tyr Gly Gly Leu Gly
   275 280 285
   Ser Gln Gly Ala Gly Arg Gly Gly Leu Gly Gly Gln Gly Ala Gly Ala
   290 295 300
   Ala Ala Ala Ala Ala Gly Gly Ala Gly Gln Gly Gly Leu Gly Gly Gln
   305 310 315 320
   Gly Ala Gly Gln Gly Ala Gly Ala Ala Ala Ala Ala Ala Gly Gly Ala
   325 330 335
   Gly Gln Gly Gly Tyr Gly Gly Leu Gly Ser Gln Gly Ala Gly Arg Gly
   340 345 350
   Gly Leu Gly Gly Gln Gly Ala Gly Ala Ala Ala Ala Ala Ala Gly Gly
   355 360 365
   Ala Gly Gln Gly Gly Tyr Gly Gly Leu Gly Ser Gln Gly Ala Gly Arg
   370 375 380
   Gly Gly Gln Gly Ala Gly Ala Ala Ala Ala Ala Ala Gly Gly Ala Gly
   385 390 395 400
   Gln Gly Gly Tyr Gly Gly Leu Gly Ser Gln Gly Ala Gly Arg Gly Gly
   405 410 415
   Leu Gly Gly Gln Gly Ala Gly Ala Ala Ala Ala Ala Ala Gly Gly Ala
   420 425 430
   Gly Gln Gly Gly Tyr Gly Gly Leu Gly Ser Gln Gly Ala Gly Arg Gly
   435 440 445
   Gly Gln Gly Ala Gly Ala Ala Ala Ala Ala Ala Gly Gly Ala Gly Gln
   450 455 460
   Gly Gly Tyr Gly Gly Leu Gly Ser Gln Gly Ala Gly Arg Gly Gly Gln
   465 470 475 480
   Gly Ala Gly Ala Ala Ala Ala Ala Ala Gly Gly Ala Gly Gln Gly Gly
   485 490 495
   Tyr Gly Gly Leu Gly Gly Gln Gly Ala Gly Gln Gly Gly Tyr Gly Gly
   500 505 510
   Leu Gly Ser Gln Gly Ala Gly Arg Gly Gly Leu Gly Gly Gln Gly Ala
   515 520 525
   Gly Ala Ala Ala Ala Ala Ala Gly Gly Ala Gly Gln Gly Gly Leu Gly
   530 535 540
   Gly Gln Gly Ala Gly Gln Gly Ala Gly Ala Ala Ala Ala Ala Ala Gly
   545 550 555 560
   Gly Ala Gly Gln Gly Gly Tyr Gly Gly Leu Gly Ser Gln Gly Ala Gly
   565 570 575
   Arg Gly Gly Gln Gly Ala Gly Ala Ala Ala Ala Ala Ala Gly Gly Ala
   580 585 590
   Gly Gln Gly Gly Tyr Gly Gly Leu Gly Ser Gln Gly Ala Gly Arg Gly
   595 600 605
   Gly Leu Gly Gly Gln Gly Ala Gly Ala Ala Ala Ala Ala Ala Gly Gly
   610 615 620
   Ala Gly Gln Gly Gly Tyr Gly Gly Leu Gly Gly Gln Gly Ala Gly Gln
   625 630 635 640
   Gly Gly Tyr Gly Gly Leu Gly Ser Gln Gly Ala Gly Arg Gly Gly Leu
   645 650 655
   Gly Gly Gln Gly Ala Gly Ala Ala Ala Ala Ala Ala Gly Gly Ala Gly
   660 665 670
   Gln Gly Gly Leu Gly Gly Gln Gly Ala Gly Gln Gly Ala Gly Ala Ala
   675 680 685
   Ala Ala Ala Ala Gly Gly Ala Gly Gln Gly Gly Tyr Gly Gly Leu Gly
   690 695 700
   Ser Gln Gly Ala Gly Arg Gly Gly Leu Gly Gly Gln Gly Ala Gly Ala
   705 710 715 720
   Ala Ala Ala Ala Ala Gly Gly Ala Gly Gln Gly Gly Tyr Gly Gly Leu
   725 730 735
   Gly Ser Gln Gly Ala Gly Arg Gly Gly Gln Gly Ala Gly Ala Ala Ala
   740 745 750
   Ala Ala Ala Gly Gly Ala Gly Gln Gly Gly Tyr Gly Gly Leu Gly Ser
   755 760 765
   Gln Gly Ala Gly Arg Gly Gly Leu Gly Gly Gln Gly Ala Gly Ala Ala
   770 775 780
   Ala Ala Ala Ala Gly Gly Ala Gly Gln Gly Gly Tyr Gly Gly Leu Gly
   785 790 795 800
   Ser Gln Gly Ala Gly Arg Gly Gly Gln Gly Ala Gly Ala Ala Ala Ala
   805 810 815
   Ala Ala Gly Gly Ala Gly Gln Gly Gly Tyr Gly Gly Leu Gly Ser Gln
   820 825 830
   Gly Ala Gly Arg Gly Gly Gln Gly Ala Gly Ala Ala Ala Ala Ala Ala
   835 840 845
   Gly Gly Ala Gly Gln Gly Gly Tyr Gly Gly Leu Gly Gly Gln Gly Ala
   850 855 860
   Gly Gln Gly Gly Tyr Gly Gly Leu Gly Ser Gln Gly Ala Gly Arg Gly
   865 870 875 880
   Gly Leu Gly Gly Gln Gly Ala Gly Ala Ala Ala Ala Ala Ala Gly Gly
   885 890 895
   Ala Gly Gln Gly Gly Leu Gly Gly Gln Gly Ala Gly Gln Gly Ala Gly
   900 905 910
   Ala Ala Ala Ala Ala Ala Gly Gly Ala Gly Gln Gly Gly Tyr Gly Gly
   915 920 925
   Leu Gly Ser Gln Gly Ala Gly Arg Gly Gly Gln Gly Ala Gly Ala Ala
   930 935 940
   Ala Ala Ala Ala Gly Gly Ala Gly Gln Gly Gly Tyr Gly Gly Leu Gly
   945 950 955 960
   Ser Gln Gly Ala Gly Arg Gly Gly Leu Gly Gly Gln Gly Ala Gly Ala
   965 970 975
   Ala Ala Ala Ala Ala Gly Gly Ala Gly Gly Gln Ala Ala
   980 985
<210> 35
   <211> 1880
   <212> PRT
   <213> Künstliche Sequenz
<220>
   <223> Beschreibung der künstlichen Sequenz:SO1SO1SO1-Protein
<400> 35
   Gly Gln Gly Gly Tyr Gly Gly Leu Gly Gly Gln Gly Ala Gly Gln Gly
   1 5 10 15
   Gly Tyr Gly Gly Leu Gly Gly Gln Gly Ala Gly Gln Gly Ala Gly Ala
   20 25 30
   Ala Ala Ala Ala Ala Gly Gly Ala Gly Gln Gly Gly Tyr Gly Gly Leu
   35 40 45
   Gly Ser Gln Gly Ala Gly Arg Gly Gly Gln Gly Ala Gly Ala Ala Ala
   50 55 60
   Ala Ala Ala Gly Gly Ala Gly Gln Gly Gly Tyr Gly Gly Leu Gly Ser
   65 70 75 80
   Gln Gly Ala Gly Arg Gly Gly Leu Gly Gly Gln Gly Ala Gly Ala Ala
   85 90 95
   Ala Ala Ala Ala Gly Gly Ala Gly Gln Gly Gly Tyr Gly Gly Leu Gly
   100 105 110
   Ser Gln Gly Ala Gly Arg Gly Gly Gln Gly Ala Gly Ala Ala Ala Ala
   115 120 125
   Ala Ala Gly Gly Ala Gly Gln Gly Gly Tyr Gly Gly Leu Gly Ser Gln
   130 135 140
   Gly Ala Gly Arg Gly Gly Leu Gly Gly Gln Gly Ala Gly Ala Ala Ala
   145 150 155 160
   Ala Ala Ala Gly Gly Ala Gly Gln Gly Gly Tyr Gly Gly Leu Gly Gly
   165 170 175
   Gln Gly Ala Gly Gln Gly Gly Tyr Gly Gly Leu Gly Ser Gln Gly Ala
   180 185 190
   Gly Arg Gly Gly Leu Gly Gly Gln Gly Ala Gly Ala Ala Ala Ala Ala
   195 200 205
   Ala Gly Gly Ala Gly Gln Gly Gly Leu Gly Gly Gln Gly Ala Gly Gln
   210 215 220
   Gly Ala Gly Ala Ala Ala Ala Ala Ala Gly Gly Ala Gly Gln Gly Gly
   225 230 235 240
   Tyr Gly Gly Leu Gly Ser Gln Gly Ala Gly Arg Gly Gly Gln Gly Ala
   245 250 255
   Gly Ala Ala Ala Ala Ala Ala Gly Gly Ala Gly Gln Gly Gly Tyr Gly
   260 265 270
   Gly Leu Gly Gly Gln Gly Ala Gly Gln Gly Gly Tyr Gly Gly Leu Gly
   275 280 285
   Ser Gln Gly Ala Gly Arg Gly Gly Leu Gly Gly Gln Gly Ala Gly Ala
   290 295 300
   Ala Ala Ala Ala Ala Gly Gly Ala Gly Gln Gly Gly Leu Gly Gly Gln
   305 310 315 320
   Gly Ala Gly Gln Gly Ala Gly Ala Ala Ala Ala Ala Ala Gly Gly Ala
   325 330 335
   Gly Gln Gly Gly Tyr Gly Gly Leu Gly Ser Gln Gly Ala Gly Arg Gly
   340 345 350
   Gly Leu Gly Gly Gln Gly Ala Gly Ala Ala Ala Ala Ala Ala Gly Gly
   355 360 365
   Ala Gly Gln Gly Gly Tyr Gly Gly Leu Gly Ser Gln Gly Ala Gly Arg
   370 375 380
   Gly Gly Gln Gly Ala Gly Ala Ala Ala Ala Ala Ala Gly Gly Ala Gly
   385 390 395 400
   Gln Gly Gly Tyr Gly Gly Leu Gly Ser Gln Gly Ala Gly Arg Gly Gly
   405 410 415
   Leu Gly Gly Gln Gly Ala Gly Ala Ala Ala Ala Ala Ala Gly Gly Ala
   420 425 430
   Gly Gln Gly Gly Tyr Gly Gly Leu Gly Ser Gln Gly Ala Gly Arg Gly
   435 440 445
   Gly Gln Gly Ala Gly Ala Ala Ala Ala Ala Ala Gly Gly Ala Gly Gln
   450 455 460
   Gly Gly Tyr Gly Gly Leu Gly Ser Gln Gly Ala Gly Arg Gly Gly Gln
   465 470 475 480
   Gly Ala Gly Ala Ala Ala Ala Ala Ala Gly Gly Ala Gly Gln Gly Gly
   485 490 495
   Tyr Gly Gly Leu Gly Gly Gln Gly Ala Gly Gln Gly Gly Tyr Gly Gly
   500 505 510
   Leu Gly Ser Gln Gly Ala Gly Arg Gly Gly Leu Gly Gly Gln Gly Ala
   515 520 525
   Gly Ala Ala Ala Ala Ala Ala Gly Gly Ala Gly Gln Gly Gly Leu Gly
   530 535 540
   Gly Gln Gly Ala Gly Gln Gly Ala Gly Ala Ala Ala Ala Ala Ala Gly
   545 550 555 560
   Gly Ala Gly Gln Gly Gly Tyr Gly Gly Leu Gly Ser Gln Gly Ala Gly
   565 570 575
   Arg Gly Gly Gln Gly Ala Gly Ala Ala Ala Ala Ala Ala Gly Gly Ala
   580 585 590
   Gly Gln Gly Gly Tyr Gly Gly Leu Gly Ser Gln Gly Ala Gly Arg Gly
   595 600 605
   Gly Leu Gly Gly Gln Gly Ala Gly Ala Ala Ala Ala Ala Ala Gly Gly
   610 615 620
   Ala Gly Gln Gly Gly Tyr Gly Gly Leu Gly Gly Gln Gly Ala Gly Gln
   625 630 635 640
   Gly Gly Tyr Gly Gly Leu Gly Gly Gln Gly Ala Gly Gln Gly Ala Gly
   645 650 655
   Ala Ala Ala Ala Ala Ala Gly Gly Ala Gly Gln Gly Gly Tyr Gly Gly
   660 665 670
   Leu Gly Ser Gln Gly Ala Gly Arg Gly Gly Gln Gly Ala Gly Ala Ala
   675 680 685
   Ala Ala Ala Ala Gly Gly Ala Gly Gln Gly Gly Tyr Gly Gly Leu Gly
   690 695 700
   Ser Gln Gly Ala Gly Arg Gly Gly Leu Gly Gly Gln Gly Ala Gly Ala
   705 710 715 720
   Ala Ala Ala Ala Ala Gly Gly Ala Gly Gln Gly Gly Tyr Gly Gly Leu
   725 730 735
   Gly Ser Gln Gly Ala Gly Arg Gly Gly Gln Gly Ala Gly Ala Ala Ala
   740 745 750
   Ala Ala Ala Gly Gly Ala Gly Gln Gly Gly Tyr Gly Gly Leu Gly Ser
   755 760 765
   Gln Gly Ala Gly Arg Gly Gly Leu Gly Gly Gln Gly Ala Gly Ala Ala
   770 775 780
   Ala Ala Ala Ala Gly Gly Ala Gly Gln Gly Gly Tyr Gly Gly Leu Gly
   785 790 795 800
   Gly Gln Gly Ala Gly Gln Gly Gly Tyr Gly Gly Leu Gly Ser Gln Gly
   805 810 815
   Ala Gly Arg Gly Gly Leu Gly Gly Gln Gly Ala Gly Ala Ala Ala Ala
   820 825 830
   Ala Ala Gly Gly Ala Gly Gln Gly Gly Leu Gly Gly Gln Gly Ala Gly
   835 840 845
   Gln Gly Ala Gly Ala Ala Ala Ala Ala Ala Gly Gly Ala Gly Gln Gly
   850 855 860
   Gly Tyr Gly Gly Leu Gly Ser Gln Gly Ala Gly Arg Gly Gly Gln Gly
   865 870 875 880
   Ala Gly Ala Ala Ala Ala Ala Ala Gly Gly Ala Gly Gln Gly Gly Tyr
   885 890 895
   Gly Gly Leu Gly Gly Gln Gly Ala Gly Gln Gly Gly Tyr Gly Gly Leu
   900 905 910
   Gly Ser Gln Gly Ala Gly Arg Gly Gly Leu Gly Gly Gln Gly Ala Gly
   915 920 925
   Ala Ala Ala Ala Ala Ala Gly Gly Ala Gly Gln Gly Gly Leu Gly Gly
   930 935 940
   Gln Gly Ala Gly Gln Gly Ala Gly Ala Ala Ala Ala Ala Ala Gly Gly
   945 950 955 960
   Ala Gly Gln Gly Gly Tyr Gly Gly Leu Gly Ser Gln Gly Ala Gly Arg
   965 970 975
   Gly Gly Leu Gly Gly Gln Gly Ala Gly Ala Ala Ala Ala Ala Ala Gly
   980 985 990
   Gly Ala Gly Gln Gly Gly Tyr Gly Gly Leu Gly Ser Gln Gly Ala Gly
   995 1000 1005
   Arg Gly Gly Gln Gly Ala Gly Ala Ala Ala Ala Ala Ala Gly Gly Ala
   1010 1015 1020
   Gly Gln Gly Gly Tyr Gly Gly Leu Gly Ser Gln Gly Ala Gly Arg Gly
   1025 1030 1035 1040
   Gly Leu Gly Gly Gln Gly Ala Gly Ala Ala Ala Ala Ala Ala Gly Gly
   1045 1050 1055
   Ala Gly Gln Gly Gly Tyr Gly Gly Leu Gly Ser Gln Gly Ala Gly Arg
   1060 1065 1070
   Gly Gly Gln Gly Ala Gly Ala Ala Ala Ala Ala Ala Gly Gly Ala Gly
   1075 1080 1085
   Gln Gly Gly Tyr Gly Gly Leu Gly Ser Gln Gly Ala Gly Arg Gly Gly
   1090 1095 1100
   Gln Gly Ala Gly Ala Ala Ala Ala Ala Ala Gly Gly Ala Gly Gln Gly
   1105 1110 1115 1120
   Gly Tyr Gly Gly Leu Gly Gly Gln Gly Ala Gly Gln Gly Gly Tyr Gly
   1125 1130 1135
   Gly Leu Gly Ser Gln Gly Ala Gly Arg Gly Gly Leu Gly Gly Gln Gly
   1140 1145 1150
   Ala Gly Ala Ala Ala Ala Ala Ala Gly Gly Ala Gly Gln Gly Gly Leu
   1155 1160 1165
   Gly Gly Gln Gly Ala Gly Gln Gly Ala Gly Ala Ala Ala Ala Ala Ala
   1170 1175 1180
   Gly Gly Ala Gly Gln Gly Gly Tyr Gly Gly Leu Gly Ser Gln Gly Ala
   1185 1190 1195 1200
   Gly Arg Gly Gly Gln Gly Ala Gly Ala Ala Ala Ala Ala Ala Gly Gly
   1205 1210 1215
   Ala Gly Gln Gly Gly Tyr Gly Gly Leu Gly Ser Gln Gly Ala Gly Arg
   1220 1225 1230
   Gly Gly Leu Gly Gly Gln Gly Ala Gly Ala Ala Ala Ala Ala Ala Gly
   1235 1240 1245
   Gly Ala Gly Gln Gly Gly Tyr Gly Gly Leu Gly Gly Gln Gly Ala Gly
   1250 1255 1260
   Gln Gly Gly Tyr Gly Gly Leu Gly Gly Gln Gly Ala Gly Gln Gly Ala
   1265 1270 1275 1280
   Gly Ala Ala Ala Ala Ala Ala Gly Gly Ala Gly Gln Gly Gly Tyr Gly
   1285 1290 1295
   Gly Leu Gly Ser Gln Gly Ala Gly Arg Gly Gly Gln Gly Ala Gly Ala
   1300 1305 1310
   Ala Ala Ala Ala Ala Gly Gly Ala Gly Gln Gly Gly Tyr Gly Gly Leu
   1315 1320 1325
   Gly Ser Gln Gly Ala Gly Arg Gly Gly Leu Gly Gly Gln Gly Ala Gly
   1330 1335 1340
   Ala Ala Ala Ala Ala Ala Gly Gly Ala Gly Gln Gly Gly Tyr Gly Gly
   1345 1350 1355 1360
   Leu Gly Ser Gln Gly Ala Gly Arg Gly Gly Gln Gly Ala Gly Ala Ala
   1365 1370 1375
   Ala Ala Ala Ala Gly Gly Ala Gly Gln Gly Gly Tyr Gly Gly Leu Gly
   1380 1385 1390
   Ser Gln Gly Ala Gly Arg Gly Gly Leu Gly Gly Gln Gly Ala Gly Ala
   1395 1400 1405
   Ala Ala Ala Ala Ala Gly Gly Ala Gly Gln Gly Gly Tyr Gly Gly Leu
   1410 1415 1420
   Gly Gly Gln Gly Ala Gly Gln Gly Gly Tyr Gly Gly Leu Gly Ser Gln
   1425 1430 1435 1440
   Gly Ala Gly Arg Gly Gly Leu Gly Gly Gln Gly Ala Gly Ala Ala Ala
   1445 1450 1455
   Ala Ala Ala Gly Gly Ala Gly Gln Gly Gly Leu Gly Gly Gln Gly Ala
   1460 1465 1470
   Gly Gln Gly Ala Gly Ala Ala Ala Ala Ala Ala Gly Gly Ala Gly Gln
   1475 1480 1485
   Gly Gly Tyr Gly Gly Leu Gly Ser Gln Gly Ala Gly Arg Gly Gly Gln
   1490 1495 1500
   Gly Ala Gly Ala Ala Ala Ala Ala Ala Gly Gly Ala Gly Gln Gly Gly
   1505 1510 1515 1520
   Tyr Gly Gly Leu Gly Gly Gln Gly Ala Gly Gln Gly Gly Tyr Gly Gly
   1525 1530 1535
   Leu Gly Ser Gln Gly Ala Gly Arg Gly Gly Leu Gly Gly Gln Gly Ala
   1540 1545 1550
   Gly Ala Ala Ala Ala Ala Ala Gly Gly Ala Gly Gln Gly Gly Leu Gly
   1555 1560 1565
   Gly Gln Gly Ala Gly Gln Gly Ala Gly Ala Ala Ala Ala Ala Ala Gly
   1570 1575 1580
   Gly Ala Gly Gln Gly Gly Tyr Gly Gly Leu Gly Ser Gln Gly Ala Gly
   1585 1590 1595 1600
   Arg Gly Gly Leu Gly Gly Gln Gly Ala Gly Ala Ala Ala Ala Ala Ala
   1605 1610 1615
   Gly Gly Ala Gly Gln Gly Gly Tyr Gly Gly Leu Gly Ser Gln Gly Ala
   1620 1625 1630
   Gly Arg Gly Gly Gln Gly Ala Gly Ala Ala Ala Ala Ala Ala Gly Gly
   1635 1640 1645
   Ala Gly Gln Gly Gly Tyr Gly Gly Leu Gly Ser Gln Gly Ala Gly Arg
   1650 1655 1660
   Gly Gly Leu Gly Gly Gln Gly Ala Gly Ala Ala Ala Ala Ala Ala Gly
   1665 1670 1675 1680
   Gly Ala Gly Gln Gly Gly Tyr Gly Gly Leu Gly Ser Gln Gly Ala Gly
   1685 1690 1695
   Arg Gly Gly Gln Gly Ala Gly Ala Ala Ala Ala Ala Ala Gly Gly Ala
   1700 1705 1710
   Gly Gln Gly Gly Tyr Gly Gly Leu Gly Ser Gln Gly Ala Gly Arg Gly
   1715 1720 1725
   Gly Gln Gly Ala Gly Ala Ala Ala Ala Ala Ala Gly Gly Ala Gly Gln
   1730 1735 1740
   Gly Gly Tyr Gly Gly Leu Gly Gly Gln Gly Ala Gly Gln Gly Gly Tyr
   1745 1750 1755 1760
   Gly Gly Leu Gly Ser Gln Gly Ala Gly Arg Gly Gly Leu Gly Gly Gln
   1765 1770 1775
   Gly Ala Gly Ala Ala Ala Ala Ala Ala Gly Gly Ala Gly Gln Gly Gly
   1780 1785 1790
   Leu Gly Gly Gln Gly Ala Gly Gln Gly Ala Gly Ala Ala Ala Ala Ala
   1795 1800 1805
   Ala Gly Gly Ala Gly Gln Gly Gly Tyr Gly Gly Leu Gly Ser Gln Gly
   1810 1815 1820
   Ala Gly Arg Gly Gly Gln Gly Ala Gly Ala Ala Ala Ala Ala Ala Gly
   1825 1830 1835 1840
   Gly Ala Gly Gln Gly Gly Tyr Gly Gly Leu Gly Ser Gln Gly Ala Gly
   1845 1850 1855
   Arg Gly Gly Leu Gly Gly Gln Gly Ala Gly Ala Ala Ala Ala Ala Ala
   1860 1865 1870
   Gly Gly Ala Gly Gly Gln Ala Ala
   1875 1880
<210> 36
   <211> 219
   <212> PRT
   <213> Künstliche Sequenz
<220>
   <223> Beschreibung der künstlichen Sequenz:FA2-Protein
<400> 36
   Gly Gln Gly Gly Tyr Gly Gly Leu Gly Gly Gln Gly Ala Gly Gln Gly
   1 5 10 15
   Gly Tyr Gly Gly Leu Gly Ser Gln Gly Ala Gly Arg Gly Gly Leu Gly
   20 25 30
   Gly Gln Gly Ala Gly Ala Ala Ala Ala Ala Ala Gly Gly Ala Gly Gln
   35 40 45
   Gly Gly Leu Gly Gly Gln Gly Ala Gly Gln Gly Ala Gly Ala Ala Ala
   50 55 60
   Ala Ala Ala Gly Gly Ala Gly Gln Gly Gly Tyr Gly Gly Leu Gly Ser
   65 70 75 80
   Gln Gly Ala Gly Arg Gly Gly Leu Gly Gly Gln Gly Ala Gly Ala Ala
   85 90 95
   Ala Ala Ala Ala Gly Gly Ala Gly Ser Gly Ser Gly Ala Gly Ala Gly
   100 105 110
   Ser Gly Ala Gly Ala Gly Ala Gly Ser Gly Ala Gly Ala Gly Ser Gly
   115 120 125
   Ala Gly Ala Gly Ser Gly Ala Gly Ala Gly Ser Gly Ala Gly Ala Gly
   130 135 140
   Tyr Gly Ala Gly Ala Gly Val Gly Tyr Gly Ala Gly Tyr Gly Ala Gly
   145 150 155 160
   Ala Gly Val Gly Tyr Gly Ala Gly Ala Gly Ser Gly Ala Ala Ser Gly
   165 170 175
   Ala Gly Ala Gly Ala Gly Ala Gly Thr Gly Ser Ser Gly Phe Gly Pro
   180 185 190
   Tyr Val Ala Asn Gly Gly Tyr Ser Gly Tyr Glu Tyr Ala Trp Ser Ser
   195 200 205
   Lys Ser Asp Phe Glu Thr Ala Gly Gln Ala Ala
   210 215
<210> 37
   <211> 170
   <212> PRT
   <213> Künstliche Sequenz
<220>
   <223> Beschreibung der künstlichen Sequenz:SA1-Protein
<400> 37
   Gly Gln Gly Gly Tyr Gly Gly Leu Gly Gly Gln Gly Ala Gly Gln Gly
     1               5                  10                  15
   Gly Tyr Gly Gly Leu Gly Gly Gln Gly Ala Gly Gln Gly Ala Gly Ala
                20                  25                  30
   Ala Ala Ala Ala Ala Gly Gly Ala Gly Gln Gly Gly Tyr Gly Gly Leu
            35                  40                  45
   Gly Ser Gln Gly Ala Gly Arg Gly Gly Gln Gly Ala Gly Ala Ala Ala
        50                  55                  60
   Ala Ala Ala Gly Gly Ala Gly Gln Gly Gly Tyr Gly Gly Leu Gly Ser
    65                  70                  75                  80
   Gln Gly Ala Gly Arg Gly Gly Leu Gly Gly Gln Gly Ala Gly Ala Ala
                    85                  90                  95
   Ala Ala Ala Ala Gly Gly Ala Gly Gln Gly Gly Tyr Gly Gly Leu Gly
               100                 105                 110
   Ser Gln Gly Ala Gly Arg Gly Gly Gln Gly Ala Gly Ala Ala Ala Ala
           115                 120                 125
   Ala Ala Gly Gly Ala Gly Gln Gly Gly Tyr Gly Gly Leu Gly Ser Gln
       130                 135                 140
   Gly Ala Gly Arg Gly Gly Leu Gly Gly Gln Gly Ala Gly Ala Ala Ala
   145                 150                 155                 160
   Ala Ala Ala Gly Gly Ala Gly Gln Ala Ala
                   165                 170
<210> 38
   <211> 630
   <212> PRT
   <213> Künstliche Sequenz
<220>
   <223> Beschreibung der künstlichen Sequenz:SO1-Protein
<400> 38
   Gly Gln Gly Gly Tyr Gly Gly Leu Gly Gly Gln Gly Ala Gly Gln Gly
    1               5                  10                  15
   Gly Tyr Gly Gly Leu Gly Gly Gln Gly Ala Gly Gln Gly Ala Gly Ala
               20                  25                  30
   Ala Ala Ala Ala Ala Gly Gly Ala Gly Gln Gly Gly Tyr Gly Gly Leu
           35                  40                  45
   Gly Ser Gln Gly Ala Gly Arg Gly Gly Gln Gly Ala Gly Ala Ala Ala
       50                  55                  60
   Ala Ala Ala Gly Gly Ala Gly Gln Gly Gly Tyr Gly Gly Leu Gly Ser
   65                  70                  75                  80
   Gln Gly Ala Gly Arg Gly Gly Leu Gly Gly Gln Gly Ala Gly Ala Ala
                   85                  90                  95
   Ala Ala Ala Ala Gly Gly Ala Gly Gln Gly Gly Tyr Gly Gly Leu Gly
               100                 105                 110
   Ser Gln Gly Ala Gly Arg Gly Gly Gln Gly Ala Gly Ala Ala Ala Ala
           115                 120                 125
   Ala Ala Gly Gly Ala Gly Gln Gly Gly Tyr Gly Gly Leu Gly Ser Gln
       130                 135                 140
   Gly Ala Gly Arg Gly Gly Leu Gly Gly Gln Gly Ala Gly Ala Ala Ala
   145                 150                 155                 160
   Ala Ala Ala Gly Gly Ala Gly Gln Gly Gly Tyr Gly Gly Leu Gly Gly
                   165                 170                 175
   Gln Gly Ala Gly Gln Gly Gly Tyr Gly Gly Leu Gly Ser Gln Gly Ala
               180                 185                 190
   Gly Arg Gly Gly Leu Gly Gly Gln Gly Ala Gly Ala Ala Ala Ala Ala
   195 200 205
   Ala Gly Gly Ala Gly Gln Gly Gly Leu Gly Gly Gln Gly Ala Gly Gln
   210 215 220
   Gly Ala Gly Ala Ala Ala Ala Ala Ala Gly Gly Ala Gly Gln Gly Gly
   225 230 235 240
   Tyr Gly Gly Leu Gly Ser Gln Gly Ala Gly Arg Gly Gly Gln Gly Ala
   245 250 255
   Gly Ala Ala Ala Ala Ala Ala Gly Gly Ala Gly Gln Gly Gly Tyr Gly
   260 265 270
   Gly Leu Gly Gly Gln Gly Ala Gly Gln Gly Gly Tyr Gly Gly Leu Gly
   275 280 285
   Ser Gln Gly Ala Gly Arg Gly Gly Leu Gly Gly Gln Gly Ala Gly Ala
   290 295 300
   Ala Ala Ala Ala Ala Gly Gly Ala Gly Gln Gly Gly Leu Gly Gly Gln
   305 310 315 320
   Gly Ala Gly Gln Gly Ala Gly Ala Ala Ala Ala Ala Ala Gly Gly Ala
   325 330 335
   Gly Gln Gly Gly Tyr Gly Gly Leu Gly Ser Gln Gly Ala Gly Arg Gly
   340 345 350
   Gly Leu Gly Gly Gln Gly Ala Gly Ala Ala Ala Ala Ala Ala Gly Gly
   355 360 365
   Ala Gly Gln Gly Gly Tyr Gly Gly Leu Gly Ser Gln Gly Ala Gly Arg
   370 375 380
   Gly Gly Gln Gly Ala Gly Ala Ala Ala Ala Ala Ala Gly Gly Ala Gly
   385 390 395 400
   Gln Gly Gly Tyr Gly Gly Leu Gly Ser Gln Gly Ala Gly Arg Gly Gly
   405 410 415
   Leu Gly Gly Gln Gly Ala Gly Ala Ala Ala Ala Ala Ala Gly Gly Ala
   420 425 430
   Gly Gln Gly Gly Tyr Gly Gly Leu Gly Ser Gln Gly Ala Gly Arg Gly
   435 440 445
   Gly Gln Gly Ala Gly Ala Ala Ala Ala Ala Ala Gly Gly Ala Gly Gln
   450 455 460
   Gly Gly Tyr Gly Gly Leu Gly Ser Gln Gly Ala Gly Arg Gly Gly Gln
   465 470 475 480
   Gly Ala Gly Ala Ala Ala Ala Ala Ala Gly Gly Ala Gly Gln Gly Gly
   485 490 495
   Tyr Gly Gly Leu Gly Gly Gln Gly Ala Gly Gln Gly Gly Tyr Gly Gly
   500 505 510
   Leu Gly Ser Gln Gly Ala Gly Arg Gly Gly Leu Gly Gly Gln Gly Ala
   515 520 525
   Gly Ala Ala Ala Ala Ala Ala Gly Gly Ala Gly Gln Gly Gly Leu Gly
   530 535 540
   Gly Gln Gly Ala Gly Gln Gly Ala Gly Ala Ala Ala Ala Ala Ala Gly
   545 550 555 560
   Gly Ala Gly Gln Gly Gly Tyr Gly Gly Leu Gly Ser Gln Gly Ala Gly
   565 570 575
   Arg Gly Gly Gln Gly Ala Gly Ala Ala Ala Ala Ala Ala Gly Gly Ala
   580 585 590
   Gly Gln Gly Gly Tyr Gly Gly Leu Gly Ser Gln Gly Ala Gly Arg Gly
   595 600 605
   Gly Leu Gly Gly Gln Gly Ala Gly Ala Ala Ala Ala Ala Ala Gly Gly
   610 615 620
   Ala Gly Gly Gln Ala Ala
   625 630
<210> 39
   <211> 364
   <212> PRT
   <213> Künstliche Sequenz
<220>
   <223> Beschreibung der künstlichen Sequenz:SM12-Protein
<400> 39
   Gly Gln Gly Gly Tyr Gly Gly Leu Gly Gly Gln Gly Ala Gly Gln Gly
   1 5 10 15
   Gly Tyr Gly Gly Leu Gly Ser Gln Gly Ala Gly Arg Gly Gly Leu Gly
   20 25 30
   Gly Gln Gly Ala Gly Ala Ala Ala Ala Ala Ala Gly Gly Ala Gly Gln
   35 40 45
   Gly Gly Leu Gly Gly Gln Gly Ala Gly Gln Gly Ala Gly Ala Ala Ala
   50 55 60
   Ala Ala Ala Gly Gly Ala Gly Gln Gly Gly Tyr Gly Gly Leu Gly Ser
   65 70 75 80
   Gln Gly Ala Gly Arg Gly Gly Leu Gly Gly Gln Gly Ala Gly Ala Ala
   85 90 95
   Ala Ala Ala Ala Gly Gly Ala Gly Gln Gly Gly Tyr Gly Gly Leu Gly
   100 105 110
   Ser Gln Gly Ala Gly Arg Gly Gly Gln Gly Ala Gly Ala Ala Ala Ala
   115 120 125
   Ala Ala Gly Gly Ala Gly Gln Gly Gly Tyr Gly Gly Leu Gly Ser Gln
   130 135 140
   Gly Ala Gly Arg Gly Gly Leu Gly Gly Gln Gly Ala Gly Ala Ala Ala
   145 150 155 160
   Ala Ala Ala Gly Gly Ala Gly Gln Gly Gly Tyr Gly Gly Leu Gly Ser
   165 170 175
   Gln Gly Ala Gly Arg Gly Gly Gln Gly Ala Gly Ala Ala Ala Ala Ala
   180 185 190
   Ala Gly Gly Ala Gly Gln Gly Gly Tyr Gly Gly Leu Gly Ser Gln Gly
   195 200 205
   Ala Gly Arg Gly Gly Gln Gly Ala Gly Ala Ala Ala Ala Ala Ala Gly
   210 215 220
   Gly Ala Gly Gln Gly Gly Tyr Gly Gly Leu Gly Gly Gln Gly Ala Gly
   225 230 235 240
   Gln Gly Gly Tyr Gly Gly Leu Gly Ser Gln Gly Ala Gly Arg Gly Gly
   245 250 255
   Leu Gly Gly Gln Gly Ala Gly Ala Ala Ala Ala Ala Ala Gly Gly Ala
   260 265 270
   Gly Gln Gly Gly Leu Gly Gly Gln Gly Ala Gly Gln Gly Ala Gly Ala
   275 280 285
   Ala Ala Ala Ala Ala Gly Gly Ala Gly Gln Gly Gly Tyr Gly Gly Leu
   290 295 300
   Gly Ser Gln Gly Ala Gly Arg Gly Gly Gln Gly Ala Gly Ala Ala Ala
   305 310 315 320
   Ala Ala Ala Gly Gly Ala Gly Gln Gly Gly Tyr Gly Gly Leu Gly Ser
   325 330 335
   Gln Gly Ala Gly Arg Gly Gly Leu Gly Gly Gln Gly Ala Gly Ala Ala
   340 345 350
   Ala Ala Ala Ala Gly Gly Ala Gly Gly Gln Ala Ala
   355 360
<210> 40
   <211> 271
   <212> PRT
   <213> Künstliche Sequenz
<220>
   <223> Beschreibung der künstlichen Sequenz:SF1-Protein
<400> 40
   Gly Gln Gly Gly Tyr Gly Gly Leu Gly Gly Gln Gly Ala Gly Gln Gly
    1               5                  10                  15
   Gly Tyr Gly Gly Leu Gly Gly Gln Gly Ala Gly Gln Gly Ala Gly Ala
               20                  25                  30
   Ala Ala Ala Ala Ala Gly Gly Ala Gly Gln Gly Gly Tyr Gly Gly Leu
           35                  40                  45
   Gly Ser Gln Gly Ala Gly Arg Gly Gly Gln Gly Ala Gly Ala Ala Ala
       50                  55                  60
   Ala Ala Ala Gly Gly Ala Gly Gln Gly Gly Tyr Gly Gly Leu Gly Ser
   65                  70                  75                  80
   Gln Gly Ala Gly Arg Gly Gly Leu Gly Gly Gln Gly Ala Gly Ala Ala
                   85                  90                  95
   Ala Ala Ala Ala Gly Gly Ala Gly Gln Gly Gly Tyr Gly Gly Leu Gly
               100                 105                 110
   Ser Gln Gly Ala Gly Arg Gly Gly Gln Gly Ala Gly Ala Ala Ala Ala
           115                 120                 125
   Ala Ala Gly Gly Ala Gly Gln Gly Gly Tyr Gly Gly Leu Gly Ser Gln
       130                 135                 140
   Gly Ala Gly Arg Gly Gly Leu Gly Gly Gln Gly Ala Gly Ala Ala Ala
   145                 150                 155                 160
   Ala Ala Ala Gly Gly Ala Gly Gln Gly Gly Tyr Gly Gly Leu Gly Gly
                   165                 170                 175
   Gln Gly Ala Gly Gln Gly Gly Tyr Gly Gly Leu Gly Ser Gln Gly Ala
               180                 185                 190
   Gly Arg Gly Gly Leu Gly Gly Gln Gly Ala Gly Ala Ala Ala Ala Ala
           195                 200                 205
   Ala Gly Gly Ala Gly Gln Gly Gly Leu Gly Gly Gln Gly Ala Gly Gln
       210                 215                 220
   Gly Ala Gly Ala Ala Ala Ala Ala Ala Gly Gly Ala Gly Gln Gly Gly
   225                 230                 235                 240
   Tyr Gly Gly Leu Gly Ser Gln Gly Ala Gly Arg Gly Gly Gln Gly Ala
                   245                 250                 255
   Gly Ala Ala Ala Ala Ala Ala Gly Gly Ala Gly Gly Gln Ala Ala
               260                 265                 270
<210> 41
   <211> 182
   <212> DNA
   <213> Künstliche Sequenz
<220>
   <223> Beschreibung der künstlichen Sequenz:ELP mit 10 Pentamereinheiten
<400> 41
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgg gctggcggcc 180
   gc                                                                182
<210> 42
   <211> 332
   <212> DNA
   <213> Künstliche Sequenz
<220>
   <223> Beschreibung der künstlichen Sequenz:ELP mit 20 Pentamereinheiten
<400> 42
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgg gctggcggcc gc                               332
<210> 43
   <211> 482
   <212> DNA
   <213> Künstliche Sequenz
<220>
   <223> Beschreibung der künstlichen Sequenz:ELP mit 30 Pentamereinheiten
<400> 43
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgt gggtgttccg ggcgtgggtg ttccgggtgg cggtgtgccg 360
   ggcgcaggtg ttcctggtgt aggtgtgccg ggtgttggtg tgccgggtgt tggtgtacca 420
   ggtggcggtg ttccgggtgc aggcgttccg ggtggcggtg tgccgggcgg gctggcggcc 480
   gc                                                                482
<210> 44
   <211> 632
   <212> DNA
   <213> Künstliche Sequenz
<220>
   <223> Beschreibung der künstlichen Sequenz:ELP mit 40 Pentamereinheiten
<400> 44
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgt gggtgttccg ggcgtgggtg ttccgggtgg cggtgtgccg 360
   ggcgcaggtg ttcctggtgt aggtgtgccg ggtgttggtg tgccgggtgt tggtgtacca 420
   ggtggcggtg ttccgggtgc aggcgttccg ggtggcggtg tgccgggcgt gggtgttccg 480
   ggcgtgggtg ttccgggtgg cggtgtgccg ggcgcaggtg ttcctggtgt aggtgtgccg 540
   ggtgttggtg tgccgggtgt tggtgtacca ggtggcggtg ttccgggtgc aggcgttccg 600
   ggtggcggtg tgccgggcgg gctggcggcc gc                               632
<210> 45
   <211> 932
   <212> DNA
   <213> Künstliche Sequenz
<220>
   <223> Beschreibung der künstlichen Sequenz:ELP mit 60 Pentamereinheiten
<400> 45
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgt gggtgttccg ggcgtgggtg ttccgggtgg cggtgtgccg 360
   ggcgcaggtg ttcctggtgt aggtgtgccg ggtgttggtg tgccgggtgt tggtgtacca 420
   ggtggcggtg ttccgggtgc aggcgttccg ggtggcggtg tgccgggcgt gggtgttccg 480
   ggcgtgggtg ttccgggtgg cggtgtgccg ggcgcaggtg ttcctggtgt aggtgtgccg 540
   ggtgttggtg tgccgggtgt tggtgtacca ggtggcggtg ttccgggtgc aggcgttccg 600
   ggtggcggtg tgccgggcgt gggtgttccg ggcgtgggtg ttccgggtgg cggtgtgccg 660
   ggcgcaggtg ttcctggtgt aggtgtgccg ggtgttggtg tgccgggtgt tggtgtacca 720
   ggtggcggtg ttccgggtgc aggcgttccg ggtggcggtg tgccgggcgt gggtgttccg 780
   ggcgtgggtg ttccgggtgg cggtgtgccg ggcgcaggtg ttcctggtgt aggtgtgccg 840
   ggtgttggtg tgccgggtgt tggtgtacca ggtggcggtg ttccgggtgc aggcgttccg 900
   ggtggcggtg tgccgggcgg gctggcggcc gc                               932
<210> 46
   <211> 1082
   <212> DNA
   <213> Künstliche Sequenz
<220>
   <223> Beschreibung der künstlichen Sequenz:ELP mit 70 Pentamereinheiten
<400> 46
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgt gggtgttccg ggcgtgggtg ttccgggtgg cggtgtgccg 360
   ggcgcaggtg ttcctggtgt aggtgtgccg ggtgttggtg tgccgggtgt tggtgtacca 420
   ggtggcggtg ttccgggtgc aggcgttccg ggtggcggtg tgccgggcgt gggtgttccg 480
   ggcgtgggtg ttccgggtgg cggtgtgccg ggcgcaggtg ttcctggtgt aggtgtgccg 540
   ggtgttggtg tgccgggtgt tggtgtacca ggtggcggtg ttccgggtgc aggcgttccg 600
   ggtggcggtg tgccgggcgt gggtgttccg ggcgtgggtg ttccgggtgg cggtgtgccg 660
   ggcgcaggtg ttcctggtgt aggtgtgccg ggtgttggtg tgccgggtgt tggtgtacca 720
   ggtggcggtg ttccgggtgc aggcgttccg ggtggcggtg tgccgggcgt gggtgttccg 780
   ggcgtgggtg ttccgggtgg cggtgtgccg ggcgcaggtg ttcctggtgt aggtgtgccg 840
   ggtgttggtg tgccgggtgt tggtgtacca ggtggcggtg ttccgggtgc aggcgttccg 900
   ggtggcggtg tgccgggcgt gggtgttccg ggcgtgggtg ttccgggtgg cggtgtgccg 960
   ggcgcaggtg ttcctggtgt aggtgtgccg ggtgttggtg tgccgggtgt tggtgtacca 1020
   ggtggcggtg ttccgggtgc aggcgttccg ggtggcggtg tgccgggcgg gctggcggcc 1080
   gc 1082
<210> 47
   <211> 1532
   <212> DNA
   <213> Künstliche Sequenz
<220>
   <223> Beschreibung der künstlichen Sequenz:ELP mit 100 Pentamereinheiten
<400> 47
   ctcgagatgg gccacggcgt gggtgttccg ggcgtgggtg ttccgggtgg cggtgtgccg 60
   ggcgcaggtg ttcctggtgt aggtgtgccg ggtgttggtg tgccgggtgt tggtgtacca 120
   ggtggcggtg ttccgggtgc aggcgttccg ggtggcggtg tgccgggcgt gggtgttccg 180
   ggcgtgggtg ttccgggtgg cggtgtgccg ggcgcaggtg ttcctggtgt aggtgtgccg 240
   ggtgttggtg tgccgggtgt tggtgtacca ggtggcggtg ttccgggtgc aggcgttccg 300
   ggtggcggtg tgccgggcgt gggtgttccg ggcgtgggtg ttccgggtgg cggtgtgccg 360
   ggcgcaggtg ttcctggtgt aggtgtgccg ggtgttggtg tgccgggtgt tggtgtacca 420
   ggtggcggtg ttccgggtgc aggcgttccg ggtggcggtg tgccgggcgt gggtgttccg 480
   ggcgtgggtg ttccgggtgg cggtgtgccg ggcgcaggtg ttcctggtgt aggtgtgccg 540
   ggtgttggtg tgccgggtgt tggtgtacca ggtggcggtg ttccgggtgc aggcgttccg 600
   ggtggcggtg tgccgggcgt gggtgttccg ggcgtgggtg ttccgggtgg cggtgtgccg 660
   ggcgcaggtg ttcctggtgt aggtgtgccg ggtgttggtg tgccgggtgt tggtgtacca 720
   ggtggcggtg ttccgggtgc aggcgttccg ggtggcggtg tgccgggcgt gggtgttccg 780
   ggcgtgggtg ttccgggtgg cggtgtgccg ggcgcaggtg ttcctggtgt aggtgtgccg 840
   ggtgttggtg tgccgggtgt tggtgtacca ggtggcggtg ttccgggtgc aggcgttccg 900
   ggtggcggtg tgccgggcgt gggtgttccg ggcgtgggtg ttccgggtgg cggtgtgccg 960
   ggcgcaggtg ttcctggtgt aggtgtgccg ggtgttggtg tgccgggtgt tggtgtacca 1020
   ggtggcggtg ttccgggtgc aggcgttccg ggtggcggtg tgccgggcgt gggtgttccg 1080
   ggcgtgggtg ttccgggtgg cggtgtgccg ggcgcaggtg ttcctggtgt aggtgtgccg 1140
   ggtgttggtg tgccgggtgt tggtgtacca ggtggcggtg ttccgggtgc aggcgttccg 1200
   ggtggcggtg tgccgggcgt gggtgttccg ggcgtgggtg ttccgggtgg cggtgtgccg 1260
   ggcgcaggtg ttcctggtgt aggtgtgccg ggtgttggtg tgccgggtgt tggtgtacca 1320
   ggtggcggtg ttccgggtgc aggcgttccg ggtggcggtg tgccgggcgt gggtgttccg 1380
   ggcgtgggtg ttccgggtgg cggtgtgccg ggcgcaggtg ttcctggtgt aggtgtgccg 1440
   ggtgttggtg tgccgggtgt tggtgtacca ggtggcggtg ttccgggtgc aggcgttccg 1500
   ggtggcggtg tgccgggcgg gctggcggcc gc 1532
<210> 48
   <211> 2322
   <212> DNA
   <213> Künstliche Sequenz
<220>
   <223> Beschreibung der künstlichen Sequenz:SM12-70xELP (Pflanzen)
<400> 48
   atggcttcca aaccttttct atctttgctt tcactttcct tgcttctctt tacaagcaca 60
   tgtttagcag gatcccagtt acccgggcag ggaggttatg gtggtctggg gggccagggt 120
   gctggccaag gaggttatgg tggtctgggg agtcagggcg ctggtcgtgg gggactgggt 180
   ggccaaggtg caggagctgc tgctgcagct gcaggtggag ccgggcaggg aggtctggga 240
   gggcagggag cgggccaagg tgcaggagca gctgcagcag ctgcaggtgg agccgggcag 300
   ggaggttatg gtggtctggg gagtcagggc gctggtcgtg gggactgggt tggccaaggt 360
   gcaggagcag ctgcagctgc tgcaggtgga gccgggcagg gaggttatgg tggtctgggg 420
   agtcagggtg ctggtcgtgg aggccaaggt gcaggagctg cagcagcagc tgcaggtgga 480
   gccgggcagg gaggttatgg tggtctgggg agtcagggcg ctggtcgtgg gggactgggt 540
   ggccaaggtg caggagcagc tgcagctgct gcaggtggag ccgggcaggg aggttatggt 600
   ggtctgggga gtcagggtgc tggtcgtgga ggccaaggtg caggagctgc agcagcagct 660
   gcaggtggag ccgggcaggg aggttatggt ggtctgggga gtcagggtgc tggtcgtgga 720
   ggccaaggtg caggagctgc agcagcagct gcaggtggag ccgggcaggg aggttatggt 780
   ggtctggggg gccagggtgc tggccaagga ggttatggtg gtctggggag tcagggcgct 840
   ggtcgtgggg gactgggtgg ccaaggtgca ggagctgctg ctgcagctgc aggtggagcc 900
   gggcagggag gtctgggagg gcagggagcg ggccaaggtg caggagcagc tgcagcagct 960
   gcaggtggag ccgggcaggg aggttatggt ggtctgggga gtcagggtgc tggtcgtgga 1020
   ggccaaggtg caggagctgc agcagcagct gcaggtggag ccgggcaggg aggttatggt 1080
   ggtctgggga gtcagggcgc tggtcgtggg ggactgggtg gccaaggtgc aggagcagct 1140
   gcagctgctg caggtggagc cggcggacaa gcggccgcag aacaaaaact catctcagaa 1200
   gaggatctga atggggccgt cgagatgggc cacggcgtgg gtgttccggg cgtgggtgtt 1260
   ccgggtggcg gtgtgccggg cgcaggtgtt cctggtgtag gtgtgccggg tgttggtgtg 1320
   ccgggtgttg gtgtaccagg tggcggtgtt ccgggtgcag gcgttccggg tggcggtgtg 1380
   ccgggcgtgg gtgttccggg cgtgggtgtt ccgggtggcg gtgtgccggg cgcaggtgtt 1440
   cctggtgtag gtgtgccggg tgttggtgtg ccgggtgttg gtgtaccagg tggcggtgtt 1500
   ccgggtgcag gcgttccggg tggcggtgtg ccgggcgtgg gtgttccggg cgtgggtgtt 1560
   ccgggtggcg gtgtgccggg cgcaggtgtt cctggtgtag gtgtgccggg tgttggtgtg 1620
   ccgggtgttg gtgtaccagg tggcggtgtt ccgggtgcag gcgttccggg tggcggtgtg 1680
   ccgggcgtgg gtgttccggg cgtgggtgtt ccgggtggcg gtgtgccggg cgcaggtgtt 1740
   cctggtgtag gtgtgccggg tgttggtgtg ccgggtgttg gtgtaccagg tggcggtgtt 1800
   ccgggtgcag gcgttccggg tggcggtgtg ccgggcgtgg gtgttccggg cgtgggtgtt 1860
   ccgggtggcg gtgtgccggg cgcaggtgtt cctggtgtag gtgtgccggg tgttggtgtg 1920
   ccgggtgttg gtgtaccagg tggcggtgtt ccgggtgcag gcgttccggg tggcggtgtg 1980
   ccgggcgtgg gtgttccggg cgtgggtgtt ccgggtggcg gtgtgccggg cgcaggtgtt 2040
   cctggtgtag gtgtgccggg tgttggtgtg ccgggtgttg gtgtaccagg tggcggtgtt 2100
   ccgggtgcag gcgttccggg tggcggtgtg ccgggcgtgg gtgttccggg cgtgggtgtt 2160
   ccgggtggcg gtgtgccggg cgcaggtgtt cctggtgtag gtgtgccggg tgttggtgtg 2220
   ccgggtgttg gtgtaccagg tggcggtgtt ccgggtgcag gcgttccggg tggcggtgtg 2280
   ccgggcgggc tggcggccgc agaacccaaa gacgaactct ag 2322
<210> 49
   <211> 773
   <212> PRT
   <213> Künstliche Sequenz
<220>
   <223> Beschreibung der künstlichen Sequenz:SM12-70xELP (Pflanzen)
<400> 49
   Met Ala Ser Lys Pro Phe Leu Ser Leu Leu Ser Leu Ser Leu Leu Leu
    1               5                  10                  15
   Phe Thr Ser Thr Cys Leu Ala Gly Ser Gln Leu Pro Gly Gln Gly Gly
               20                  25                  30
   Tyr Gly Gly Leu Gly Gly Gln Gly Ala Gly Gln Gly Gly Tyr Gly Gly
           35                  40                  45
   Leu Gly Ser Gln Gly Ala Gly Arg Gly Gly Leu Gly Gly Gln Gly Ala
       50                  55                  60
   Gly Ala Ala Ala Ala Ala Ala Gly Gly Ala Gly Gln Gly Gly Leu Gly
   65                  70                  75                  80
   Gly Gln Gly Ala Gly Gln Gly Ala Gly Ala Ala Ala Ala Ala Ala Gly
                   85                  90                  95
   Gly Ala Gly Gln Gly Gly Tyr Gly Gly Leu Gly Ser Gln Gly Ala Gly
               100                 105                 110
   Arg Gly Gly Leu Gly Gly Gln Gly Ala Gly Ala Ala Ala Ala Ala Ala
           115                 120                 125
   Gly Gly Ala Gly Gln Gly Gly Tyr Gly Gly Leu Gly Ser Gln Gly Ala
       130                 135                 140
   Gly Arg Gly Gly Gln Gly Ala Gly Ala Ala Ala Ala Ala Ala Gly Gly
   145                 150                 155                 160
   Ala Gly Gln Gly Gly Tyr Gly Gly Leu Gly Ser Gln Gly Ala Gly Arg
                   165                 170                 175
   Gly Gly Leu Gly Gly Gln Gly Ala Gly Ala Ala Ala Ala Ala Ala Gly
               180                 185                 190
   Gly Ala Gly Gln Gly Gly Tyr Gly Gly Leu Gly Ser Gln Gly Ala Gly
           195                 200                 205
   Arg Gly Gly Gln Gly Ala Gly Ala Ala Ala Ala Ala Ala Gly Gly Ala
       210                 215                 220
   Gly Gln Gly Gly Tyr Gly Gly Leu Gly Ser Gln Gly Ala Gly Arg Gly
   225                 230                 235                 240
   Gly Gln Gly Ala Gly Ala Ala Ala Ala Ala Ala Gly Gly Ala Gly Gln
                   245                 250                 255
   Gly Gly Tyr Gly Gly Leu Gly Gly Gln Gly Ala Gly Gln Gly Gly Tyr
               260                 265                 270
   Gly Gly Leu Gly Ser Gln Gly Ala Gly Arg Gly Gly Leu Gly Gly Gln
           275                 280                 285
   Gly Ala Gly Ala Ala Ala Ala Ala Ala Gly Gly Ala Gly Gln Gly Gly
       290                 295                 300
   Leu Gly Gly Gln Gly Ala Gly Gln Gly Ala Gly Ala Ala Ala Ala Ala
   305                 310                 315                 320
   Ala Gly Gly Ala Gly Gln Gly Gly Tyr Gly Gly Leu Gly Ser Gln Gly
                   325                 330                 335
   Ala Gly Arg Gly Gly Gln Gly Ala Gly Ala Ala Ala Ala Ala Ala Gly
               340                 345                 350
   Gly Ala Gly Gln Gly Gly Tyr Gly Gly Leu Gly Ser Gln Gly Ala Gly
           355                 360                 365
   Arg Gly Gly Leu Gly Gly Gln Gly Ala Gly Ala Ala Ala Ala Ala Ala
       370                 375                 380
   Gly Gly Ala Gly Gly Gln Ala Ala Ala Glu Gln Lys Leu Ile Ser Glu
   385                 390                 395                 400
   Glu Asp Leu Asn Gly Ala Val Glu Met Gly His Gly Val Gly Val Pro
                   405                 410                 415
   Gly Val Gly Val Pro Gly Gly Gly Val Pro Gly Ala Gly Val Pro Gly
               420                 425                 430
   Val Gly Val Pro Gly Val Gly Val Pro Gly Val Gly Val Pro Gly Gly
           435                 440                 445
   Gly Val Pro Gly Ala Gly Val Pro Gly Gly Gly Val Pro Gly Val Gly
       450                 455                 460
   Val Pro Gly Val Gly Val Pro Gly Gly Gly Val Pro Gly Ala Gly Val
   465                 470                 475                 480
   Pro Gly Val Gly Val Pro Gly Val Gly Val Pro Gly Val Gly Val Pro
                   485                 490                 495
   Gly Gly Gly Val Pro Gly Ala Gly Val Pro Gly Gly Gly Val Pro Gly
               500                 505                 510
   Val Gly Val Pro Gly Val Gly Val Pro Gly Gly Gly Val Pro Gly Ala
           515                 520                 525
   Gly Val Pro Gly Val Gly Val Pro Gly Val Gly Val Pro Gly Val Gly
       530                 535                 540
   Val Pro Gly Gly Gly Val Pro Gly Ala Gly Val Pro Gly Gly Gly Val
   545                 550                 555                 560
   Pro Gly Val Gly Val Pro Gly Val Gly Val Pro Gly Gly Gly Val Pro
                   565                 570                 575
   Gly Ala Gly Val Pro Gly Val Gly Val Pro Gly Val Gly Val Pro Gly
               580                 585                 590
   Val Gly Val Pro Gly Gly Gly Val Pro Gly Ala Gly Val Pro Gly Gly
           595                 600                 605
   Gly Val Pro Gly Val Gly Val Pro Gly Val Gly Val Pro Gly Gly Gly
       610                 615                 620
   Val Pro Gly Ala Gly Val Pro Gly Val Gly Val Pro Gly Val Gly Val
   625 630 635 640
   Pro Gly Val Gly Val Pro Gly Gly Gly Val Pro Gly Ala Gly Val Pro
   645 650 655
   Gly Gly Gly Val Pro Gly Val Gly Val Pro Gly Val Gly Val Pro Gly
   660 665 670
   Gly Gly Val Pro Gly Ala Gly Val Pro Gly Val Gly Val Pro Gly Val
   675 680 685
   Gly Val Pro Gly Val Gly Val Pro Gly Gly Gly Val Pro Gly Ala Gly
   690 695 700
   Val Pro Gly Gly Gly Val Pro Gly Val Gly Val Pro Gly Val Gly Val
   705 710 715 720
   Pro Gly Gly Gly Val Pro Gly Ala Gly Val Pro Gly Val Gly Val Pro
   725 730 735
   Gly Val Gly Val Pro Gly Val Gly Val Pro Gly Gly Gly Val Pro Gly
   740 745 750
   Ala Gly Val Pro Gly Gly Gly Val Pro Gly Gly Leu Ala Ala Ala Glu
   755 760 765
   Pro Lys Asp Glu Leu
   770
<210> 50
   <211> 2334
   <212> DNA
   <213> Künstliche Sequenz
<220>
   <223> Beschreibung der künstlichen Sequenz:SM12-70xELP (E.coli)
<400> 50
   atggctagca tgactggtgg acagcaaatg ggtcgcggat cccagttacc cgggcaggga 60
   ggttatggtg gtctgggggg ccagggtgct ggccaaggag gttatggtgg tctggggagt 120
   cagggcgctg gtcgtggggg actgggtggc caaggtgcag gagctgctgc tgcagctgca 180
   ggtggagccg ggcagggagg tctgggaggg cagggagcgg gccaaggtgc aggagcagct 240
   gcagcagctg caggtggagc cgggcaggga ggttatggtg gtctggggag tcagggcgct 300
   ggtcgtgggg gactgggtgg ccaaggtgca ggagcagctg cagctgctgc aggtggagcc 360
   gggcagggag gttatggtgg tctggggagt cagggtgctg gtcgtggagg ccaaggtgca 420
   ggagctgcag cagcagctgc aggtggagcc gggcagggag gttatggtgg tctggggagt 480
   cagggcgctg gtcgtggggg actgggtggc caaggtgcag gagcagctgc agctgctgca 540
   ggtggagccg ggcagggagg ttatggtggt ctggggagtc agggtgctgg tcgtggaggc 600
   caaggtgcag gagctgcagc agcagctgca ggtggagccg ggcagggagg ttatggtggt 660
   ctggggagtc agggtgctgg tcgtggaggc caaggtgcag gagctgcagc agcagctgca 720
   ggtggagccg ggcagggagg ttatggtggt ctggggggcc agggtgctgg ccaaggaggt 780
   tatggtggtc tggggagtca gggcgctggt cgtgggggac tgggtggcca aggtgcagga 840
   gctgctgctg cagctgcagg tggagccggg cagggaggtc tgggagggca gggagcgggc 900
   caaggtgcag gagcagctgc agcagctgca ggtggagccg ggcagggagg ttatggtggt 960
   ctggggagtc agggtgctgg tcgtggaggc caaggtgcag gagctgcagc agcagctgca 1020
   ggtggagccg ggcagggagg ttatggtggt ctggggagtc agggcgctgg tcgtggggga 1080
   ctgggtggcc aaggtgcagg agcagctgca gctgctgcag gtggagccgg cggacaagcg 1140
   gccgcagaac aaaaactcat ctcagaagag gatctgaatg gggccgtcga gatgggccac 1200
   ggcgtgggtg ttccgggcgt gggtgttccg ggtggcggtg tgccgggcgc aggtgttcct 1260
   ggtgtaggtg tgccgggtgt tggtgtgccg ggtgttggtg taccaggtgg cggtgttccg 1320
   ggtgcaggcg ttccgggtgg cggtgtgccg ggcgtgggtg ttccgggcgt gggtgttccg 1380
   ggtggcggtg tgccgggcgc aggtgttcct ggtgtaggtg tgccgggtgt tggtgtgccg 1440
   ggtgttggtg taccaggtgg cggtgttccg ggtgcaggcg ttccgggtgg cggtgtgccg 1500
   ggcgtgggtg ttccgggcgt gggtgttccg ggtggcggtg tgccgggcgc aggtgttcct 1560
   ggtgtaggtg tgccgggtgt tggtgtgccg ggtgttggtg taccaggtgg cggtgttccg 1620
   ggtgcaggcg ttccgggtgg cggtgtgccg ggcgtgggtg ttccgggcgt gggtgttccg 1680
   ggtggcggtg tgccgggcgc aggtgttcct ggtgtaggtg tgccgggtgt tggtgtgccg 1740
   ggtgttggtg taccaggtgg cggtgttccg ggtgcaggcg ttccgggtgg cggtgtgccg 1800
   ggcgtgggtg ttccgggcgt gggtgttccg ggtggcggtg tgccgggcgc aggtgttcct 1860
   ggtgtaggtg tgccgggtgt tggtgtgccg ggtgttggtg taccaggtgg cggtgttccg 1920
   ggtgcaggcg ttccgggtgg cggtgtgccg ggcgtgggtg ttccgggcgt gggtgttccg 1980
   ggtggcggtg tgccgggcgc aggtgttcct ggtgtaggtg tgccgggtgt tggtgtgccg 2040
   ggtgttggtg taccaggtgg cggtgttccg ggtgcaggcg ttccgggtgg cggtgtgccg 2100
   ggcgtgggtg ttccgggcgt gggtgttccg ggtggcggtg tgccgggcgc aggtgttcct 2160
   ggtgtaggtg tgccgggtgt tggtgtgccg ggtgttggtg taccaggtgg cggtgttccg 2220
   ggtgcaggcg ttccgggtgg cggtgtgccg ggcgggctgg cggccgcaga acaaaaactc 2280
   atctcagaag aggatctgaa tggggccgtc gagcaccacc accaccacca ctga       2334
<210> 51
   <211> 777
   <212> PRT
   <213> Künstliche Sequenz
<220>
   <223> Beschreibung der künstlichen Sequenz:SM12-70xELP (E.coli)
<400> 51
   Met Ala Ser Met Thr Gly Gly Gln Gln Met Gly Arg Gly Ser Gln Leu
    1               5                  10                  15
   Pro Gly Gln Gly Gly Tyr Gly Gly Leu Gly Gly Gln Gly Ala Gly Gln
               20                  25                  30
   Gly Gly Tyr Gly Gly Leu Gly Ser Gln Gly Ala Gly Arg Gly Gly Leu
           35                  40                  45
   Gly Gly Gln Gly Ala Gly Ala Ala Ala Ala Ala Ala Gly Gly Ala Gly
       50                  55                  60
   Gln Gly Gly Leu Gly Gly Gln Gly Ala Gly Gln Gly Ala Gly Ala Ala
   65                  70                  75                  80
   Ala Ala Ala Ala Gly Gly Ala Gly Gln Gly Gly Tyr Gly Gly Leu Gly
                   85                  90                  95
   Ser Gln Gly Ala Gly Arg Gly Gly Leu Gly Gly Gln Gly Ala Gly Ala
               100                 105                 110
   Ala Ala Ala Ala Ala Gly Gly Ala Gly Gln Gly Gly Tyr Gly Gly Leu
           115                 120                 125
   Gly Ser Gln Gly Ala Gly Arg Gly Gly Gln Gly Ala Gly Ala Ala Ala
       130                 135                 140
   Ala Ala Ala Gly Gly Ala Gly Gln Gly Gly Tyr Gly Gly Leu Gly Ser
   145                 150                 155                 160
   Gln Gly Ala Gly Arg Gly Gly Leu Gly Gly Gln Gly Ala Gly Ala Ala
                   165                 170                 175
   Ala Ala Ala Ala Gly Gly Ala Gly Gln Gly Gly Tyr Gly Gly Leu Gly
               180                 185                 190
   Ser Gln Gly Ala Gly Arg Gly Gly Gln Gly Ala Gly Ala Ala Ala Ala
           195                 200                 205
   Ala Ala Gly Gly Ala Gly Gln Gly Gly Tyr Gly Gly Leu Gly Ser Gln
       210                 215                 220
   Gly Ala Gly Arg Gly Gly Gln Gly Ala Gly Ala Ala Ala Ala Ala Ala
   225                 230                 235                 240
   Gly Gly Ala Gly Gln Gly Gly Tyr Gly Gly Leu Gly Gly Gln Gly Ala
                   245                 250                 255
   Gly Gln Gly Gly Tyr Gly Gly Leu Gly Ser Gln Gly Ala Gly Arg Gly
               260                 265                 270
   Gly Leu Gly Gly Gln Gly Ala Gly Ala Ala Ala Ala Ala Ala Gly Gly
   275 280 285
   Ala Gly Gln Gly Gly Leu Gly Gly Gln Gly Ala Gly Gln Gly Ala Gly
   290 295 300
   Ala Ala Ala Ala Ala Ala Gly Gly Ala Gly Gln Gly Gly Tyr Gly Gly
   305 310 315 320
   Leu Gly Ser Gln Gly Ala Gly Arg Gly Gly Gln Gly Ala Gly Ala Ala
   325 330 335
   Ala Ala Ala Ala Gly Gly Ala Gly Gln Gly Gly Tyr Gly Gly Leu Gly
   340 345 350
   Ser Gln Gly Ala Gly Arg Gly Gly Leu Gly Gly Gln Gly Ala Gly Ala
   355 360 365
   Ala Ala Ala Ala Ala Gly Gly Ala Gly Gly Gln Ala Ala Ala Glu Gln
   370 375 380
   Lys Leu Ile Ser Glu Glu Asp Leu Asn Gly Ala Val Glu Met Gly His
   385 390 395 400
   Gly Val Gly Val Pro Gly Val Gly Val Pro Gly Gly Gly Val Pro Gly
   405 410 415
   Ala Gly Val Pro Gly Val Gly Val Pro Gly Val Gly Val Pro Gly Val
   420 425 430
   Gly Val Pro Gly Gly Gly Val Pro Gly Ala Gly Val Pro Gly Gly Gly
   435 440 445
   Val Pro Gly Val Gly Val Pro Gly Val Gly Val Pro Gly Gly Gly Val
   450 455 460
   Pro Gly Ala Gly Val Pro Gly Val Gly Val Pro Gly Val Gly Val Pro
   465 470 475 480
   Gly Val Gly Val Pro Gly Gly Gly Val Pro Gly Ala Gly Val Pro Gly
   485 490 495
   Gly Gly Val Pro Gly Val Gly Val Pro Gly Val Gly Val Pro Gly Gly
   500 505 510
   Gly Val Pro Gly Ala Gly Val Pro Gly Val Gly Val Pro Gly Val Gly
   515 520 525
   Val Pro Gly Val Gly Val Pro Gly Gly Gly Val Pro Gly Ala Gly Val
       530                 535                 540
   Pro Gly Gly Gly Val Pro Gly Val Gly Val Pro Gly Val Gly Val Pro
   545                 550                 555                 560
   Gly Gly Gly Val Pro Gly Ala Gly Val Pro Gly Val Gly Val Pro Gly
                   565                 570                 575
   Val Gly Val Pro Gly Val Gly Val Pro Gly Gly Gly Val Pro Gly Ala
               580                 585                 590
   Gly Val Pro Gly Gly Gly Val Pro Gly Val Gly Val Pro Gly Val Gly
           595                 600                 605
   Val Pro Gly Gly Gly Val Pro Gly Ala Gly Val Pro Gly Val Gly Val
       610                 615                 620
   Pro Gly Val Gly Val Pro Gly Val Gly Val Pro Gly Gly Gly Val Pro
   625                 630                 635                 640
   Gly Ala Gly Val Pro Gly Gly Gly Val Pro Gly Val Gly Val Pro Gly
                   645                 650                 655
   Val Gly Val Pro Gly Gly Gly Val Pro Gly Ala Gly Val Pro Gly Val
               660                 665                 670
   Gly Val Pro Gly Val Gly Val Pro Gly Val Gly Val Pro Gly Gly Gly
           675                 680                 685
   Val Pro Gly Ala Gly Val Pro Gly Gly Gly Val Pro Gly Val Gly Val
       690                 695                 700
   Pro Gly Val Gly Val Pro Gly Gly Gly Val Pro Gly Ala Gly Val Pro
   705                 710                 715                 720
   Gly Val Gly Val Pro Gly Val Gly Val Pro Gly Val Gly Val Pro Gly
                   725                 730                 735
   Gly Gly Val Pro Gly Ala Gly Val Pro Gly Gly Gly Val Pro Gly Gly
               740                 745                 750
   Leu Ala Ala Ala Glu Gln Lys Leu Ile Ser Glu Glu Asp Leu Asn Gly
           755                 760                 765
   Ala Val Glu His His His His His His
       770                 775